# EUROPEAN PATENT SPECIFICATION

(11) **EP 2 788 372 B1**
(45) Date of publication and mention of the grant of the patent: **10.07.2019**
(21) Application number: 12806388.0
(22) Date of filing: 07.12.2012
(51) Int. Cl.: A61K 39/12, C07K 14/005

(54) **MUTATED LENTIVIRAL ENV PROTEINS AND THEIR USE AS DRUGS**
MUTIERTE LENTIVIRALE ENV-PROTEINE UND IHRE VERWENDUNG ALS ARZNEIMITTEL
PROTÉINES ENV LENTIVIRALES MUTÉES ET APPLICATIONS COMME MÉDICAMENTS

(30) Priority: 07.12.2011 EP 11306625
(43) Date of publication of application: 15.10.2014
(73) Proprietor: Viroxis S.A.S., 75017 Paris (FR); Centre National de la Recherche Scientifique, 75794 Paris Cedex 16 (FR); Université Paris Sud XI, 91405 Orsay Cedex (FR); Institut Gustave Roussy, 94805 Villejuif Cédex (FR)
(72) Inventor: HEIDMANN, Thierry, F-75017 Paris (FR)
(74) Representative: Grosset-Fournier, Chantal Catherine
(86) International application number: PCT/EP2012/074835
(87) International publication number: WO 2013/083799

(56) References cited:
- EP-A1- 2 338 984
- WO-A1-2005/095442
- WO-A2-2005/012502
- WO-A2-2010/022740
- DENNER J ET AL: "THE IMMUNOSUPPRESSIVE PEPTIDE OF HIV-1: FUNCTIONAL DOMAINS AND IMMUNE RESPONSE IN AIDS PATIENTS", AIDS, PHILADELPHIA,PA, US, vol. 8, no. 8, 1 August 1994 (1994-08-01), pages 1063-1072, XP000647542,
- SCHLECHT-LOUF ET AL.: PROC NATL ACAD SCI U S A., vol. 107, no. 8, 2010, pages 3782-7, XP002675453,
- DATABASE UniProt [Online] 19 October 2011 (2011-10-19), "SubName: Full=Envelope glycoprotein; Flags: Fragment;", XP002675454, retrieved from EBI accession no. UNIPROT:G1JQN5 Database accession no. G1JQN5
- DATABASE UniProt [Online] 15 June 2010 (2010-06-15), "SubName: Full=Cell division protein FtsY; PPREVPTEPT TRLAKLRNRL AKSNNAMGRG LLALLSRDTL DEATWEEIED TLIVADLGVD", XP002675455, retrieved from EBI accession no. UNIPROT:D4YP64 Database accession no. D4YP64
- MANGENEY M ET AL: "Tumor cells expressing a retroviral envelope escape immune rejection in vivo", PROCEEDINGS OF THE NATIONAL ACADEMY OF SCIENCES, NATIONAL ACADEMY OF SCIENCES, US, vol. 95, no. 25, 1 December 1998 (1998-12-01), pages 14920-14925, XP002298109, ISSN: 0027-8424, DOI: 10.1073/PNAS.95.25.14920 cited in the application
- MANGENEY M ET AL: "Placental syncytins: Genetic disjunction between the fusogenic and immunosuppressive activity of retroviral envelope proteins", PROCEEDINGS OF THE NATIONAL ACADEMY OF SCIENCES, NATIONAL ACADEMY OF SCIENCES, US, vol. 104, no. 51, 18 December 2007 (2007-12-18), pages 20534-20539, XP002633415, ISSN: 0027-8424, DOI: 10.1073/PNAS.0707873105 [retrieved on 2007-12-12]

## Description

The present invention relates to mutated lentiviral ENV proteins and their use as drugs.

Lentivirus is a genus of slow viruses of the Retroviridae family, characterized by a long incubation period. Lentiviruses can deliver a significant amount of genetic information into the DNA of the host cell and have the unique ability among retroviruses of being able to replicate in non-dividing cells, so they are one of the most efficient methods of a gene delivery vector. HIV, SIV, and FIV are all examples of lentiviruses.

Human immunodeficiency virus (HIV) is according to WHO one of the most serious health crisis the world faces today. AIDS has killed more than 25 million people since 1981. In the most severely affected countries, the average life expectancy is now declining to 49 years of age - 13 years less than in the absence of AIDS. According to UNAIDS an estimated number of 39.5 million people were living with HIV virus in 2006 and 4.3 million were infected in 2006. In many regions new infections are heavily concentrated in the younger generations (15-24 years of age). Access to treatment and care has greatly increased in recent years. Determining real time trends to HIV incidence and in particular the impact of prevention programmes ideally requires long studies of a large number of people. Given the practical difficulties of conducting such studies focus has been placed on young women and their infants. Children living with HIV typically acquire infection through a mother-to-child-transmission (MTCT), which occur during pregnancy, delivery or during breastfeeding. Renewed efforts are urgently required to increase access to comprehensive and integrated programs to prevent HIV infection in infants and young children, which will indicate a route to HIV-free generations.
There are two known types of HIV; HIV-1 and HIV-2 that infect humans. They belong to a group of retroviruses called Lentiviruses and a virus similar to HIV has been found in African monkeys. Retroviruses transfer their genes from a producer cell to a target cell as a genomic RNA transcript, which is reverse-transcribed after infection and integrated into the DNA genome of the target cell.
The first person with a documented HIV-infection died in 1959. In the early 1980s doctors in the US become aware, that more and more patients suffered from abnormal infections and showed signs of immune failure. The syndrome was named Acquired Immune Deficiency Syndrome (AIDS) and it was soon after discovered that HIV was the causative agent for the observed destruction of the immune system. Initially patients were offered a treatment based solely on pain relief and almost all inevitably died. In mid 1990s there were two important breakthroughs in treatment. Firstly, a new group of antiretroviral agents were discovered and secondly it became possible to measure the amount of HIV virus in blood. These two advances made it possible to treat patients with a combination of different agents and doctors were able to check, whether the treatment actually worked. The result was that the immune system of infected patients gradually became normal and patients lived longer. Today infected people in Western countries are having the same level of quality of life as those not infected and they are able to have children, although the economical and psychological consequences of having HIV are huge. The situation is, however, even more severe in developing countries, where more than 95% of those people infected with HIV/AIDS are living. Worldwide more than 25 million people have died from AIDS in the last 25 years.
Approximately 95% of the people who get infected today live in the developing countries, where expensive antiviral drugs are not available. Therefore, there is an urgent need for an effective vaccine - the only effective solution to the uncontrolled HIV pandemic. During the last few years research has brought up new knowledge on the fundamental biology of HIV-virus which is leading to new antiviral drugs and strategies for vaccine design. In spite of these substantial advances, an effective vaccine does not yet exist. Only attenuated (that is live but weakened) HIV-strains has been able to provide immunity in primate studies even though they will never reach a required safety profile suitable for mass vaccination.
The replication process for HIV-1 has an error rate of about one per 10,000 base pairs. Since the entire viral genome is just under 10,000 base pairs, it is estimated that on average one error is introduced into the HIV-1 genome at each viral replication cycle.
This high mutation rate contributes to extensive variability of the viruses inside any one person and an even wider variability across populations.
This variability has resulted in three HIV-1 variants being described, and the subspecies of virus called "clades." The distinctions are based on the structure of the envelope proteins, which are especially variable. The M (for major) variant is by far the most prevalent world wide. Within the M variant are clades A, B. C, D, E, F. G H. I, J and K, with clades A through E representing the vast majority of infections globally. Clades A, C and D are dominant in Africa, while clade B is the most prevalent in Europe, North and South America and Southeast Asia. Clades E and C are dominant in Asia. These clades differ by as much as 35%. Another variant is clade O, which is observed in Cameroun isolates of HIV-1. The greatest variation in structure is seen in the envelope proteins gp120 and gp41. There are two important results from the very high mutation rate of HIV- 1 that have profound consequences for the epidemic. First, the high mutation rate is one of the mechanisms that allow the virus to escape from control by drug therapies. These new viruses represent resistant strains. The high mutation rate also allows the virus to escape the patient's immune system by altering the structures that are recognized by immune components. An added consequence of this extensive variability is that the virus can also escape from control by vaccines, and therefore makes it difficult to find vaccines based on envelope proteins which are effective.
Moreover, the virus produces proteins having immunosuppressive properties, allowing to escape the patient's immune system survey. Thus, cell expressing such proteins become "invisible" to the immune system.
Consequently for a vaccine, there is a need to provide proteins as antigens having lost, or substantially lost, their immunosuppressive functions, in order to generate an efficient response. This will enable the individuals once infected by the virus to allow the immune system to destroy the infected cells and prevent/cure the infection..
Prior art has already intended to provide such proteins.
For instance, the international application WO 2005/095,442 (Inventors : Renard, Mangeney & Heidmann) discloses mutations in the immunosuppressive domains of endogenous retroviruses (ERV) or onco retroviruses, such as HTLV or FeLV, ENV proteins. This document demonstrates that mutations at a specific position abolish the immunosuppressive properties of ENV proteins of ERV or onco retroviruses. However, the international application WO 2005/095, 442 never mentions or suggests that the mutations made in the immunosuppressive domain of ERV- or onco retroviruses ENV proteins can be transposable to lentiviral ENV proteins.

The international application WO 2010/022,740 discloses an extremely wide consensus sequence of a region of HIV ENV protein, described as follows:
X(1-22)-C(23)-X(24-28)-C(29)-(X30-50)
wherein the amino acid residues of the consensus sequence are selected from the groups of residues consisting of:
X(1): L, S, R, P, F, A, V, M, and I; and
X(2): Q, R, K, H, L, M, and P; and
X(3): A, T, V, H, S, R, Q, G, M, and E; and
X(4): R, K, G, E, T, S, C, M, and H; and
X(5): V, I, L, D, A, S, F, M, and G; and
X(6): L, Q, V, M, P, W, T, and I; and
X(7): A, S, T, V, L, G, F, D, M, and E; and
X(8): V, L, I, M, A, W, K, G, and E; and
X(9): E, K, G, D, A, V, M, and F; and
X(10): X; and
X(11): Y, L, F, H, C, I, T, M, and N; and
X(12): L, I, V, M, Q, P, T, Y, and A; and
X(13): K, R, Q, G, S, E, H, W, T, V, M, N, Z, Y, A, P, and C; and
X(14): D, N, G, E, Y, V, S, H, A, M, and I; and
X(15): Q, R, H, K, P, L, M, and N; and
X(16): Q, K, R, T, H, E, S, P, M, and L; and
X(17): L, F, I, R, V, P, S, M, and H, and
X(18): L, M, P, I, H, and S; and
X(19): X; and
X(20): I, L, M, V, S, F, T, D, A, R, P, and J; and
X(21): W, R, G, F, L, M, and T; and
X(22): G, D, A, R, M, and C; and
X(24): X; and
X(25): G, R, E, N, A, M, and D; and
X(26): K, R, N, E, Q, T, S, I, M, and G; and
X(27): L, H, I, T, V, F, R, Q, S, P, A, J, M, and Y; and
X(28): I, V, T, L, R, F and M; and
X(30): T, P, Y, A, N, S, I, V, R, L, M, and H; and
X(31): T, S, P, N, M and I; and
X(32): A, N, T, S, D, R, FQ, P, I, E, V, M, L, K, H, C, and B; and
X(33): V, A, L, M, G, R, and C; and
X(34): X; and
X(35): W, R, G, L, M, and P; and
X(36): N, S, D, B, K, E, R, Q, M, and G; and
X(37): S, T, A, N, D, V, I, E, Y, K, L, R, G, P, M, F, W, H, Q, B, and C; and
X(38): S, T, N, I, G, R, L, C, A, W, M and E; and
X(39): W, G, A, R, E, C, Y, V, S, M, and H; and
X(40): X; and
X(41): N, G, K, S, D, E, T, R, H, P, A, B, V, Q, Y, M, and I; and
X(42): K, R, N, D, S, T, G, E, I, V, Y, Q, P, H, A, W, M, and C, and
X(43): S, T, N, K, I, R, D, E, P, L, A, W, G, M, H, Y, F, V, and C,
X(44): L, Y, Q, F, E, H, S, V, K, M, T, I, W, N, D, R, P, A, and G; and
X(45): D, E, N, S, T, K, G, L, A, Q, H, I, Y, B, R, V, P, M, F, W, Z, and C; and
X(46): E, D, Q, Y, K, N, T, S, A, W, H, M, R, I, G, L, V, Z, F, B, and P; and
X(47): I, D, E, M, G, T, Q, S, W, L, N, Y, K, V, R, F, A, P, and H, and
X(48): W, I, T, N, D, E, L, G, S, Y, R, V, K, H, A, Q, M, and F; and
X((49): D, N, E, G, W, Q, K, H, L, B, S, I, Y, T, A, R, M, Z, and V; and
X(50): N, D, T, K, S, H, L, G, E, W, I, Q, M, R, B, Y, P, and A;

This consensus sequence contains 50 amino acids, in which the specific amino acids in position 10, 19, 24, 34 and 40 are defined as affecting the immunogenic properties of a HIV-1 envelope polypeptide, and the 45 remaining positions are randomly defined including the most common amino acids of wild-type HIV ENV proteins.

In fact, the teaching of WO 2010/022,740 is a transposition from endogenous retroviruses (ERV) or onco retroviruses to lentivirus on the basis of the teaching of WO 2005/095,442 (Inventors : Renard, Mangeney & Heidmann), but said transposition is inappropriate in the case of lentivirus, as shown by the Inventor of the present invention.
Briefly speaking, Dr Heidmann is an Inventor in WO 2005/095,442 and in the present invention. As a matter of fact, the effects of the mutations described in WO 2005/095,442 were also tested in lentivirus by the Inventor of the present invention, but no effect was observed when the mutations identified in endogenous retroviruses or onco retroviruses were transposed into ENV protein of lentivirus.

Moreover, since any amino acid can be assigned to the positions 10, 19, 24, 34 or 40 in the consensus sequence, WO 2010/022,740 teaches that such mutations can be effective using any amino acid residue. This teaching is in contradiction with the present invention, showing that the immunosuppressive properties of HIV-1 ENV protein are only affected by specific mutations that are defined not only by their position, but also by the nature of the substituted amino acid residues.

Furthermore, WO 2010/022,740 discloses experimental results for only one specific mutation within the immunosuppressive domain of HIV ENV protein, as defined by the 50 amino acids consensus sequence. The mutation, a substitution by R as the only one exemplified in the international application WO 2010/022,740, occurs at the amino acid in position 19, which again is equivalent to the position disclosed in the international application WO 2005/095,442 if one simply aligns ENV sequences (see Fig 3 in WO 2010/022,740, which is a copy of Figure 3 in Benit et al. 2001, Journal of Virology, Vol. 75, No. 23, p.11709-11719) (Of note not only the position of the amino acid, but also the nature of the substitution (by arginine) is similar to the one described in WO 2005/095,442).

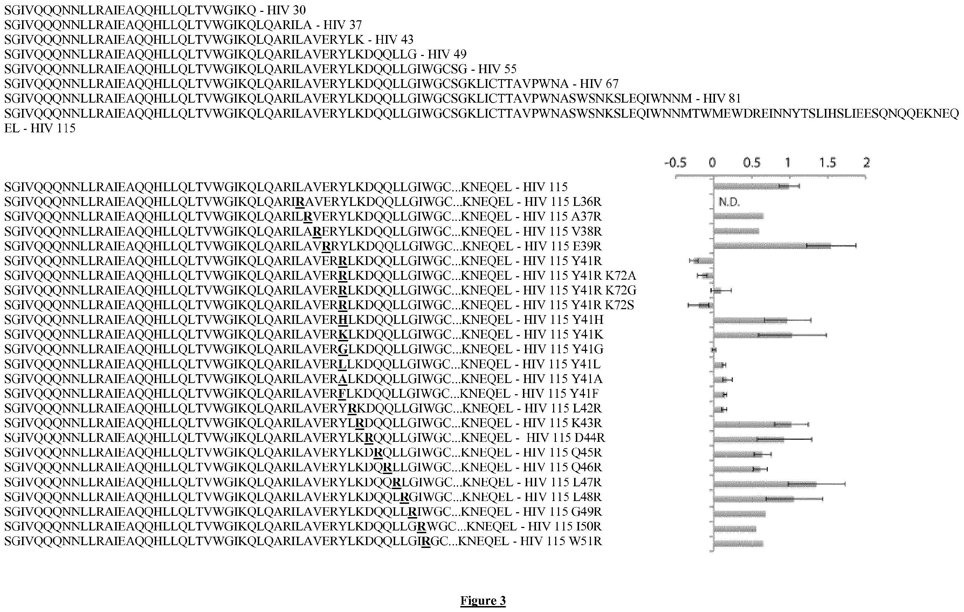

More specifically, when aligning the ENV sequences respectively of an endogenous retrovirus or onco retrovirus and a lentivirus, according to Figure 3 of Benit et al.: it appears that the position 19 in lentivirus corresponds to the position in WO 2005/095,442 where a specific substitution into arginine, E→R for endogenous or onco retrovirus, results in loss of immunosuppressive activity.

The international application WO 2010/022,740 discloses that said mutated HIV ENV protein inhibits proliferation of PBMC *ex vivo,* but such *ex vivo* result has no *in vivo* predictive value.

However, WO 2010/022,740 never discloses or suggests that mutants are efficient *in vivo,* i.e. that cell expressing mutants are detected by the immune system.
Moreover, as disclosed hereafter, such mutations are not efficient *in vivo.* Indeed, results disclosed hereafter in the example section relative to the present invention demonstrate that said substitution G19R does not inhibit *in vivo* the immunosuppressive properties of the ENV protein.
As a consequence, WO 2010/022,740 raises the same technical problem as the present invention, but does not offer an appropriate technical solution. This prior art reveals the difficulties to overcome the identification of the effective mutations affecting the immunosuppressive properties of the lentiviral ENV proteins.
Thus, the provision of *in vivo* effective non immunosuppressive lentiviral ENV proteins remains.
One aim of the invention is to provide new mutated ENV proteins devoid of immunosuppressive properties.
Another aim of the invention is to provide a new pharmaceutical composition efficient for treating lentiviral infection.
Another aim of the invention is to provide an efficient vaccine.

The description relates to a pharmaceutical composition comprising as active substance:
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein, said mutated human or simian lentiviral ENV protein having at least 70% identity, preferably at least 80% identity, to one sequence chosen from the group consisting of SEQ ID NO: 216, SEQ ID NO: 420 and SEQ ID NO: 421,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, F, G, L, R or deleted, and X_{b} is C, D, E, H, I, K, L, M, N, P, Q, S, T, V, W or Y, or
   Xₐ is C, D, E, H, I, K, M, N, P, Q, S, T, V, W or Y, and X_{b} is A, F, G, R or deleted, or Xₐ is A, F, G, L, R or deleted, and X_{b} is A, F, G, R or deleted,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, F, G, L, R or deleted, and X_{b} is C, D, E, H, I, K, L, M, N, P, Q, S, T, V, W or Y, or
   Xₐ is C, D, E, H, I, K, M, N, P, Q, S, T, V, W or Y, and X_{b} is A, F, G, R or deleted, or Xₐ is A, F, G, L, R or deleted, and X_{b} is A, F, G, R or deleted,
   in association with a pharmaceutically acceptable carrier,
   said substantial absence of immunosuppressive activity of the above mentioned mutated human or simian lentiviral ENV protein or of the above defined fragment being liable to be assessed by the fact that in an *in vivo* assay involving engrafted tumor cells rejection,
   said tumor cells being transduced either so as to express said mutated ENV protein or said fragment ("mutated ENV tumor cells"),
   or said tumor cells being transduced so as to express said wild type ENV protein or a fragment thereof ("wild type ENV tumor cells"),
   or said tumor cells being not transduced ("normal tumor cells"),
   the following ratio :
   immunosuppression index of said mutated ENV protein or of said fragment (i_{mutated env}) / immunosuppression index of wild type ENV protein (i_{wild type env}) is less than 0.5, i_{mutated env} being defined by : (maximum area reached by mutated ENV tumor cells - maximum area reached by normal tumor cells) / (maximum area reached by normal tumor cells), and
   i_{wild type env} being defined by : (maximum area reached by wild type ENV tumor cells - maximum area reached by normal tumor cells) / (maximum area reached by normal tumor cells).

The invention relates to a pharmaceutical composition comprising as active substance :
a) an isolated mutated human or simian lentiviral ENV protein having substantially no immunosuppressive activity, said mutated human or simian lentiviral ENV protein resulting from mutation of the transmembrane (TM) subunit of a wild type human or simian lentiviral ENV protein, said mutated human or simian lentiviral ENV protein having at least 90% identity to one sequence chosen from the group consisting of SEQ ID NO: 216, SEQ ID NO: 420 and SEQ ID NO: 421, said mutated human or simian lentiviral ENV protein comprising a mutated immunosuppressive domain (ISU) containing the following amino acid sequence: A-[I/V]-E-[K/R]-Xa-Xb-X-D-Q) (SEQ ID NO: 416), wherein
   X represents any amino acid,
   and either
   Xₐ is A, F, G, L, R and X_{b} is C, D, E, H, I, K, L, M, N, P, Q, S, T, V, W or Y, or
   Xₐ is C, D, E, H, I, K, M, N, P, Q, S, T, V, W or Y, and X_{b} is A, F, G, R
   or
   Xₐ is A, F, G, L, R and X_{b} is A, F, G, R said mutated human or simian lentiviral ENV protein retains the antigenic structure of the wild type human or simian lentiviral ENV protein
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, F, G, L, R and X_{b} is C, D, E, H, I, K, L, M, N, P, Q, S, T, V, W or Y, or
   Xₐ is C, D, E, H, I, K, M, N, P, Q, S, T, V, W or Y, and X_{b} is A, F, G, R or
   Xₐ is A, F, G, L, R and X_{b} is A, F, G, R said fragment retains the antigenic structure of the full length isolated mutated human or simian lentiviral ENV protein from which it derives
   in association with a pharmaceutically acceptable carrier,
   said substantial absence of immunosuppressive activity of the above mentioned mutated human or simian lentiviral ENV protein or of the above defined fragment being liable to be assessed by the fact that in an *in vivo* assay involving engrafted tumor cells rejection,
   said tumor cells being transduced either so as to express said mutated ENV protein or said fragment ("mutated ENV tumor cells"),
   or said tumor cells being transduced so as to express said wild type ENV protein or a fragment thereof ("wild type ENV tumor cells"),
   or said tumor cells being not transduced ("normal tumor cells"),
   the following ratio :
   immunosuppression index of said mutated ENV protein or of said fragment (i_{mutated env}) / immunosuppression index of wild type ENV protein (i_{wild type env}) is less than 0.5,
   i_{mutated env} being defined by : (maximum area reached by mutated ENV tumor cells - maximum area reached by normal tumor cells) / (maximum area reached by normal tumor cells), and
   i_{wild type env} being defined by: (maximum area reached by wild type ENV tumor cells - maximum area reached by normal tumor cells) / (maximum area reached by normal tumor cells).

The present application is based on the unexpected observation made by the Inventors that some specific amino acids of the immunosuppressive domain of a lentiviral ENV protein can be mutated conferring to said lentiviral ENV protein essentially no immunosuppressive properties, or no immunosuppressive properties, while retaining its antigenicity, the three-dimentional structure of the immunosuppressive domain, and its expression at the plasma membrane. Moreover, the mutated lentiviral ENV protein according to the invention does not alter the infectivity of a virus expressing it.
In the invention, the "mutated simian or human lentiviral ENV proteins" means that the ENV proteins derive from the expression of an *env* gene of a lentivirus of human or simian.

Lentiviruses according to the invention encompassed by the invention are HIV-1 and 2 and Simian immunodeficiency virus (SIV).
Because of the high mutation rate of HIV-1, HIV-2 and SIV viruses, the "mutated ENV protein", as defined in the invention, encompasses two meanings.
According to the first meaning, the said "mutated ENV protein" is the unnatural result of the intervention of human beings.

According to the second meaning, the mutated ENV protein also encompasses naturally occurring variants for which up to now the non immunosuppressive properties remain unknown.
This second meaning takes into consideration the natural variability of HIV and SIV variants inside a same infected individual, wherein the said "mutated ENV protein" might be non immunosuppressive but its property is undetectable because an HIV infected patient always carries many HIV variants, the majority of which is immunosuppressive.

The three following proteins correspond to wild type sequences of the ENV protein of HIV-1, HIV-2 and SIV respectively. In the invention, they are considered as reference sequences of wild type ENV proteins.

| | |
|---|---|
| SEQ ID NO: 417 wild type HIV-1 | |
| SEQ ID NO: 418 Wild type HIV-2 | |
| | |
| SEQ ID NO: 419 wild type SIV mac239 | |

Variant in the invention encompasses SIV, HIV-1 and HIV-2 ENV proteins.

Variants of the HIV-1 mutated ENV proteins according to the invention have at least 90% of identity with the wild type amino acid sequence of the HIV-1 ENV protein, and comprises the mutations as described above, and harbour no immunosuppressive activity.

Variants of the HIV-2 mutated ENV proteins according to the invention have at least 90% of identity with the wild type amino acid sequence of the HIV-2 ENV protein, and comprises the mutations as described above, and harbour no immunosuppressive activity.

Variants of the SIV mutated ENV proteins according to the invention have at least 90% of identity with the wild type amino acid sequence of the SIV ENV protein, and comprises the mutations as described above, and harbour no immunosuppressive activity.

The three following proteins (SEQ ID NO : 216, 420 and 421) correspond to three mutated sequences of the ENV protein of HIV-1, HIV-2 and SIV respectively. In the invention, they are considered as reference sequences of the mutated ENV proteins.

More specifically, SEQ ID NO : 216 corresponds to the SEQ ID NO : 417 in which the amino acid residue Y in position 5 (Xₐ) of the sequence A-[I/V]-E-[K/R]-Xₐ-X_{b}-X-D-Q has been substituted by R.

SEQ ID NO : 420 corresponds to the SEQ ID NO : 418 in which the amino acid residue Y in position 5 (Xₐ) of the sequence A-[I/V]-E-[K/R]-Xₐ-X_{b}-X-D-Q has been substituted by R.

SEQ ID NO : 421 corresponds to the SEQ ID NO : 419 in which the amino acid residue Y in position 5 (Xₐ) of the sequence A-[I/V]-E-[K/R]-Xₐ-X_{b}-X-D-Q has been substituted by R.

| | |
|---|---|
| SEQ ID NO: 216 mutated HIV-1 | |
| SEQ ID NO: 420 mutated HIV-2 | |
| | |
| SEQ ID NO: 421 mutated SIV mac239 | |

The invention also encompasses the variants of the "mutated simian or human lentiviral ENV protein", harbouring the above mentioned mutations, and conferring a lack of immunosuppressive properties to said variant.

Variants of the HIV-1 mutated ENV proteins according to the invention have at least 90% of identity with the reference mutated sequence of HIV-1 ENV protein (SEQ ID NO : 216), and comprises the mutations as described above, and harbour no immunosuppressive activity.

Variants of the HIV-2 mutated ENV proteins according to the invention have at least 90% of identity with the reference mutated sequence of the HIV-2 ENV protein (SEQ ID NO : 420), and comprises the mutations as described above, and harbour no immunosuppressive activity.

Variants of the SIV mutated ENV proteins according to the invention have at least 90% of identity with the reference mutated sequence of the SIV ENV protein (SEQ ID NO : 421), and comprises the mutations as described above, and harbour no immunosuppressive activity.

The immunosuppressive domain (ISU) of the lentivirus according to the invention can be delimited by the sequence SEQ ID NO: 6, xGIVQQQxxLLxxxxxxQxxLxLxxWGxKxLQxRxxA[I/V]E[K/R]YLxDQxxLx (SEQ ID NO: 6)
in which x represents any amino acid.

SEQ ID NO: 6 corresponds to the non mutated ISU domain.

In this SEQ ID NO : 6, "x" (in small letters) is to be considered independently from "X" (in capital letters) used for the first time in SEQ ID NO : 416 of the present invention. SEQ ID NO: 6 comprises SEQ ID NO: 1.

The most advantageous immunosuppressive domains of the wild type ENV proteins according to the invention comprise the following sequences:
HIV-1 ENV protein comprises the amino acid sequence AVERYLKDQ (SEQ ID NO: 7),
HIV-2 ENV protein comprises the amino acid sequence AIEKYLKDQ (SEQ ID NO: 8), and
SIV ENV protein comprises the amino acid sequence SEQ ID NO: 7 or 8.

When applying the mutations as defined above, the ISU domain looses its immunosuppressive properties.

In said SEQ ID NO: 6, the underlined amino acids are the essential amino acids.

In the invention, the ISU domain, as defined by SEQ ID NO: 6, is the minimal essential domain of the ENV protein according to the invention which harbours an immunosuppressive activity.

| Examples of wild type ISU domains: |
|---|
| HIV-1 |
| SGIVQQQNNLLRAIEAQQHLLQLTVWGIKQLQARILAVERYLKDQQLLG (SEQ ID NO: 366) |
| SGIVQQQNNLLRAIEAQQHLLKLTVWGIKQLQARILAVERYLKDQQLLG (SEQ ID NO : 367) |
| SGIVQQQNNLLRAIEAQQHLLQLTVWGIKQLQARVLAVERYLKDQQLLG (SEQ ID NO : 368) |
| SGIVQQQNNLLRAIEAQQHLLQLTVWGIKQLQARILAVERYLKDQQLLG (SEQ ID NO : 369) |
| SGIVQQQNNLLRAIEAQQQMLQLTVWGIKQLRARVLAVERYLRDQQLLG (SEQ ID NO : 370) |
| SGIVQQQSNLLRAIQARQHMLQLTVWGIKQLQARVLAVERYLRDQQLLG (SEQ ID NO : 371) |

| SIV |
|---|
| SGIVQQQNNLLKAIEAQQHLLQLSIWGVKQLQARLLAVERYLQDQQILG (SEQ ID NO : 372) |
| SGIVQQQNNLLRAIEAQQHLLQLSVWGIKQLQARVLAIERYLRDQQILG (SEQ ID NO : 373) |
| AGIVQQQQQLLDVVKRQQELLRLTVWGTKNLQTRVSAIEKYLKDQAQLN (SEQ ID NO: 374) |
| AGIVQQQQQLLDVVKRQQELLRLTVWGTKNLQTRVTAIEKYLKDQAQLN (SEQ ID NO: 375) |

| HIV2 |
|---|
| AGIVQQQQQLLDVVKRQQEMLRLTVWGTKNLQARVTAIEKYLKDQAQLN (SEQ ID NO: 376) |
| AGIVQQQQQLLDVVKRQQELLRLTVWGTKNLQTRVTAIEKYLKDQALLN (SEQ ID NO: 377) |

The localization of an ISU domain can be determined in all ENV proteins of viruses as described in Benit et al. 2001, Journal of Virology, Vol. 75, No. 23, p.11709-11719*.* In a broad meaning, the ISU domain is defined by its structure and its localization, irrespective of the fact that it possesses or not an immunosuppressive activity.
In the invention, the ISU domain refers to a specific domain in which a mutation can affect the immunosuppressive property of the ENV protein.

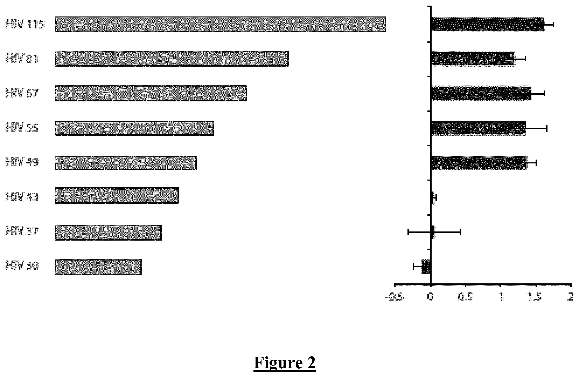

The immunosuppressive property of the ENV protein is preferably measured using *in vivo* procedures, which are representative of the physiological environment.
The immunosuppressive properties of the mutated ENV proteins according to the invention are measured according to an *in vivo* procedure to assay the immunosuppressive activity of a ENV protein disclosed previously [Mangeney and Heidmann Proc Natl Acad Sci USA 1998;95: 14920-14925*;* Mangeney et al. Proc Natl Acad Sci USA, 2007, 104(51):20534-9].
As a physiological test, this *in vivo* procedure is performed using ENV proteins, or fragment thereof, which are not associated to another component or carrier proteins, such as BSA.
Briefly, a wild-type (wild type lentiviral ENV protein) or modified nucleic acid expressing the protein to be tested (mutated lentiviral ENV protein) is transfected in tumour cell lines such as MCA 205 or C18.1 cell lines by known transfection methods. The tumour cells expressing the protein to be tested are then injected especially subcutaneous (s.c.) injection to a host, generally mice. Following said injection, the establishment of tumour or, to the contrary, its rejection, is determined and the tumour area is measured. Tumor establishment was determined by palpation and tumor area (mm²) was determined by measuring perpendicular tumor diameters. Immunosuppression index is defined as i= (Sₑₙᵥ-Sₙₒₙₑ)/Sₙₒₙₑ, wherein Sₑₙᵥ is the maximum area reached by a tumour expressing an envelope protein and Sₙₒₙₑ is the maximum area reached by a tumour not expressing ENV protein (negative control).
According to an embodiment of the invention, the above defined ratio relative to the immunosuppressive index can be less than 0.2, and can even have a negative value (see Figures 2 and 3).

*In vitro* assay could be carried out, using high doses of synthetic peptides but they are indirect and less convincing, since the expression "immunosuppressive" is relevant when applied to animals possessing a complete immune system and not to cell lines.
An additional difficulty for the functional characterization of an ISU domain relies on the fact that the ISU carried by the retroviral ENV proteins is a highly structured proteic domain, with trimer formation within the complete ENV proteins (Caffrey M., Biochimica et Biophysica Acta, 1536:116-122, 2001*;* Caffrey et al., The EMBO Journal, Vol. 17, No. 16, p.4572-4584, 1998*).* Such structures are not naturally formed with ISU peptides of limited length, and this is most probably why most studies carried out with peptides provide irrelevant results and/or are dependent on specific coupling of the peptides to carrier proteins (such as BSA, e.g. Denner et al., Current Science, AIDS 1994, 8:1063-1072*).*
As mentioned above, the ENV proteins according to the invention are mutated. This mutation is made *in vitro.* Thus, the mutated ENV proteins according to the invention are isolated, and does not correspond to naturally occurring counterpart.
As mentioned above, the lentiviral mutated ENV proteins are substantially devoid of immunosuppressive properties. This means that the mutated ENV proteins according to the invention have no, or have reduced immunosuppressive properties with respect to the natural non mutated ENV protein from a virus of the same species. For instance, a mutated HIV ENV protein according to the invention has reduced immunosuppressive properties with respect to the wild type HIV ENV protein.
In the invention, the terms "substantially devoid of immunosuppressive properties" means that the mutated ENV proteins according to the invention have an immunosuppressive index less than about 0.2 [Mangeney and Heidmann Proc Natl Acad Sci USA 1998;95: 14920-14925*;* Mangeney et al. Proc Natl Acad Sci USA, 2007, 104(51):20534-9]*.*
In the invention, structures responsible for the antigenicity of the mutated lentiviral ENV protein are essentially preserved.
As intended herein, the expression "structures responsible for antigenicity" relates to structures of the protein which are liable to interact with components of the immune system such as antibodies or membrane receptors of immune cells, in particular T cells.

The mutation(s) within the immunosuppressive domain of the lentiviral ENV proteins is (are) sufficient to decrease the immunosuppressive activity of the mutated lentiviral ENV protein with respect to the corresponding wild type ENV. However, it might be advantageous that another amino acid be also mutated because it ensures that the structure of the mutated ENV protein is essentially conserved with respect to the corresponding wild type ENV protein.
The mutated lentiviral ENV protein has substantially retained the structure, especially the antigenic structure, e.g., immunogenic determinants, of the original determined lentiviral ENV protein, i.e. the wild type non mutated lentiviral ENV protein.
These properties can be evaluated by measuring the fusion and/or infectious properties of said mutated lentiviral ENV with respect to the same properties in the wild type non mutated lentiviral ENV protein (see example).

Generally speaking, the mutated ENV protein involved in the present invention has an average length of about 700 to about 950 amino acids.

The invention encompasses fragments of the mutated ENV protein as defined above, provided that said fragment:
- comprises at least the sequence SEQ ID NO: 2, as defined above,
- comprises at least 40 amino acids, preferably comprises at least 50 amino acids,
- is substantially devoid of immunosuppressive properties, as defined above,
- preferably, comprises the extracellular parts of the ENV protein,
- retains the structure of the ENV protein from which it derives,
- harbours the same epitopes as the corresponding fragment in the wild type ENV protein.

According to a particular embodiment, the fragment of the mutated ENV protein of the invention can comprise about 40, 50, 100, 150, 200, 250, 300, 350, 400, 450, 500, 550, 600, 650 or 700 amino acids. These values are given only in an illustrative way, as the man skilled in the art will understand that the fragment can comprise any number of amino acids comprised between 40 and 700.

Advantageously, the fragments according to the invention are such that, while retaining the antigenic structure of the full length mutated ENV protein, and thus of the wild type ENV protein, they have lost major antigenic regions that are responsible for antigenicity in another region than the region corresponding to the immunosuppressive domain.

The invention also relates to a pharmaceutical composition wherein the active substance is an isolated non naturally occurring mutated human or simian lentiviral ENV protein having substantially no immunosuppressive activity, as above defined, or fragments thereof.

In a particular embodiment, the invention relates to a pharmaceutical composition as defined above comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid, and either
   Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R,
   in association with a pharmaceutically acceptable carrier.

The invention relates to a pharmaceutical composition as defined above comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, F, G, L or R, and X_{b} is L, I, V, M or P, or
   Xₐ is Y, I, H, C or T, and X_{b} is A, F, G or R, or
   Xₐ is A, F, G, L or R, and X_{b} is A, F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, F, G, L or R, and X_{b} is L, I, V, M or P, or
   Xₐ is Y, I, H, C or T, and X_{b} is A, F, G or R, or
   Xₐ is A, F, G, L or R, and X_{b} is A, F, G or R,
   in association with a pharmaceutically acceptable carrier.

According to a particular embodiment, the invention relates to a pharmaceutical composition as defined above comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, G, or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is F, G or R,
   or
   Xₐ is F or L, and X_{b} is F, G or R,
   or
   Xₐ is A, G or R, and X_{b} is A,
   or
   Xₐ is A, G or R, and X_{b} is F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, G, or R, and X_{b} is C, D, E, H, I, K, L, M, N, P, Q, S, T, V, W or Y,
   or
   Xₐ is C, D, E, H, I, K, M, N, P, Q, S, T, V, W or Y, and X_{b} is F, G or R,
   or
   Xₐ is F or L, and X_{b} is F, G or R,
   or
   Xₐ is A, G or R, and X_{b} is A,
   or
   Xₐ is A, G or R, and X_{b} is F, G, R,
   in association with a pharmaceutically acceptable carrier.

According to a particular embodiment, the invention relates to a pharmaceutical composition as defined above comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, G, or R, and X_{b} is L, I, V, M or P,
   or
   Xₐ is Y, I, H, C or T, and X_{b} is F, G or R,
   or
   Xₐ is F or L, and X_{b} is F, G or R,
   or
   Xₐ is A, G or R, and X_{b} is A,
   or
   Xₐ is A, G or R, and X_{b} is F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein
   X represents any amino acid,
   and either
   Xₐ is A, G, or R, and X_{b} is L, I, V, M or P,
   or
   Xₐ is Y, I, H, C or T, and X_{b} is F, G or R,
   or
   Xₐ is F or L, and X_{b} is F, G or R,
   or
   Xₐ is A, G or R, and X_{b} is A,
   or
   Xₐ is A, G or R, and X_{b} is F, G or R,
   in association with a pharmaceutically acceptable carrier.

According to another particular embodiment, the invention also relates to a pharmaceutical composition as defined above comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, G, or R, and X_{b} is C, D, E, H, I, K, L, M, N, P, Q, S, T, V, W or Y,
   or
   Xₐ is C, D, E, H, I, K, M, N, P, Q, S, T, V, W or Y, and X_{b} is F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, G, or R, and X_{b} is C, D, E, H, I, K, L, M, N, P, Q, S, T, V, W or Y,
   or
   Xₐ is C, D, E, H, I, K, M, N, P, Q, S, T, V, W or Y, and X_{b} is F, G or R,
   in association with a pharmaceutically acceptable carrier.
According to another particular embodiment, the invention relates to a pharmaceutical composition as defined above, comprising as active substance :
a) an isolated mutated human or simian lentiviral ENV protein having substantially no immunosuppressive activity,
   said mutated human or simian lentiviral ENV protein resulting from mutation of the transmembrane (TM) subunit of a wild type human or simian lentiviral ENV protein,
   said mutated human or simian lentiviral ENV protein comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, G, or R, and X_{b} is L, I, V, M or P,
   or
   Xₐ is Y, I, H, C or T, and X_{b} is F, G or R,
   or
b) at least one fragment of said isolated mutated human or simian lentiviral ENV protein having substantially no immunosuppressive activity,
   said fragment comprising at least 40 amino acids,
   said fragment comprising a mutated immunosuppressive domain (ISU) containing the following amino acid sequence:
   A-[I/V]-E-[K/R]-Xₐ-X_{b}-X-D-Q (SEQ ID NO: 416),
   wherein,
   X represents any amino acid,
   and either
   Xₐ is A, G, or R, and X_{b} is L, I, V, M or P,
   or
   Xₐ is Y, I, H, C or T, and X_{b} is F, G or R,
   in association with a pharmaceutically acceptable carrier.

According to another particular embodiment, the invention relates to a pharmaceutical composition as defined above,
wherein
Xₐ is R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is R, or
Xₐ is R, and X_{b} is R.
According to another particular embodiment, the invention relates to a pharmaceutical composition as defined above, wherein said isolated mutated human or simian lentiviral ENV protein or said fragment of said isolated mutated human or simian lentiviral ENV protein comprises one of the following amino acid sequences :
A-I-E-K-Xₐ-X_{b}-X-DQ (SEQ ID NO: 422),
A-I-E-R-Xₐ-X_{b}-X-DQ (SEQ ID NO: 423),
A-V-E-K-Xₐ-X_{b}-X-DQ (SEQ ID NO: 424),
A-V-E-R-Xₐ-X_{b}-X-DQ (SEQ ID NO: 425).

In one advantageous embodiment, the invention relates to a pharmaceutical composition as defined above, wherein the resulting immunosuppressive domain, contained in the mutated lentiviral protein, comprises the amino acid sequence of the list consisting of: SEQ ID NO :9 to SEQ ID NO :37.
In the invention "SEQ ID NO: 9 to SEQ ID NO :37" encompass SEQ ID NO : 9, SEQ ID NO: 10, SEQ ID NO: 11, SEQ ID NO: 12, SEQ ID NO: 13, SEQ ID NO: 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO : 32, SEQ ID NO : 33, SEQ ID NO: 34, SEQ ID NO: 35, SEQ ID NO: 36 and SEQ ID NO: 37.

The correspondence is the following one:

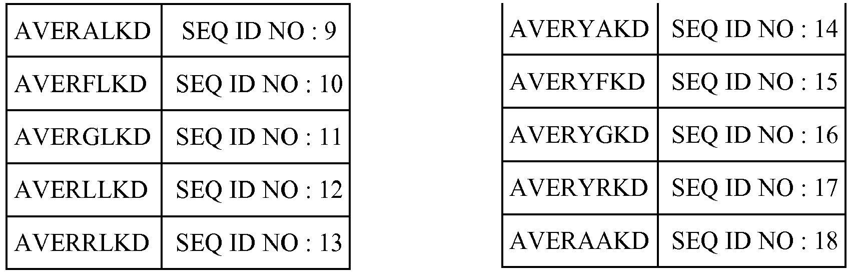

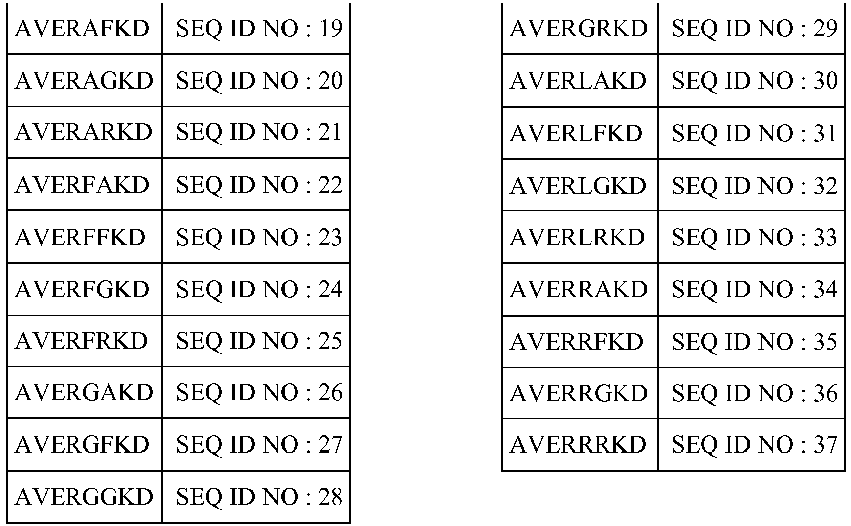

In one advantageous embodiment, the invention relates to a pharmaceutical composition as defined above, wherein the resulting immunosuppressive domain, contained in the mutated lentiviral protein, comprises the amino acid sequence of the list consisting of: SEQ ID NO :38 to SEQ ID NO :66.
In the invention "SEQ ID NO : 38 to SEQ ID NO :66" encompass SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, SEQ ID NO : 46, SEQ ID NO : 47, SEQ ID NO : 48, SEQ ID NO : 49, SEQ ID NO : 50, SEQ ID NO : 51, SEQ ID NO : 52, SEQ ID NO : 53, SEQ ID NO : 54, SEQ ID NO : 55, SEQ ID NO : 56, SEQ ID NO : 57, SEQ ID NO : 58, SEQ ID NO : 59, SEQ ID NO : 60, SEQ ID NO : 61, SEQ ID NO : 62, SEQ ID NO : 63, SEQ ID NO: 64, SEQ ID NO: 65 and SEQ ID NO: 66.

The correspondence is the following one:

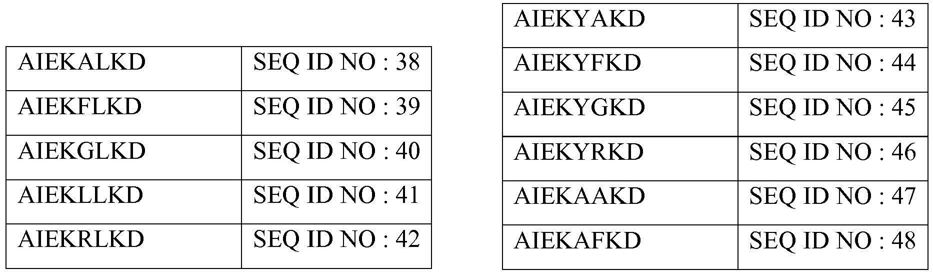

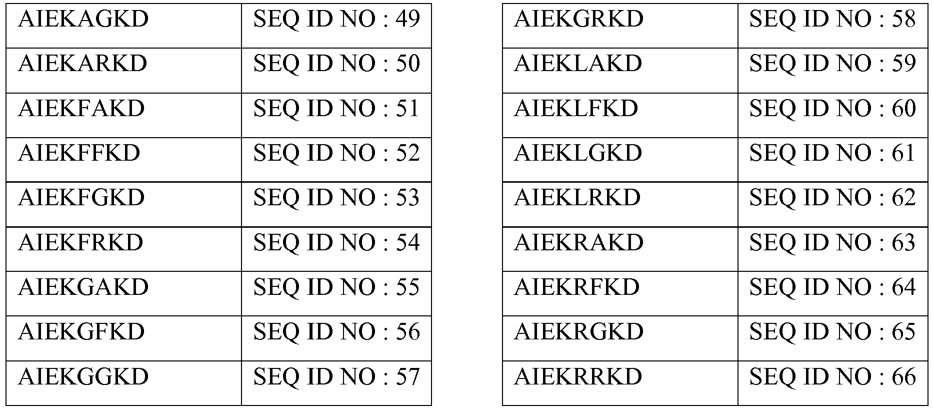

In one advantageous embodiment, the invention relates to a pharmaceutical composition as defined above, wherein the resulting immunosuppressive domain, contained in the mutated lentiviral protein, comprises the amino acid sequence of the list consisting of: SEQ ID NO : 67 to SEQ ID NO: 95.
In the invention "SEQ ID NO : 67 to SEQ ID NO :95" encompass SEQ ID NO : 67, SEQ ID NO : 68, SEQ ID NO : 69, SEQ ID NO : 70, SEQ ID NO : 71, SEQ ID NO : 72, SEQ ID NO : 73, SEQ ID NO : 74, SEQ ID NO : 75, SEQ ID NO : 76, SEQ ID NO : 77, SEQ ID NO : 78, SEQ ID NO : 79, SEQ ID NO : 80, SEQ ID NO : 81, SEQ ID NO : 82, SEQ ID NO : 83, SEQ ID NO : 84, SEQ ID NO : 85, SEQ ID NO : 86, SEQ ID NO : 87, SEQ ID NO: 88, SEQ ID NO: 89, SEQ ID NO: 90, SEQ ID NO: 91, SEQ ID NO: 92, SEQ ID NO: 93, SEQ ID NO: 94 and SEQ ID NO: 95.

The correspondence is the following one:

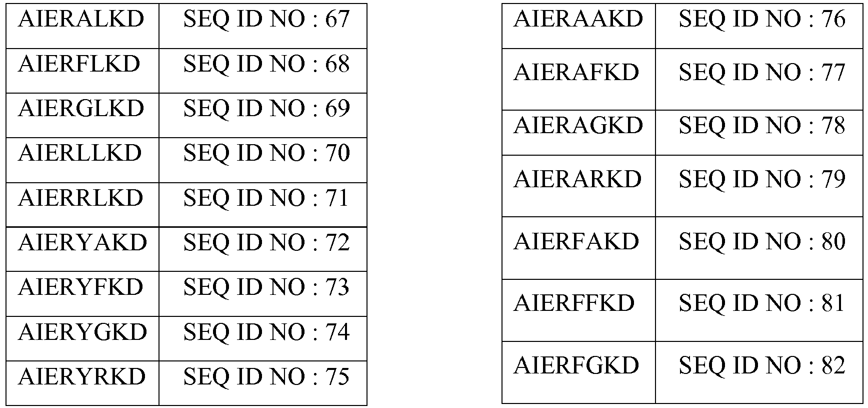

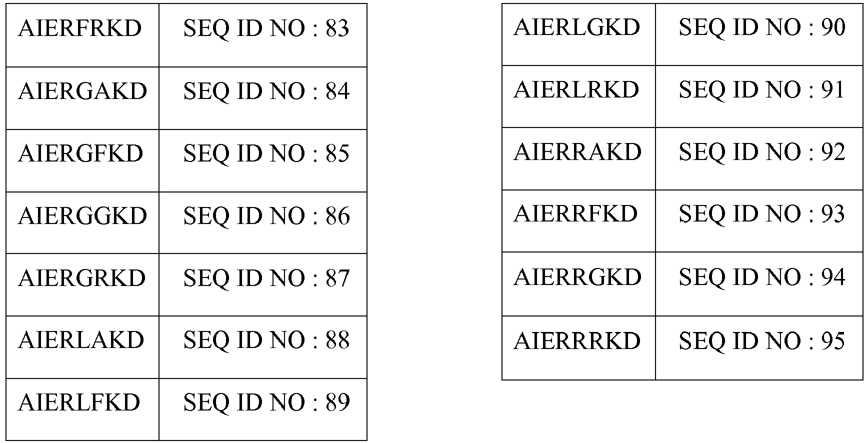

As mentioned above, the previous ENV proteins having their ISU comprising the above sequence are devoid of immunosuppressive properties.
Thus, in other words, any ENV protein of human or simian lentivirus having in their ISU an amino acid sequence comprising of the sequences SEQ ID NO: 9 to 95, is devoid of immunosuppressive properties.
In other words, a simian or human lentiviral ENV protein comprising, within its ISU domain, an amino acid sequence selected from SEQ ID NO: 9 to 95 is devoid of immunosuppressive properties.

In particular, the invention relates to a pharmaceutical composition as defined above, wherein said isolated mutated human or simian lentiviral ENV protein or said fragment of said isolated mutated human or simian lentiviral ENV protein comprises one of the amino acid sequences :
SEQ ID NO : 13, SEQ ID NO : 42, SEQ ID NO : 71,
SEQ ID NO: 9 to 12,
SEQ ID NO: 14 to 41,
SEQ ID NO: 43 to 70, and
SEQ ID NO: 72 to 95.

In particular, the invention relates to a pharmaceutical composition as defined above, wherein said isolated mutated human or simian lentiviral ENV protein or said fragment of said isolated mutated human or simian lentiviral ENV protein comprises one of the amino acid sequences :
SEQ ID NO : 13, SEQ ID NO : 42, SEQ ID NO : 71,
SEQ ID NO: 9, 11, 15 to 21, 23 to 29, 31 to 38, 40, 44 to 50, 52 to 58, 60 to 67, 69, 73 to 79, 81 to 87, 89 to 95.

In the invention, the fragments of the mutated ENV proteins according to the invention comprise or consist of the following sequences: SEQ ID NO: 67 to 211.
These fragments are also devoid of immunosuppressive properties.

However, these fragments retain the structure and the antigenicity of the corresponding immunosuppressive domain that is not mutated, i.e. the wild type immunosuppressive domain.
In the invention, the fragments of the mutated ENV proteins according to the invention comprise or consist of the following sequences: SEQ ID NO: 96 to 211.

The correspondences are as follows

| | |
|---|---|
| | SEQ ID NO: 96 |
| | SEQ ID NO: 97 |
| | SEQ ID NO: 98 |
| | SEQ ID NO: 99 |
| | SEQ ID NO: 100 |
| | SEQ ID NO: 101 |
| | SEQ ID NO: 102 |
| | SEQ ID NO: 103 |
| | SEQ ID NO: 104 |
| | SEQ ID NO: 105 |
| | SEQ ID NO: 106 |
| | SEQ ID NO: 107 |
| | SEQ ID NO: 108 |
| | SEQ ID NO: 109 |
| | SEQ ID NO: 110 |
| | SEQ ID NO: 111 |
| | SEQ ID NO: 112 |
| | SEQ ID NO: 113 |
| | SEQ ID NO: 114 |
| | SEQ ID NO: 115 |
| | SEQ ID NO: 116 |
| | SEQ ID NO: 117 |
| | SEQ ID NO : 118 |
| | SEQ ID NO: 119 |
| | SEQ ID NO: 120 |
| | SEQ ID NO: 121 |
| | SEQ ID NO: 122 |
| | SEQ ID NO: 123 |
| | SEQ ID NO: 124 |

| | |
|---|---|
| | SEQ ID NO : 125 |
| | SEQ ID NO : 126 |
| | SEQ ID NO : 127 |
| | SEQ ID NO : 128 |
| | SEQ ID NO : 129 |
| | SEQ ID NO : 130 |
| | SEQ ID NO: 131 |
| | SEQ ID NO : 132 |
| | SEQ ID NO : 133 |
| | SEQ ID NO : 134 |
| | SEQ ID NO : 135 |
| | SEQ ID NO : 136 |
| | SEQ ID NO: 137 |
| | SEQ ID NO : 138 |
| | SEQ ID NO: 139 |
| | SEQ ID NO: 140 |
| | SEQ ID NO : 141 |
| | SEQ ID NO: 142 |
| | SEQ ID NO: 143 |
| | SEQ ID NO: 144 |
| | SEQ ID NO: 145 |
| | SEQ ID NO: 146 |
| | SEQ ID NO : 147 |
| | SEQ ID NO: 148 |
| | SEQ ID NO: 149 |
| | SEQ ID NO: 150 |
| | SEQ ID NO: 151 |
| | SEQ ID NO: 152 |
| | SEQ ID NO: 153 |

| | |
|---|---|
| | SEQ ID NO: 154 |
| | SEQ ID NO: 155 |
| | SEQ ID NO: 156 |
| | SEQ ID NO: 157 |
| | SEQ ID NO : 158 |
| | SEQ ID NO: 159 |
| | SEQ ID NO: 160 |
| | SEQ ID NO: 161 |
| | SEQ ID NO: 162 |
| | SEQ ID NO: 163 |
| | SEQ ID NO: 164 |
| | SEQ ID NO: 165 |
| | SEQ ID NO: 166 |
| | SEQ ID NO: 167 |
| | SEQ ID NO: 168 |
| | SEQ ID NO: 169 |
| | SEQ ID NO: 170 |
| | SEQ ID NO: 171 |
| | SEQ ID NO: 172 |
| | SEQ ID NO: 173 |
| | SEQ ID NO: 174 |
| | SEQ ID NO: 175 |
| | SEQ ID NO: 176 |
| | SEQ ID NO: 177 |
| | SEQ ID NO: 178 |
| | SEQ ID NO : 179 |
| | SEQ ID NO: 180 |
| | SEQ ID NO: 181 |
| | SEQ ID NO: 182 |
| | SEQ ID NO: 183 |
| | SEQ ID NO: 184 |
| | SEQ ID NO: 185 |
| | SEQ ID NO: 186 |
| | SEQ ID NO: 187 |
| | SEQ ID NO: 188 |
| | SEQ ID NO: 189 |
| | SEQ ID NO: 190 |
| | SEQ ID NO : 191 |
| | SEQ ID NO: 192 |
| | SEQ ID NO: 193 |
| | SEQ ID NO: 194 |
| | SEQ ID NO: 195 |
| | SEQ ID NO: 196 |
| | SEQ ID NO: 197 |
| | SEQ ID NO: 198 |
| | SEQ ID NO: 199 |
| | SEQ ID NO: 200 |
| | SEQ ID NO: 201 |
| | SEQ ID NO: 202 |
| | SEQ ID NO: 203 |
| | SEQ ID NO: 204 |
| | SEQ ID NO: 205 |
| | SEQ ID NO: 206 |
| | |
| | SEQ ID NO: 207 |
| | SEQ ID NO: 208 |
| | SEQ ID NO: 209 |
| | SEQ ID NO : 210 |
| | SEQ ID NO: 211 |

The pharmaceutical composition according to the disclosure may comprise an isolated mutated human or simian lentiviral ENV protein or said fragment of said isolated mutated human or simian lentiviral ENV protein comprises one of the amino acid sequences : SEQ ID NO : 96, 98, 100, 102 to 108, 110 to 116, 118 to 125, 127, 129, 131 to 137, 139 to 145, 147 to 154, 156, 158, 160 to 166, 168 to 174, 176 to 183, 185, 187, 189 to 195, 197 to 203, 205 to 211.

Advantageously, the invention relates to the pharmaceutical composition as defined above, wherein said mutated protein consists of one of the following sequences SEQ ID NO : 212 to 269

**HIV-1 LAI**

| | |
|---|---|
| | SEQ ID NO : 212 |
| | |
| | SEQ ID NO : 213 |
| | SEQ ID NO : 214 |
| | SEQ ID NO : 215 |
| | |
| | SEQ ID NO : 216 |
| | SEQ ID NO : 217 |
| | |
| | SEQ ID NO : 218 |
| | SEQ ID NO : 219 |
| | |
| | SEQ ID NO: 220 |
| | SEQ ID NO : 221 |
| | SEQ ID NO: 222 |
| | |
| | SEQ ID NO: 223 |
| | SEQ ID NO: 224 |
| | |
| | SEQ ID NO: 225 |
| | SEQ ID NO: 226 |
| | |
| | SEQ ID NO: 227 |
| | SEQ ID NO: 228 |
| | |
| | SEQ ID NO: 229 |
| | SEQ ID NO: 230 |
| | SEQ ID NO : 231 |
| | |
| | SEQ ID NO: 232 |
| | SEQ ID NO: 233 |
| | |
| | SEQ ID NO: 234 |
| | SEQ ID NO: 235 |
| | |
| | SEQ ID NO: 236 |
| | SEQ ID NO: 237 |
| | SEQ ID NO : 238 |
| | |
| | SEQ ID NO: 239 |
| | SEQ ID NO: 240 |
| | |

**HIV-BH10**

| | |
|---|---|
| | SEQ ID NO: 241 |
| | SEQ ID NO: 242 |
| | |
| | SEQ ID NO: 243 |
| | SEQ ID NO: 244 |
| | |
| | SEQ ID NO: 245 |
| | SEQ ID NO: 246 |
| | SEQ ID NO: 247 |
| | |
| | SEQ ID NO : 248 |
| | SEQ ID NO : 249 |
| | |
| | SEQ ID NO : 250 |
| | SEQ ID NO : 251 |
| | |
| | SEQ ID NO : 252 |
| | SEQ ID NO : 253 |
| | |
| | SEQ ID NO : 254 |
| | SEQ ID NO : 255 |
| | SEQ ID NO : 256 |
| | |
| | SEQ ID NO : 257 |
| | SEQ ID NO : 258 |
| | |
| | SEQ ID NO : 259 |
| | SEQ ID NO : 260 |
| | |
| | SEQ ID NO : 261 |
| | SEQ ID NO : 262 |
| | SEQ ID NO : 263 |
| | |
| | SEQ ID NO : 264 |
| | SEQ ID NO : 265 |
| | |
| | SEQ ID NO : 266 |
| | SEQ ID NO : 267 |
| | |
| | SEQ ID NO : 268 |
| | SEQ ID NO : 269 |
| | |

The disclosure also encompasses the variants of the above sequences, harbouring the above mentioned mutations, and conferring to said variant a lack of immunosuppressive properties.

In particular, the invention relates to a pharmaceutical composition as defined above, wherein said isolated mutated human or simian lentiviral ENV protein consists of one of the amino acid sequences : SEQ ID NO : 212, 214, 216, 218 to 224, 226 to 232, 234 to 241, 243, 245, 247 to 253, 255 to 261, 263 to 269.

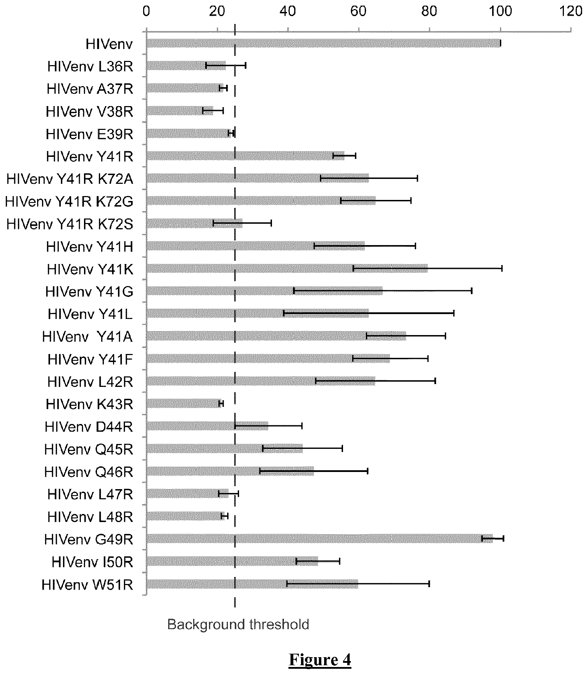

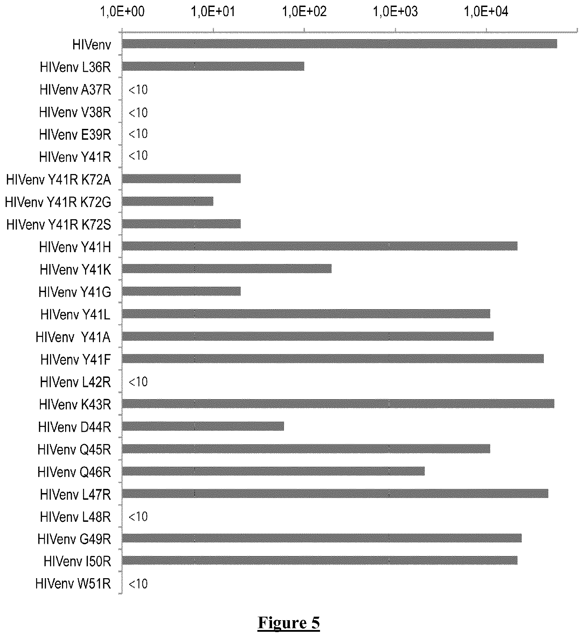

The invention also relates to a pharmaceutical composition as defined above, wherein said mutated protein comprises additional mutations either downstream the C-terminal end of the sequence SEQ ID NO : 416 or upstream the N-terminal end of SEQ ID NO : 416. These additional mutations can be advantageous to maintain the three-dimensional structure of the immunosuppressive domain and of the ENV protein (see details concerning the membrane expression of the ENV protein [see figure 4 and example] and concerning infectivity [see figure 5 and example]).

In a particular embodiment, the invention relates to a pharmaceutical composition as defined above, wherein said mutated protein comprises an additional mutation in one at least of the amino acids at positions 29, 36 and 37 of SEQ ID NO: 426 : A[I/V]E[K/R]XₐX_{b}X₁DQX₂X₃LX₄X₅WGC[A/S][F/G]X₆X₇CVX₈TX₉VPX_{c}X₁₀Z₁Z₂Z₃Z₄Z₅ X_{d}Xₑ[S/T](SEQ ID NO: 426)
wherein
Xₐ and X_{b} are as defined above,
X₁ to X₁₀ represent any amino acid,
Z₁ to Z₅ represent no amino acid or any amino acid, independently from each other such that
   ∘ X_{c} is A, C, D, E, F, G, H, I, K, L, M, N, P, Q, R, S, T, V, Y, preferably A, D, or N,
   ∘ X_{d} is A, C, D, E, F, G, H, I, L, M, N, P, Q, R, S, T, V, Y, W, preferably A, G, S or Y,
   ∘ Xₑ is A, C, D, E, F, G, H, I, K, L, M, N, P, Q, R, T, V, Y, W, preferably A, D or N.

This sequence contains the following amino acid sequence A[I/V]E[K/R]XₐX_{b}X₁DQ (SEQ ID NO : 416) elongated on its C-terminal end in which additional mutations are present.

It is to be noted that "X₁" in the above mentioned sequence SEQ ID NO : 426 has the same meaning as "X" in SEQ ID NO : 416.

In one advantageous embodiment, the invention relates to a pharmaceutical composition as defined above, said mutated protein comprising the amino acid sequences of the group consisting of SEQ ID NO: 271 to SEQ ID NO: 283.
SEQ ID NO: 271, contains the mutations Y41R, K72A:
SEQ ID NO: 272, contains the mutations Y41R, K72G:
SEQ ID NO: 273, contains the mutations Y41R, K72S:
SEQ ID NO: 274, contains the mutations Y41R, W65D:
SEQ ID NO: 275, contains the mutations Y41R, W65A:
SEQ ID NO: 276, contains the mutations Y41R, W65N:
SEQ ID NO: 277, contains the mutations Y41R, S73D:
SEQ ID NO: 278, contains the mutations Y41R, S73A:
SEQ ID NO: 279, contains the mutations Y41R, S73N:
SEQ ID NO: 280, contains the mutations Y41R, K72Y:
SEQ ID NO: 281, contains the mutations R40A, Y41R, K72A:
SEQ ID NO: 282, contains the mutations Y41R, K72A, S73A:
SEQ ID NO: 283, contains the mutations Y41R, W65A, K72A, S73A:

The mutations in the above mutated peptides are indicated by the amino acid residues which are bolded and underlined.

Also disclosed is a pharmaceutical composition as defined above, comprising an additional mutation of one at least of the amino acids X₁₁, X₁₂, X₁₃ and X₁₄ in the following sequence (containing the above defined SEQ ID NO: 416 sequence) :
X₁₁X₁₂ X₁₃X₁₄I LA[I/V]E[K/R]XₐX_{b}X₁DQ (SEQ ID NO: 428),
wherein
X₁ represents any amino acid,
Xa and Xb are as defined above,
X₁₁ is :
- either deleted,
- or A, C, D, E, F, G, H, I, K, M, N, P, Q, R, S, T, V, Y or W, in particular A, G or R,
and/or
X₁₂ is :
- either deleted,
- or A, C, D, E, F, G, H, I, K, L, M, N, P, R, S, T, V, Y or W, in particular A, G or R,
and/or
X₁₃ is :
- either deleted,
- or C, D, E, F, G, H, I, K, L, M, N, P, Q, R, S, T, V, Y or W, in particular R or G,
and/or
X₁₄ is :
- either deleted,
- or A, C, D, E, F, G, H, I, K, L, M, N, P, Q, S, T, V, Y or W, in particular A or G.

This sequence contains the following amino acid sequence A[I/V]E[K/R]XₐX_{b}X₁DQ (SEQ ID NO : 416) elongated on its N-terminal end in which additional mutations are present.

It is to be noted that "X₁" in SEQ ID NO : 428 has exactly the same meaning as "X" in SEQ ID NO : 416.

In another advantageous embodiment, the invention relates to a pharmaceutical composition as defined above, wherein said mutated lentiviral protein, said variant or said fragment harbour a three-dimensional structure similar to the structure of the natural non mutated lentiviral ENV protein, non mutated lentiviral ENV variant or non mutated lentiviral ENV fragment thereof.

The skilled person knows how to measure the antigenicity, by using standard proceedings.

The disclosure also provides a pharmaceutical composition as defined above, wherein said mutated lentiviral protein, said variant or said fragment are expressed at the plasma membrane at a level substantially identical to the expression at the plasma membrane of the natural non mutated lentiviral ENV protein, non mutated lentiviral ENV variant or non mutated lentiviral ENV fragment thereof.

The membrane expression of the lentiviral ENV protein can be measured by any techniques allowing determination of a plasma membrane protein. For instance, cells can be transfected with an expression vector allowing the expression of the mutated lentiviral ENV protein according to the disclosure. Cells are then incubated with an antibody recognizing specifically the extracellular part of said lentiviral mutated ENV protein. The complex (antibody/ENV protein) is detected by another antibody, and the complex can be quantified by flow cytometry (see for instance figure 4, and example).

If no complex is detected, the mutated ENV protein is not expressed at the plasma membrane. On the contrary, if the protein is expressed, this means that the mutated ENV protein is expressed at the plasma membrane.

Also disclosed is a pharmaceutical composition as defined above, wherein said mutated lentiviral ENV protein is such that a lentivirus, or a pseudotype, expressing said mutated lentiviral ENV protein, instead of the non mutated ENV protein, has a viral titer similar to the viral titer of said lentivirus, or pseudotype, expressing the non mutated lentiviral protein.

Viral titer is measured according to a commonly used protocol, and as described in the example (see figure 5).

A pharmaceutical composition as defined above may comprise a mutated lentiviral ENV protein which is a HIV-1 lentiviral protein consisting of the amino acid sequences SEQ ID NO: 284 to SEQ ID NO: 292.

| | |
|---|---|
| SEQ ID NO: 284 Y41R | |
| | |
| SEQ ID NO: 285 Y41R, K72A | |
| SEQ ID NO: 286 Y41R, K72G | |
| SEQ ID NO: 287 Y41R, K72S | |
| | |
| SEQ ID NO: 288 Y41G | |
| SEQ ID NO: 289 Y41L | |
| | |
| SEQ ID NO: 290 Y41A | |
| SEQ ID NO: 291 Y41F | |
| SEQ ID NO: 292 L42R | |
| | |

Also, the following proteins are advantageous in the invention:

| | |
|---|---|
| SEQ ID NO: 293 SIV MM251 L42R | |

| | |
|---|---|
| SEQ ID NO: 294 HIV2 L42R | |
| | |

Advantageously: the mutated lentiviral proteins are
1- SEQ ID NO: 284 to 294; these mutated ENV proteins having substantially no immunosuppressive properties
   advantageously,
2- SEQ ID NO: 284 to SEQ ID NO: 292; these mutated ENV proteins having substantially no immunosuppressive properties and being highly expressed at the plasma membrane, more advantageously,
3- SEQ ID NO: 284 to SEQ ID NO: 291; these mutated ENV proteins having substantially no immunosuppressive properties, being highly expressed at the plasma membrane and conferring to a virus expressing them a medium or high viral titer,
   in particular
4- Y41F (SEQ ID NO: 291), Y41L (SEQ ID NO: 289), Y41A (SEQ ID NO: 290); these mutated ENV proteins having substantially no immunosuppressive properties, being highly expressed at the plasma membrane and confers to a virus expressing them a high viral titer.

As mentioned above "confering to a virus expressing them a medium or high viral titer" means that, when the sequence of the wild type ENV protein is substituted by the sequence of the mutated ENV in the lentiviral or pseudotype retrovirus, the virus thus expresses a mutated lentiviral ENV protein, along with other wild type viral proteins (GAG, PRO, POL), said mutated ENV protein conferring to the virus the ability to enter in its target cell:
- either at a level comparable or higher to the ability of the wild type virus, i.e. high ability
- or at a lower level compared to the ability of the wild type virus, i.e. medium or low ability.

The ability to enter in the target cell can be measured by the viral load. Viral load is a measure of the amount of target cells that can be infected by a given amount (1 mL) of virus (see figure 5 and example).

The invention also relates to a pharmaceutical composition comprising a nucleic acid molecule coding for a mutated lentiviral ENV protein, or variant of said protein, or fragments thereof, as defined above, in association with a pharmaceutically acceptable carrier.

The nucleic acid may be comprised in a vector, said vector comprising means allowing the expression of said mutated lentiviral ENV protein, or variant of said protein, or fragments thereof.

If the nucleic acid is comprised in a vector, said nucleic acid may be placed under the control of sequences that allow the expression of said mutated lentiviral ENV protein, or variant of said protein, or fragments thereof.

A pharmaceutical composition, as defined above, in particular as a vaccine, comprises a DNA molecule coding for said mutated lentiviral ENV protein, or variant of said protein, or fragments thereof.

DNA vaccines expressing ENV proteins can be produced as described in Bellier et al., (DNA vaccines expressing retrovirus-like particles are efficient immunogens to induce neutralizing antibodies, Vaccine, 27(42):5772-80, 2009).

In a pharmaceutical composition as defined above, said vector may be chosen among an eukaryotic or prokaryotic expression vector , in particular an eukaryotic vector which is a viral vector, in particular a pox vector, such as a fowlpox, a canarypox, or a MVA (modified vaccinia virus Ankara) vector, an adenoviral vector, a lentiviral vector, a measles vector, a Sendai virus or a CMV (cytomegalovirus) vector.

A pharmaceutical composition as defined above, may further comprise at least one nucleotide molecule coding for a GAG and/or a PRO and/or a POL protein and/or a mutated NEF protein substantially devoid of immunosuppressive properties, of a lentivirus, preferably a lentivirus of the same origin as the mutated lentiviral ENV protein.

The advantageous mutated Nef protein contains a substitution at position 93, as described in the international application WO 2006/018289 (Inventors : Renard M., Mangeney M. and Heidmann T.) and a deletion of the N-terminus of the protein involved in its myristoylation.

GAG expression will produce virus like particles (VLPs) which are particularly advantageous for a vaccine, in particular if the ENV protein is associated with the VLP (Guerbois et al., Virology, 388:191-203, 2009).

In a pharmaceutical composition, as defined above, said nucleic acid molecule coding for a mutated human or simian lentiviral ENV protein, or a fragment of said mutated human or simian lentiviral ENV protein, may be contained in the same vector as the one which also contains said at least one nucleic acid molecule coding for a GAG protein and/or a PRO protein and/or a POL protein and/or a mutated NEF protein, wherein said mutated NEF protein is substantially devoid of immunosuppressive properties.

In a pharmaceutical composition, as defined above, said nucleic acid molecule coding for a mutated human or simian lentiviral ENV protein, or a fragment of said mutated human or simian lentiviral ENV protein, may be contained in the same vector as the one which also contains all said at least one nucleic acid molecule coding for a GAG protein and/or a PRO protein and/or a POL protein and/or a mutated NEF protein, wherein said mutated NEF protein is substantially devoid of immunosuppressive properties.

A pharmaceutical composition, as defined above, may have a nucleic acid molecule coding for a mutated human or simian lentiviral ENV protein, or a fragment of said mutated human or simian lentiviral ENV protein, which is contained in a vector which is different from the at least one vector containing said at least one nucleic acid molecule coding for a GAG protein and/or a PRO protein and/or a POL protein and/or a mutated NEF protein, wherein said mutated NEF protein is substantially devoid of immunosuppressive properties.

In the pharmaceutical composition, as defined above, said nucleic acid molecule coding for a mutated human or simian lentiviral ENV protein, or a fragment of said mutated human or simian lentiviral ENV protein, said nucleic acid molecule coding for a GAG protein, said nucleic acid molecule coding for a PRO protein, said nucleic acid molecule coding for a POL protein and said nucleic acid molecule coding for a mutated NEF protein substantially devoid of immunosuppressive properties, may be all contained in vectors which are different from each other.

A pharmaceutical composition, as defined above, may comprise at least one nucleic acid molecule coding for a GAG protein and/or a mutated NEF protein, wherein said mutated NEF protein is substantially devoid of immunosuppressive properties,
of a human or simian lentivirus, said lentivirus being preferably of the same origin as the mutated lentiviral ENV protein.

In a pharmaceutical composition, as defined above, said nucleic acid molecule coding for a mutated human or simian lentiviral ENV protein, or a fragment of said mutated human or simian lentiviral ENV protein, may be contained in the same vector as the one which also contains said at least one nucleic acid molecule coding for a GAG protein and/or a mutated NEF protein, wherein said mutated NEF protein is substantially devoid of immunosuppressive properties.

In a pharmaceutical composition, as defined above, said nucleic acid molecule coding for a mutated human or simian lentiviral ENV protein, or a fragment of said mutated human or simian lentiviral ENV protein, may be contained in the same vector as the one which also contains a nucleic acid molecule coding for a GAG protein and for a mutated NEF protein, wherein said mutated NEF protein is substantially devoid of immunosuppressive properties.

In a pharmaceutical composition, as defined above, said nucleic acid molecule coding for a mutated human or simian lentiviral ENV protein, or a fragment of said mutated human or simian lentiviral ENV protein, may be contained in the same vector as the one which also contains a nucleic acid molecule coding for a GAG protein and for a mutated NEF protein, wherein said mutated NEF protein is substantially devoid of immunosuppressive properties,
said vector being preferably a measles vector (Guerbois et al., Virology, 388:191-203, 2009) or a canary pox vector (Poulet et al., Veterinary Record, 153(5):141-145, 2003 ; Vaccari et al., Expert Review of Vaccines, Vol. 9, No 9, pages 997-1005, 2010).

In a pharmaceutical composition, as defined above, said nucleic acid molecule coding for a mutated human or simian lentiviral ENV protein, or a fragment of said mutated human or simian lentiviral ENV protein, may be contained in a vector which is different from the at least one vector containing said at least one nucleic acid molecule coding for a GAG protein and/or a mutated NEF protein, wherein said mutated NEF protein is substantially devoid of immunosuppressive properties.

In a pharmaceutical composition, as defined above, said nucleic acid molecule coding for a mutated human or simian lentiviral ENV protein, or a fragment of said mutated human or simian lentiviral ENV protein, said nucleic acid molecule coding for a GAG protein and said nucleic acid molecule coding for a mutated NEF protein substantially devoid of immunosuppressive properties, may be all contained in vectors which are different from each other.

The invention also relates to a pharmaceutical composition according the above definition, for its use as vaccine, for the treatment or the prevention of lentiviral infection, in particular HIV-1 infection, HIV-2 infection and SIV infection.

As mentioned above the pharmaceutical composition according to the invention encompasses a vaccine, comprising as active substance, a protein as defined above, or a nucleic acid as defined above, or a vector as defined above, or a combination thereof.

The pharmaceutical composition may encompass a vaccine, comprising a nucleic acid coding for a GAG protein as defined above, a nucleic acid coding for a mutated ENV protein as defined above and a nucleic acid coding for a mutated NEF protein as defined above.

The pharmaceutical composition encompassing a vaccine as defined above, may comprise a nucleic acid coding for a GAG protein as defined above, a nucleic acid coding for a mutated ENV protein as defined above and a nucleic acid coding for a mutated NEF protein as defined above, which are contained in a same vector, said same vector being preferably a canary pox vector or a measles vector, more preferably a measles vector.

The pharmaceutical composition encompassing a vaccine, may comprise a GAG protein as defined above, a mutated ENV protein as defined above and a mutated NEF protein as defined above.

The pharmaceutical composition encompassing a vaccine, may comprise a GAG protein as defined above, a mutated ENV protein as defined above and a mutated NEF protein as defined above which are associated to at least one adjuvant.

A comprehensive list of adjuvants that can be used in HIV vaccines is indicated in the FRANKLIN PIERCE LAW CENTER EDUCATIONAL REPORT: PATENT LANDSCAPE OF ADJUVANT FOR HIV VACCINES. This list includes:
- Aluminum-based compounds such as aluminum phosphate and aluminum hydroxide.
- Immunostimulatory adjuvants like CpG, MPL and QS21 orMF59 which is a squalene oil-in water emulsions.
- Freunds incomplete adjuvant (FIA) or Freund's complete adjuvant (FCA), can also be used.
- Calcium phosphate in particular orthophosphates, metaphosphates or pyrophosphates and occasionally hydrogen or hydroxide ions.
- Muramyl dipeptide (*N*-Acetyl muramyl-L-alanine-D-isoglutamine, MDP) and its synthetic analogs such as muramyl tripeptide phosphatidylethanolamine MTPPTdEtn),
- Trehalose-6,6'-dimycolate,
- Saponins (Triterpenoid glycosides) like QS-21
- Stearyl Tyrosine (octadecyl ester hydrochloride salt of tyrosine) Immunostimulatory adjuvants can include the following classes of compounds:
- Polysaccharides like:
   ∘ *Chitosan:*
   ∘ *Inulin:*
   ∘ *Beta* - *Glucans*
   ∘ *Lipo-Polysaccharides or endotoxin*
   ∘ *MGN-3Actinidia eriantha (AEPS):*
   ∘ *Eldexomer*:
   ∘ *CpG ODN*
- Liposomes like
   ∘ *Dehydration-rehydration liposome vesicles (DRVs)*
   ∘ *Cytotoxic T lymphocyte (CTL)*
   ∘ *CAF01*
   ∘ *Liposomes containing lipid A (LA)*
- Lipid Polysine Core Peptides (LCP)
- Cytokine like GM-CSF, IL-2,IL-12 or IL-15
- Lipid A and Monophosphoryl Lipid A (MPL)
- Lipopeptide which are molecules consisting of lipid connected to a peptide. They are derived from the lipoprotein of bacterial cell wall.
- Exogenous Immunostimulatory Adjuvants which are compounds, or proteins that are not found in the human body like:
   ∘ Proteosomes in particular Multiple Antigenic Peptides (MAP) or Exogenous Toxins

Suitably, the total amount mutated lentiviral ENV protein in a single dose of the immunogenic composition is 10µg and/or the total amount of unfused polypeptide in a single dose of the immunogenic composition is 20µg. In one embodiment, the total amount of all antigens in a single dose of the immunogenic composition is 0.5-50µg, 2-40µg, 5-30µg, 10µ-20µg or around 30µg, around 20µg or around 10µg.

The amount of mutated lentiviral ENV protein in a dose of the immunogenic composition is selected as an amount which induces an immune response without significant, adverse side effects in typical recipients. Such amount will vary depending upon which specific immunogen is employed and the dosing or vaccination regimen that is selected. An optimal amount for a particular immunogenic composition can be ascertained by standard studies involving observation of relevant immune responses in subjects.

Administration of the pharmaceutical composition can take the form of one or of more than one individual dose, for example as repeat doses of the same polypeptide containing composition, or in a heterologous "prime-boost" vaccination regime, including proteins and vectors. In one embodiment, the immunogenic composition of the invention is initially administered to a subject as two or three doses, wherein the doses are separated by a period of two weeks to three months, preferably one month.
Conveniently, the composition is administered to a subject (for instance as a booster) every 6-24, or 9-18 months, for instance annually. For instance, the composition is administered to a subject (for instance as a booster) at six month or 1 year intervals. Suitably in this respect, subsequent administrations of the composition to the subject boost the immune response of earlier administrations of the composition to the same subject.
In an embodiment, the immunogenic composition of the invention is used as part of a prime -boost regimen for use in the treatment or prevention of disease or infection by HIV-1 strains from one or more clades different from the one or more HIV-1 clades in the immunogenic composition. Conveniently, the composition is the priming dose. Alternatively, the composition is the boosting dose.
Suitably, two or more priming and/ or boosting doses are administered. A heterologous prime-boost regime uses administration of different forms of immunogenic composition or vaccine in the prime and the boost, each of which can itself include two or more administrations. The priming composition and the boosting composition will have at least one antigen in common, although it is not necessarily an identical form of the antigen, it can be a different form of the same antigen.
Prime boost immunisations according to the invention can be homologous prime-boost regimes or heterologous prime-boost regimes. Homologous prime-boost regimes utilize the same composition for prime and boost, for instance the immunogenic composition of the invention. Heterologous prime-boost regimes can be performed with a combination of protein and DNA- based formulations. Such a strategy is considered to be effective in inducing broad immune responses. Adjuvanted protein vaccines induce mainly antibodies and CD4+ T cell immune responses, while delivery of DNA as a plasmid or a recombinant vector induces strong CD8+ T cell responses. Thus, the combination of protein and DNA vaccination can provide for a wide variety of immune responses. This is particularly relevant in the context of HIV, since neutralizing antibodies, CD4+ T cells and CD8+ T cells are thought to be important for the immune defense against HIV-1.

The disclosure also relates to a pharmaceutical composition according the above definition, for its use for the treatment of lentiviral infection.

The disclosure also provides a method for treating patient afflicted by pathologies related to lentiviral infection, comprising the administration to a patient in a need thereof of a pharmaceutically efficient amount of the pharmaceutical composition as defined above. The disclosure also relates to a method for inducing an immune response against a lentivirus that has infected an individual, said method comprising the administration to a patient in a need thereof of a pharmaceutically efficient amount of the pharmaceutical composition as defined above.

A pharmaceutical composition as defined above, may comprise an antiviral agent.

The composition according to the disclosure can also be used in combination with the antiviral compositions listed in Table 1 below:

**Table 1: Trade names of the different antivirals used for HIV treatment, as well as their specificity of action.**

| | |
|---|---|
| **Intelence**® (TMC 125/etravirine) Tibotec - | Non-nucleoside reverse transcriptase inhibitor |
| **Agenerase**® (APV/amprenavir) GSK - | Protease inhibitor |
| **Aptivus**®(TPV/tipranavir) Boehringer - | Protease inhibitor |
| **Crixivan**®(IDV/indinavir) MSD - | Protease inhibitor |
| **Invirase**®(SQV/saquinavir) Roche - | Protease inhibitor |
| **Kaletra**®(LPV.r/lopinavir + ritonavir) Abbott | Protease inhibitor |
| **Norvir**®(ritonavir) Abbott - | Protease inhibitor |
| **Prezista**®(TMC 114/darunavir) Tibotec/Janssen-Cilag - | Protease inhibitor |
| **Reyataz**®(ATZ/atazanavir) BMS - | Protease inhibitor |
| **Telzir**®(APV/fosamprenavir) GSK - | Protease inhibitor |
| **Viracept**®(nelfinavir) Roche - | Protease inhibitor |
| **Fuzeon**®(T20/enfuvirtide) Roche - | Fusion inhibitor |
| **Celsentri**®(maraviroc) Pfizer - | Entry inhibitor |
| **Isentress**®(MK 0518/raltegravir) Merck - | Integrase inhibitor |
| **Rescriptor**®(delavirdine) Agouron - | Non-nucleoside reverse transcriptase inhibitor |
| **Sustiva**®(EFV/efavirenz) BMS - | Non-nucleoside reverse transcriptase inhibitor |
| **Viramune**®(nevirapine) Boehringer - | Non-nucleoside reverse transcriptase inhibitor |
| **Combivir**®(Retrovir®+Epivir®) GSK - | Nucleoside reverse transcriptase inhibitor |
| **Emtriva**®(FTC, emtricitabine) Gilead - | Nucleoside reverse transcriptase inhibitor |
| **Epivir**®(3TC, lamivudine) GSK - | Nucleoside reverse transcriptase inhibitor |
| **Kivexa**®(Ziagen®+ Epivir®) GSK - | Nucleoside reverse transcriptase inhibitor |
| **Retrovir**®(AZT/zidovudine) QSK - | Nucleoside reverse transcriptase inhibitor |
| **Trizivir**®(Retrovir®+Epivir®+Ziagen®) GSK - | Nucleoside reverse transcriptase inhibitor |
| **Videx**®(ddI/didanosine) BMS - | Nucleoside reverse transcriptase inhibitor |
| **Viread**®(TDF/tenofovir) Gilead - | Nucleoside reverse transcriptase inhibitor |
| **Zerit**®(d4T/stavudine) BMS - | Nucleoside reverse transcriptase inhibitor |
| **Ziagen®**(ABC/abacavir) GSK - | Nucleoside reverse transcriptase inhibitor |
| **Truvada**®(Emtriva® + Viread(1<)) Gilead - | Nucleoside reverse transcriptase inhibitor |
| **Atripla**®(Sustiva® + Emtriva® + Viread®) BMS / GILEAD | Nucleoside and non-nucleoside reverse transcriptase inhibitor |

The pharmaceutical composition as defined above, may be simultaneous, separate or sequential use.

A pharmaceutical composition as defined above may be used for stimulating an immune response in a host organism.

The disclosure relates to a pharmaceutical composition as defined above for its use for inducing a specific immune response against the HIV ENV protein.

The disclosure relates to a pharmaceutical composition as defined above for its use for the prevention or the treatment of HIV infection, or pathologies related to AIDS.

The disclosure relates to a pharmaceutical composition as defined above, as a vaccine, for its use for inducing a specific immune response against the HIV ENV protein.

The disclosure relates to a pharmaceutical composition as defined above, as a vaccine, for its use for the prevention or the treatment of HIV infection, or pathologies related to AIDS.

In another aspect, the invention also relates to a method to obtain the active substance of a pharmaceutical composition, as defined above, consisting of modifying the immunosuppressive property of:
a wild-type human or simian lentiviral ENV protein,
or a fragment of said wild-type human or simian lentiviral ENV protein, said fragment comprising at least 40 amino acids,
said ENV protein or fragment thereof presenting a transmembrane subunit (TM) comprising an immunosuppressive domain (ISU) containing the following amino acid sequence :
   A-[I/V]-E-[K/R]-X'ₐ-X'_{b}-X-D-Q (SEQ ID NO: 427),
   wherein
   X represents any amino acid,
   X'ₐ is C, D, E, H, I, K, M, N, P, Q, S, T, V, W or Y, and
   X'_{b} is C, D, E, H, I, K, L, M, N, P, Q, S, T, V, W or Y,
said method comprising a step of introduction of at least one mutation of X'ₐ and/or X'_{b}, to obtain:
   an isolated mutated human or simian lentiviral ENV protein having substantially no immunosuppressive activity,
   said mutated human or simian lentiviral ENV protein having at least 90% identity, to one sequence chosen from the group consisting of SEQ ID NO : 216, SEQ ID NO : 420 and SEQ ID NO : 421,
   or a fragment of said isolated mutated human or simian lentiviral ENV protein having substantially no immunosuppressive activity, said fragment comprising at least 40 amino acids,
   said mutated ENV protein and fragment thereof comprising a mutated immunosuppressive domain (ISU) containing the following amino sequence :
      A-[I/V]-E-[K/R]-Xₐ-X_{b}-X-D-Q (SEQ ID NO: 416),
      wherein
      X represents any amino acid,
      and either
      Xₐ is A, F, G, L, R and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G, R or Xₐ is A, F, G, L, R and X_{b} is A, F, G, R
   said substantial absence of immunosuppressive activity of the above mentioned mutated human or simian lentiviral ENV protein or of the above defined fragment being liable to be assessed by the fact that in an *in vivo* assay involving engrafted tumor cells rejection,
   said tumor cells being transduced either so as to express said mutated ENV protein or said fragment (mutated ENV tumor cells),
   or said tumor cells being transduced so as to express said wild type ENV protein or a fragment thereof (wild type ENV tumor cells),
   or said tumor cells being not transduced (normal tumor cells),
   the following ratio :
      immunosuppression index of said mutated ENV protein or of said fragment (i_{mutated env} ) / immunosuppression index of wild type ENV protein (i_{wild type env}) is less than 0.5,
      i_{mutated env} being defined by : (maximum area reached by mutated ENV tumor cells - maximum area reached by normal tumor cells) / (maximum area reached by normal tumor cells), and
      i_{wild type env} being defined by : (maximum area reached by wild type ENV tumor cells - maximum area reached by normal tumor cells) / (maximum area reached by normal tumor cells).

The invention also relates to a method to obtain the active substance of a pharmaceutical composition, according to the above defined method, consisting of modifying the immunosuppressive property of:
a wild-type human or simian lentiviral ENV protein,
or a fragment of said wild-type human or simian lentiviral ENV protein, said fragment comprising at least 40 amino acids,
said ENV protein or fragment thereof presenting a transmembrane subunit (TM) comprising an immunosuppressive domain (ISU) containing the following amino acid sequence:
   A-[I/V]-E-[K/R]-Y-L-X-D-Q (SEQ ID NO : 1),
   wherein
   X represents any amino acid,
   said method comprising a step of introduction of at least one mutation of Y in position 5 and/or L in position 6,
to obtain:
   an isolated mutated human or simian lentiviral ENV protein having substantially no immunosuppressive activity,
   or a fragment of said isolated mutated human or simian lentiviral ENV protein having substantially no immunosuppressive activity, said fragment comprising at least 40 amino acids,
   said mutated ENV protein and fragment thereof comprising a mutated immunosuppressive domain (ISU) containing the following amino sequence :
      A-[I/V]-E-[K/R]-Xₐ-X_{b}-X-D-Q (SEQ ID NO: 416),
      wherein
      X represents any amino acid,
      and either
      Xₐ is A, F, G, L, R and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G, R or Xₐ is A, F, G, L, R and X_{b} is A, F, G, R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, F, G, L or R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is A, F, G or R, or
Xₐ is A, F, G, L or R, and X_{b} is A, F, G or R.

The invention also relates to a method as defined above to obtain the active substance of a pharmaceutical composition, ,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, F, G, L or R, and X_{b} is L, I, V, M or P, or
Xₐ is Y, I, H, C or T, and X_{b} is A, F, G or R, or
Xₐ is A, F, G, L or R, and X_{b} is A, F, G or R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, G, or R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is F, G or R, or
Xₐ is F or L, and X_{b} is F, G or R, or
Xₐ is A, G or R, and X_{b} is A.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, G, or R, and X_{b} is L, I, V, M or P, or
Xₐ is Y, I, H, C or T, and X_{b} is F, G or R, or
Xₐ is F or L, and X_{b} is F, G or R, or
Xₐ is A, G or R, and X_{b} is A, or
Xₐ is A, G or R, and X_{b} is F, G or R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein,
X represents any amino acid,
and either
Xₐ is A, G, or R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is F, G or R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein,
X represents any amino acid,
and either
Xₐ is A, G, or R, and X_{b} is L, I, V, M or P, or
Xₐ is Y, I, H, C or T, and X_{b} is F, G or R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein,
X represents any amino acid,
and either
Xₐ is R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is R, or
Xₐ is R, and X_{b} is R.

The invention also relates to a method as defined above to obtain the active substance of a pharmaceutical composition,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutation in one at least of the amino acids at positions, 29, 36 and 37 of SEQ ID NO: 426 :
A[I/V]E[K/R]XₐX_{b}X₁DQX₂X₃LX₄X₅WGC[A/S][F/G]X₆X₇CVX₈TX₉VPX_{c}X₁₀Z₁Z₂Z₃Z₄Z₅ X_{d}Xₑ[S/T] (SEQ ID NO: 426) wherein Xₐ and X_{b} are as defined in anyone of claims 1 to 15, X₁ to X₁₀ represent any amino acid, Z₁ to Z₅ represent no amino acid or any amino acid, independently from each other such that
∘ X_{c} is A, C, D, E, F, G, H, I, K, L, M, N, P, Q, R, S, T, V, Y, or deleted, preferably A, D, or N,
∘ X_{d} is A, C, D, E, F, G, H, I, L, M, N, P, Q, R, S, T, V, Y, W, or deleted, preferably A, G, S or Y,
∘ Xₑ is A, C, D, E, F, G, H, I, K, L, M, N, P, Q, R, T, V, Y, W, or deleted, preferably A, D or N.

The following examples of pharmaceutical compositions are also part of the disclosure.

A *pharmaceutical composition comprising as active substance :*
*a) an isolated non naturally occurring mutated human or simian lentiviral ENV having substantially no immunosuppressive activity, said mutated human or simian lentiviral ENV resulting from mutation of the transmembrane subunit (TM) of a wild type human or simian lentiviral ENV protein, the transmembrane subunit comprising an immunosuppressive domain (ISU) comprising the following amino acid sequence:*
   *A-[I*/*V]-E-[K*/*R]-Y-L-X-D-Q (SEQ ID NO: 1),*
   *said sequence encompassing*
   *AIEKYLXDQ (SEQ ID NO: 2), AIERYLXDQ (SEQ ID NO: 3), AVEKYLXDQ (SEQ ID NO: 4) and AVERYLXDQ (SEQ ID NO: 5), wherein X represents any natural amino acid,*
   *wherein the amino acids at the positions chosen among*
   - *the position 4 of SEQ ID NO: 1,*
   - *the position 5 of SEQ ID NO: 1,*
   - *the position 6 of SEQ ID NO: 1,*
   - *the positions 4 and 5 of SEQ ID NO: 1,*
   - *the position 4 and 6 of SEQ ID NO: 1,*
   - *the position 5 and 6 of SEQ ID NO: 1, and*
   - *the position 4, 5 and 6 of SEQ ID NO: 1,*
   *are* :
   - *either deleted,*
   - *or substituted by another amino acid such that*
      ∘ *when the immunosuppressive domain comprises SEQ ID NO : 2 or 4*
         ▪ *the amino acid residue at position 4 of SEQ ID NO: 1 is substituted by A, C, D, E, F, G, H, I, L, M, N, P, Q, R, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y,*
      ∘ *when the immunosuppressive domain comprises SEQ ID NO : 3 or 5,*
         ▪ *the amino acid residue at position 4 of SEQ ID NO: 1 is substituted by A, C, D, E, F, G, H, I, K, L, M, N, P, Q, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y,*
   *or*
*b) a fragment of said mutated human or simian lentiviral ENV, said fragment comprising at least the immunosuppressive domain (ISU) of said wild type human or simian lentiviral ENV comprising the following amino acid sequence:*
   *A-[I*/*V]-E-[K*/*R]-Y-L-X-D-Q (SEQ ID NO: 1),*
   *wherein the amino acids at the positions chosen among*
   - *the position 4 of SEQ ID NO: 1,*
   - *the position 5 of SEQ ID NO: 1,*
   - *the position 6 of SEQ ID NO: 1,*
   - *the positions 4 and 5 of SEQ ID NO: 1,*
   - *the position 4 and 6 of SEQ ID NO: 1,*
   - *the position 5 and 6 of SEQ ID NO: 1, and*
   - *the position 4, 5 and 6 of SEQ ID NO: 1,*
   *are :*
   - *either deleted,*
   - *or substituted by another amino acid such that*
      ∘ *when the immunosuppressive domain comprises SEQ ID NO : 2 or 4*
         ▪ *the amino acid residue at position 4 of SEQ ID NO: 1 is substituted by A, C, D, E, F, G, H, I, L, M, N, P, Q, R, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y,*
      ∘ *when the immunosuppressive domain comprises SEQ ID NO : 3 or 5,*
         ▪ *the amino acid residue at position 4 of SEQ ID NO: 1 is substituted by A, C, D, E, F, G, H, I, K, L, M, N, P, Q, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y,*
   *said fragment having substantially no immunosuppressive properties,*
   *in association of a pharmaceutically acceptable carrier.*

A *pharmaceutical composition as defined above, wherein the amino acids at the positions of the group consisting of*
- *the position 4 of SEQ ID NO: 1,*
- *the position 5 of SEQ ID NO: 1,*
- *the position 6 of SEQ ID NO: 1,*
- *the positions 4 and 5 of SEQ ID NO: 1,*
- *the position 4 and 6 of SEQ ID NO: 1,*
- *the position 5 and 6 of SEQ ID NO: 1, and*
- *the position 4, 5 and 6 of SEQ ID NO: 1,*
   - *are substituted by another amino acid such that*
      ∘ *when the immunosuppressive domain comprises SEQ ID NO : 2 or 4*
         ▪ *the amino acid residue at position 4 of SEQ ID NO: 1 is substituted by A, C, D, E, F, G, H, I, L, M, N, P, Q, R, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y,*
      ∘ *when the immunosuppressive domain comprises SEQ ID NO : 3 or 5,*
         ▪ *the amino acid residue at position 4 of SEQ ID NO: 1 is substituted by A, C, D, E, F, G, H, I, K, L, M, N, P, Q, S, T, V, W or Y, and*/*or*
         ▪ *the amino acid residue at position 5 of SEQ ID NO: 1 is substituted by A, C, D, E, F, G, I, L, M, N, P, Q, R, S, T, V or W, and*/*or*
         ▪ *the amino acid residue at position 6 of SEQ ID NO: 1 is substituted by A, C, D, E, F, G, H, I, K, M, N, P, Q, R, S, T, V, W or Y.*

The *mutated lentiviral protein comprised in the above defined composition,* may comprise *the amino acid sequence of the list consisting of: SEQ ID NO: 407 to SEQ ID NO: 414.*

*The correspondence is the following one:*

| | | | |
|---|---|---|---|
| *AVEAALKD* | *SEQ ID NO : 407* | *AIEAALKD* | *SEQ ID NO : 411* |
| *AVEEALKD* | *SEQ ID NO : 408* | *AIEEALKD* | *SEQ ID NO : 412* |
| *AVESALKD* | *SEQ ID NO : 409* | *AIESALKD* | *SEQ ID NO : 413* |
| *AVETALKD* | *SEQ ID NO : 410* | *AIETALKD* | *SEQ ID NO : 414* |

*These proteins have been mutated at position 4 of SEQ ID NO: 1, by substitution, and the amino acid at position 5 has been substituted by A.*

A pharmaceutical composition as defined above, wherein the amino acids at the positions of the group consisting of
- *the position 5 of SEQ ID NO: 1,*
- *the position 6 of SEQ ID NO: 1, and*
- *the position 5 and 6 of SEQ ID NO: 1,*
   - *are substituted by another amino acid such that*
      ▪ *the amino acid residue at position 5 of SEQ ID NO: 1 is substituted by A, C, D, E, F, G, I, L, M, N, P, Q, R, S, T, V or W, and*/*or*
      ▪ *the amino acid residue at position 6 of SEQ ID NO: 1 is substituted by A, C, D, E, F, G, H, I, K, M, N, P, Q, R, S, T, V, W or Y.*

A pharmaceutical composition as defined above, wherein said immunosuppressive domain (ISU) comprises the amino acid sequence of the group consisting of: SEQ ID NO : 4 or 5.

A *pharmaceutical composition as defined above, wherein said immunosuppressive domain (ISU) comprises the amino acid sequence SEQ ID NO : 5, and wherein*
▪ *the amino acid residue at position 5 of SEQ ID NO: 5 is substituted by A, F, G, L or R, or*
▪ *the amino acid residue at position 6 of SEQ ID NO: 5 is substituted by A, F, G or R, or*
▪ *the amino acid residues at position 5 and 6 of SEQ ID NO: 5 are substituted by A, F, G, or R.*

*This is the case when said ENV protein is the ENV protein of HIV 1 virus.*

A *pharmaceutical composition as defined above, wherein said immunosuppressive domain (ISU) comprises the amino acid sequence SEQ ID NO : 2, and wherein*
▪ *the amino acid residue at position 5 of SEQ ID NO: 2 is substituted by A, F, G, L or R, or*
▪ *the amino acid residue at position 6 of SEQ ID NO: 2 is substituted by A, F, G or R, or*
▪ *the amino acid residues at position 5 and 6 of SEQ ID NO: 2 are substituted by A, F, G, or R.*

A *pharmaceutical composition as defined above, wherein said immunosuppressive domain (ISU) comprises the amino acid sequence SEQ ID NO : 3, and wherein*
▪ *the amino acid residue at position 5 of SEQ ID NO: 3 is substituted by A, F, G, L or R, or*
▪ *the amino acid residue at position 6 of SEQ ID NO: 3 is substituted by A, F, G or R, or*
▪ *the amino acid residues at position 5 and 6 of SEQ ID NO: 3 are substituted by A, F, G, or R.*

A *pharmaceutical composition as defined above, wherein said mutated protein further comprises the wild type amino acid sequence SEQ ID NO: 270,*
*X₁DQX₂X₃LX₄X₅WGC[A*/*S][FIG]X₆X₇CVX₈TX₉VPWX₁₀Z₁Z₂Z₃Z₄Z₅[N*/*S][E*/*D*/*N*/*A][S*/*T] (SEQ ID NO: 270)*
*wherein X₁-X₁₀ represents any amino acid,*
*wherein Z₁ to Z₅ represent no amino acid or any natural amino acid, independently from each other,*
*said mutated lentiviral protein further comprising a mutation in one at least of the amino acids at positions 23, 30 and 31 of SEQ ID NO: 270*
*said mutation being:*
- *either a deletion,*
- *or a substitution such that*
   ∘ *the amino acid at position 25 of SEQ ID NO: 270 is substituted by A, C, D, E, F, G, H, I, K, L, M, N, P, Q, R, S, T, V, Y,*
   ∘ *the amino acid at position 32 of SEQ ID NO: 270 is substituted by A, C, D, E, F, G, H, I, K, L, M, P, Q, R, T, V, Y, W,*
   ∘ *the amino acid at position 33 of SEQ ID NO: 270 is substituted by C, F, G, H, I, K, L, M, P, Q, R, S, T, V, Y, W.*

A *pharmaceutical composition comprising as active substance :*
*an isolated non naturally occurring mutated human or simian lentiviral ENV having substantially no immunosuppressive activity, said mutated human or simian lentiviral ENV resulting from mutation of a wild type human or simian lentiviral ENV protein, said wild type human or simian lentiviral ENV protein comprising the following amino acid sequence:*
   *A[I*/*V]E[K*/*R]YLX₁DQX₂X₃LX₄X₅WGC[A*/*S][F*/*G]X₆X₇CVX₈TX₉VPWX₁₀Z₁Z₂Z₃Z₄Z₅[N*/*S][ E*/*D*/*N*/*A][S*/*T] (SEQ ID NO: 415),*
   *wherein X₁-X₁₀ represents any amino acid,*
   *wherein Z₁ to Z₅ represent no amino acid or any natural amino acid, independently from*
   *each other,*
   *wherein the amino acids at the positions chosen among*
   - *the position 4 of SEQ ID NO: 1,*
   - *the position 5 of SEQ ID NO: 1,*
   - *the position 6 of SEQ ID NO: 1,*
   - *the positions 4 and 5 of SEQ ID NO: 1,*
   - *the position 4 and 6 of SEQ ID NO: 1,*
   - *the position 5 and 6 of SEQ ID NO: 1, and*
   - *the position 4, 5 and 6 of SEQ ID NO: 1,*
   *are :*
   - *either deleted,*
   - *or substituted by another amino acid such that*
      ▪ *the amino acid residue at position 4 of SEQ ID NO: 1 is substituted by A, C, D, E, F, G, H, I, L, M, N, P, Q, S, T, V, W or Y, and*/*or*
      ▪ *the amino acid residue at position 5 of SEQ ID NO: 1 is substituted by A, C, D, E, F, G, I, L, M, N, P, Q, R, S, T, V or W, and*/*or*
      ▪ *the amino acid residue at position 6 of SEQ ID NO: 1 is substituted by A, C, D, E, F, G, H, I, K, M, N, P, Q, R, S, T, V, W or Y,*
   *and further wherein*
   *one at least of the amino acids at positions 29, 36 and 37 of the amino acid sequence SEQ ID NO : 415 are mutated, by*
   - *either a deletion,*
   - *or a substitution such that*
      ∘ *the amino acid at position 29 of SEQ ID NO: 415 is substituted by A, C, D, E, F, G, H, I, K, L, M, N, P, Q, R, S, T, V, Y,*
      ∘ *the amino acid at position 36 of SEQ ID NO: 415 is substituted by A, C, D, E, F, G, H, I, K, L, M, P, Q, R, T, V, Y, W,*
      ∘ *the amino acid at position 37 of SEQ ID NO: 415 is substituted by C, F, G, H, I, K, L, M, P, Q, R, S, T, V, Y, W.*

A *pharmaceutical composition as defined above, further comprising a mutation at the position X₄ X₁DQX₂X₃LX₄X₅WGC[A*/*S][F*/*G]X₆X₇CVX₈TX₉VPWX₁₀Z₁Z₂Z₃Z₄Z₅[N*/*S][E*/*D*/*N*/*A][S*/*T] (SEQ ID NO: 270)*
*wherein X₁-X₃ and X₅-X₁₀ represents any amino acid,*
*Z₁ to Z₅ represent no amino acid or any amino acid, independently from each other*
*said mutation being :*
- *either a deletion,*
- *or a substitution by A, C, D, E, F, H, I, K, L, M, P, Q, R, S, T, V, Y or W.*

A *pharmaceutical composition comprising as active substance :*
*an isolated non naturally occurring mutated human or simian lentiviral ENV having substantially no immunosuppressive activity, said mutated human or simian lentiviral ENV resulting from mutation of a wild type human or simian lentiviral ENV protein, said wild type human or simian lentiviral ENV protein comprising the following amino acid sequence:*
*A[I*/*V]E[K*/*R]YLX₁DQX₂X₃LX₄X₅WGC[A*/*S][F*/*G]X₆X₇CVX₈TX₉VPWX₁₀Z₁Z₂Z₃Z₄Z₅[N*/*S][ E*/*D*/*N*/*A][S*/*T] (SEQ ID NO: 415),*
*wherein X₁-X₁₀ represents any amino acid,*
*wherein Z₁ to Z₅ represent no amino acid or any natural amino acid, independently from*
*each other,*
*wherein the amino acids at the positions chosen among*
- *the position 4 of SEQ ID NO: 1,*
- *the position 5 of SEQ ID NO: 1,*
- *the position 6 of SEQ ID NO: 1,*
- *the positions 4 and 5 of SEQ ID NO: 1,*
- *the position 4 and 6 of SEQ ID NO: 1,*
- *the position 5 and 6 of SEQ ID NO: 1, and*
- *the position 4, 5 and 6 of SEQ ID NO: 1,*
*are* :
- *either deleted,*
- *or substituted by another amino acid such that*
   ▪ *the amino acid residue at position 4 of SEQ ID NO: 1 is substituted by A, C, D, E, F, G, H, I, L, M, N, P, Q, S, T, V, W or Y, and*/*or*
   ▪ *the amino acid residue at position 5 of SEQ ID NO: 1 is substituted by A, C, D, E, F, G, I, L, M, N, P, Q, R, S, T, V or W, and*/*or*
   ▪ *the amino acid residue at position 6 of SEQ ID NO: 1 is substituted by A, C, D, E, F, G, H, I, K, M, N, P, Q, R, S, T, V, W or Y,*
*and possibly, further wherein*
*one at least of the amino acids at positions 29, 36 and 37 of the amino acid sequence SEQ ID NO : 415 are mutated, by*
- *either a deletion,*
- *or a substitution such that*
   ∘ *the amino acid at position 29 of SEQ ID NO: 415 is substituted by A, C, D, E, F, G, H, I, K, L, M, N, P, Q, R, S, T, V, Y,*
   ∘ *the amino acid at position 36 of SEQ ID NO: 415 is substituted by A, C, D, E, F, G, H, I, K, L, M, P, Q, R, T, V, Y, W,*
   ∘ *the amino acid at position 37 of SEQ ID NO: 415 is substituted by C, F, G, H, I, K, L, M, P, Q, R, S, T, V, Y, W,*
*and*/*or possibly further wherein the amino acid at position X₄ is either deleted or substituted by A, C, D, E, F, H, I, K, L, M, P, Q, R, S, T, V, Y or W, preferably by R or H.*

### LEGEND TO THE FIGURES

Figure 1 :
   Amino acid sequences of the HIV ectodomain deletants, with the indicated length, and of single mutant (substitution are underlined) ectodomains.
Figure 2:
   Functional delineation of the immunosuppressive domain of the HIV envelope. The immunosuppressive activity of 115aa-long and truncated HIV envelope ectodomains (see structures on the left) was tested using the MCA205 tumor rejection *in vivo* assay. Immunosuppression indexes are given as histograms on the right (mean values +/- SD).
Figure 3 :
   Functional identification of the aminoacid in the HIV envelope ectodomain directly involved in immunosuppressive activity, and search for aminoacid substitutions inhibiting this activity. Immunosuppresive activity was tested in vivo as in Figure 2.
Figure 4 :
   Expression profile of mutated HIV envelopes using full-length HIVenv-expressing vectors and an anti-SU HIVenv(110H) monoclonal antibody (FACS). The data are expressed as percentages of the control HIVenv WT.
Figure 5 :
   Infectivity of HIVenv-pseudotyped viral particles showing functionality of the full-length WT and of specifically mutated HIV envelopes. The results are expressed as viral titers (number of infected target cells/mL of virus supernatant).
Figure 6 :
   Functional identification of the amino acid in the SIV envelope ectodomain directly involved in immunosuppressive activity, and search for aminoacid substitutions inhibiting this activity. Immunosuppressive activity was tested using the in vivo MCA205 tumor rejection assay. Immunosuppression indexes are represented as histograms (mean values +/- SD).

### EXAMPLE

Immunosuppressive domains have been identified on the envelope proteins of oncoretroviruses, of either the gamma- (murine MLV) or the delta (e.g. human HTLV) type, as well as of some endogenous retroviruses (e.g. HERV-FRD). These domains have a highly conserved crystallographic structure, although their primary sequences are quite diverse, and an amino-acid (either Q, E, or K) at a definite position has previously been demonstrated to be essential for the immunosuppresive activity of the corresponding envelope protein. Mutation of this amino acid to an Arginine (R) was further demonstrated to result in inhibition of the immunosuppressive activity of the mutated envelope protein, with in some cases complete conservation of the other functional properties of the envelope protein, including its ability to be normally expressed at the cell membrane, to be captured by a nascent retroviral particle, and finally to confer infectivity of the mutant virus in vitro. Such mutants allowed an unambiguous demonstration of the essential role of the immunosuppressive activity for viremia in vivo, with the mutant IS- virus being unable to escape the host immune system and propagate in an immunocompetent animal [Schlecht-Louf et al., Proc Natl Acad Sci USA. 2010;107(8):3782-7]*.*

On the basis of the identification of an IS domain and on the ability of the IS function to be inhibited by specific mutations within the IS domain that do not disrupt the antigenic structure of the corresponding viral protein, vaccinal approaches are being developed with vaccines containing mutated « optimized » IS-negative viral antigens, which demonstrate an increased immunognicity as compared to those using the native viral antigens. Search for similar IS domains and appropriate mutations in the case of non-oncogenic-but still pathogenic- retroviruses such as HIV is therefore of interest to tentatively develop improved vaccines.

Actually, in the case of the other major class of retroviruses, namely the lentiviruses (among which the human HIV1 and HIV2, and the SIV simian homologues), the crystallographic structure of the envelope protein discloses some similarities but also very important differences, especially in the domain corresponding to the IS domain of oncoretroviruses, with evidence in HIV and SIV for a severely extended helix-loop-helix domain, and no evidence for sequence similarities with the IS domain of oncoretroviruses. Furthermore, an IS domain with strong amino-acid similarities with the IS domain of oncoretroviruses was identified within an accessory protein of HIV and SIV, namely within the Nef protein. This protein is specifically produced by these complex retroviruses and is not encoded by oncoretroviruses. The Nef protein is essential for viremia in vivo, and it possesses several domains responsible for immune escape, among which the identified IS domain, thus strongly suggesting that lentiviruses had transferred the immunosuppressive activity found in oncoretroviruses within their envelope protein, to the accessory Nef protein. In agreement with this hypothesis, Nef-deleted or Nef-mutated retroviruses have a severely attenuated pathogenicity in in vivo macaque animal models. Here, the Inventors report on the identification of an IS activity carried by the HIV and SIV envelope proteins, and on specific mutations that inhibit this activity without major disrupt ion of the overall structure of the corresponding envelope proteins.

### Results

### Delination of the immunosuppressive domain of the HIV Envelope

Delineation of the immunosuppressive domain of the HIV envelope was achieved using an *in vivo* tumor rejection assay, that the Inventors had previously used to demonstrate the immunosuppressive activity of the Env protein of oncoretroviruses (murine MoMLV and simian MPMV). The rationale of the assay can be summarized as follows: while injection of MCA205 tumor cells (H-2^{b}) into allogeneic Balb/c mice (H-2^{d}) leads to the formation of no tumor or transient tumors that are rapidly rejected, injection of the same cells, but stably expressing an immunosuppressive retroviral Env protein, leads to the growth of larger tumors that persist for a longer time -in spite of the expression of the new exogenous antigen. This difference is not associated with a difference in intrinsic cell growth rate since it is not observed in syngeneic C57BL/6 mice, and is immune system-dependent. The extent of "immunosuppression" can be quantified by an index based on tumor size: (Aₑₙᵥ-Aₙₒₙₑ)/Aₙₒₙₑ, where Aₑₙᵥ and Aₙₒₙₑ are the mean areas at the peak of growth of tumors from Balb/c mice injected with *env*-expressing or control cells, respectively. A positive index indicates that *env* expression facilitates tumor growth, as a consequence of its immunosuppressive activity; a null or negative index points to no effect or even an inhibitory effect, respectively. The latter may be explained by a stimulation of the immune response of the host against the new foreign antigen, represented by a non-immunosuppressive Env protein, expressed at the surface of tumor cells.

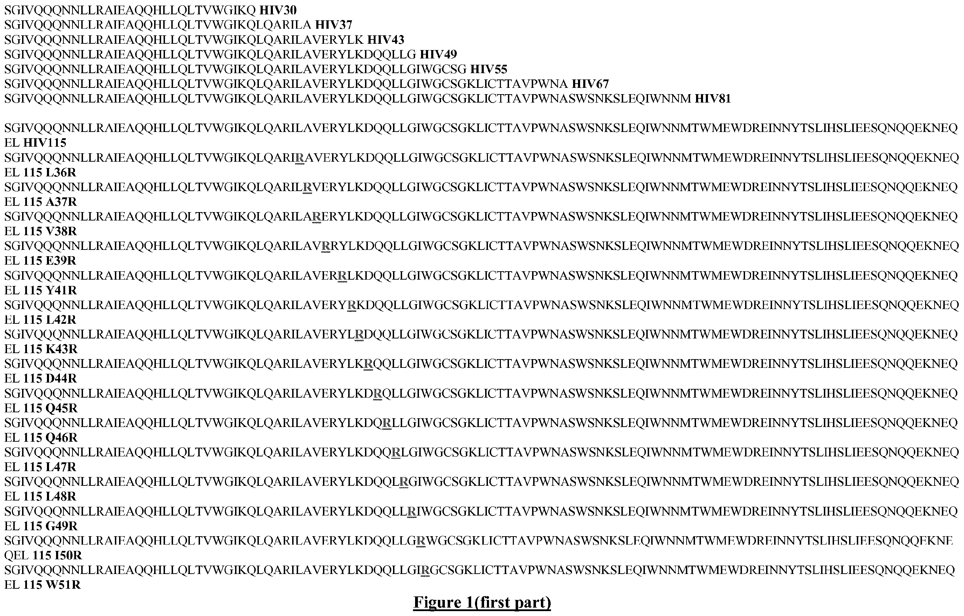

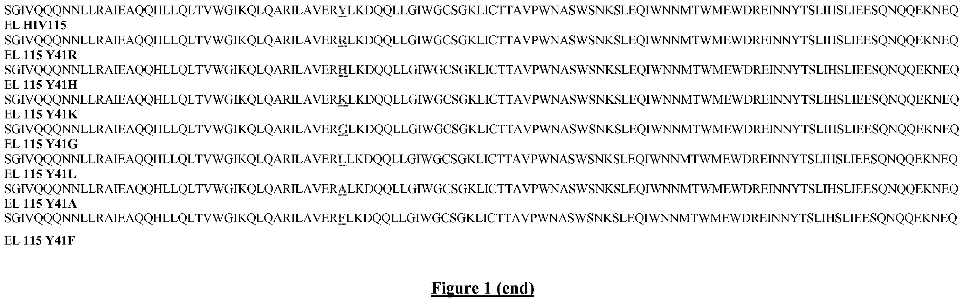

To first delineate a minimal immunosuppressive domain (ISD) active *in vivo,* the Inventors analyzed the effect of a series of truncations/deletions within the HIV *env* gene. Assay of the series of C-term truncations in Fig. 1&2 identified a 49 residue-long domain with IS activity, with the 6 aa shorter HIV43 fragment and subsequent C-term truncated HIV37 and HIV30 fragments being IS-negative. HIV49 is embedded into the so-called ectodomain, which corresponds to a soluble part of the extracellular domain of the TM subunit, and consists in the a-helical domain involved in HIV TM trimerization, and the N-term part of the loop containing the 2 well-conserved, 6 aa-distant, cysteine residues found in most retroviral envelopes. Refine delineation of the amino acis responsible for IS activity was then performed by arginine-scanning between aa 36 and 51, i.e. in the domain associated with the transition from IS positive to negative Env subdomains. As illustrated in Fig. 3, all the X-to-Arg substitutions resulted in no significant change in the IS activity of HIV115, with one exception for Y41 and L42, which resulted in loss of IS activity of the mutant peptide.

### Mutations at position 41

The Inventors then checked that the Y41R substitution did not alter the overall capacity of the HIV envelope to be expressed by an eucaryotic cell and to be exported at the cell membrane, by introducing the Y41R substitution into an expression vector for the HIV envelope. A FACS analysis of cell transfected with both the wild-type and the Y41R mutant using an anti-SU specific monoclonal antibody actually demonstrated quantitative expression of the mutant envelope at the cell surface, thus indicating that the Y41R mutation does not significantly alter the HIV Env structure and SU-TM interaction (figure 4).

A series of distinct substitutions at the Y41 position were finally performed to tentatively identify whether other amino acids could be substituted to generate IS-negative variants: the amino acid assayed included the positively charged K and H (in addition to R), the small A and G, and the hydrophobic L and F residues. Again it was checked that these substitutions did not alter the overall structure of the HIV Env with the corresponding mutations.

Among them, substitution of Y41 by A, G, L and F resulted in loss of IS (figure 3), and did not alter the overall structure of the HIV Env with the corresponding mutations (figure 4 and 5).

### Further mutation outside of the ISU domain

The effects of a set of second mutations at positions that are structurally close to Y41 position within the ENV ectodomain three-dimensional structure were also tested. Two of them (Y41R-K72A and Y41R-K78G) maintain a high level expression at cell membrane and confer infectivity (figure 5).

### Mutations at position 42

Interestingly, the adjacent position to the position Y41, position L42, also resulted in loss of IS activity of the mutant peptide when the mutation L42R is introduced. This mutation maintains a high level expression at cell membrane of the mutated HIV ENV protein (figure 4).

### Mutations within the SIV ENV protein

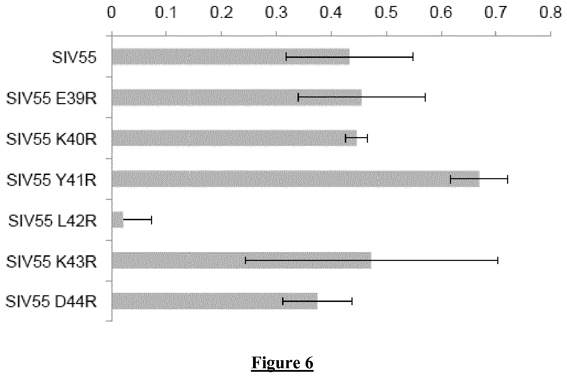

Interestingly, mutation at the homologous position in SIV ENV (L42) of the position L42 in HIV-1 ENV ectodoamin also resulted in specific loss of IS activities (Figure 6).

Accordingly, the present investigation has clearly identified a definite location and a definite substitution(s) within the HIV env resulting in the loss of its IS activity. Being compatible with the conservation of the overall structure of the human and simian lentiviral Env proteins, these substitutions should be introduced in all pharmaceutical preparations which include the Env protein as a vaccine antigen.

### Materials and Methods

**Mice and cell lines:** C57B1/6 and Balb/c mice, 6-10 weeks old, were obtained from CER Janvier (Laval, France). Mice were maintained in the animal facility of the Gustave Roussy Institute in accordance with institutional regulations. 293T (ATCC CRL11268), and MCA205 cells were cultured in DMEM supplemented with 10% fetal calf serum (Invitrogen), streptomycin (100 µg/ml) and penicillin (100 units/ml).

**Plasmid construction:** The PCEL/E160 encoding the envelope protein of the BRU/LAI HIV-1 isolate is a gift from Dr Marc Sitbon. To generate the pDFG plasmids encoding the various fragments of HIV-1 envelope ectodomain, PCR fragments generated using PCEL/E160 as a template and primer pairs 1-2 (pDFG-HIV115), 1-3 (pDFG-HIV81), 1-4 (pDFG-HIV67), 1-5 (pDFG-HIV55), 1-6 (pDFG-HIV49), 1-7 (pDFG-HIV43), 1-8 (pDFG-HIV37), 1-9 (pDFG-HIV30) were digested with SfiI and MluI and inserted into pDFG-ectoSyncytin-1 (Mangeney et al, 2007) opened with the same enzymes.

Mutated pDFG-HIV115 were obtained by successive PCR using appropriate primers. A first series of PCR was performed with pDFG HIV115 as template using primers 1-11 and 10-2, 1-13 and 12-2, 1-15 and 14-2 or 1-17 and 16-2 to introduce the E39R, Y41R, K43R or D44R mutations respectively, and primers 1-18 and 19-2, 1-20 and 21-2, 1-22 and 23-2, 1-24 and 25-2, 1-26 and 27-2, 1-28 and 29-2, 1-30 and 31-2, 1-32 and 33-2, 1-34 and 35-2, or 1-36 and 37-2 to introduce the L36R, A37R, V38R, L42R, Q45R, Q46R, L47R, L48R, G49R, or 150R mutations respectively. The PCR products were then used as templates in subsequent PCR using primers 1-2. These PCR fragments were digested with SfiI and MluI and inserted into pDFG-ectoSyncytin-1 (Mangeney et al, 2007) opened with the same enzymes.

All the constructions were sequenced before use.

**Table 1. Primer list**

| N° | Name | Primer sequence (5'-3') | SEQ ID |
|---|---|---|---|
| 1 | TM HIV Sfi-Sens | ACATggcccagccggccTCTGGTATAGTGCAGCAGC | SEQ ID NO: 295 |
| 2 | TM HIV115 Mlu-AS | GTATacgcgtTTATAATTCTTGTTCATTCTTTTC | SEQ ID NO: 296 |
| 3 | TM HIV81 Mlu AS | GTATacgcgtTTACATGTTATTCCAAATCTGTTCC | SEQ ID NO: 297 |
| 4 | TM HIV67 Mlu AS | GTATacgcgtTTAAGCATTCCAAGGCACAGC | SEQ ID NO: 298 |
| 5 | TM HIV55 Mlu AS | GTATacgcgtTTATCCAGAGCAACCCCAAATCC | SEQ ID NO: 299 |
| 6 | TMHIV49 Mlu AS | GTATacgcgtTTACCCCAGGAGCTGTTGATCC | SEQ ID NO: 300 |
| 7 | TMHIV43 Mlu AS | GTATacgcgtTTACTTTAGGTATCTTTCCACAGC | SEQ ID NO: 301 |
| 8 | TMHIV37 Mlu AS | GTATacgcgtTTAAGCCAGGATTCTTGCCTGGAG | SEQ ID NO: 302 |
| 9 | TMHIV30 Mlu AS | GTATacgcgtTTACTGCTTGATGCCCCAGAC | SEQ ID NO: 303 |
| 18 | TMHIV115 L36R as | CTTTCCACAGCCcGGATTCTTGCCTG | SEQ ID NO: 304 |
| 19 | TMHIV115 L36R s | CAGGCAAGAATCCgGGCTGTGGAAAG | SEQ ID NO: 305 |
| 20 | TMHIV115 A37R as | GTATCTTTCCACAcgCAGGATTCTTGCC | SEQ ID NO: 306 |
| 21 | TMHIV115 A37R s | GGCAAGAATCCTGcgTGTGGAAAGATAC | SEQ ID NO: 307 |
| 22 | TMHIV115 V38R as | CTTTAGGTATCTTTCCctAGCCAGGATTCTTGCC | SEQ ID NO: 308 |
| 23 | TMHIV115 V38R s | GGCAAGAATCCTGGCTagGGAAAGATACCTAAAG | SEQ ID NO: 309 |
| 11 | TMHIV115 E39R AS | CCTTTAGGTATCTTctCACAGCCAGGATTC | SEQ ID NO: 310 |
| 10 | TMHIV115 E39R S | GAATCCTGGCTGTGagAAGATACCTAAAGG | SEQ ID NO: 311 |
| 13 | TMHIV115 Y41R AS | GTTGATCCTTTAGGcgTCTTTCCACAGCCAG | SEQ ID NO: 312 |
| 12 | TMHIV115 Y41R S | CTGGCTGTGGAAAGAcgCCTAAAGGATCAAC | SEQ ID NO: 313 |
| 24 | TMHIV115 L42R as | GGAGCTGTTGATCCTTTcGGTATCTTTCCACAGCC | SEQ ID NO: 314 |
| 25 | TMHIV115 L42R s | GGCTGTGGAAAGATACCgAAAGGATCAACAGCTCC | SEQ ID NO: 315 |
| 15 | TMHIV115 K43R AS | GAGCTGTTGATCCcTTAGGTATCTTTCCAC | SEQ ID NO: 316 |
| 14 | TMHIV115 K43R S | GTGGAAAGATACCTAAgGGATCAACAGCTC | SEQ ID NO: 317 |
| 17 | TMHIV115 D44R AS | AGGAGCTGTTGAcgCTTTAGGTATCTTT | SEQ ID NO: 318 |
| 16 | TMHIV115 D44R S | AAAGATACCTAAAGcgTCAACAGCTCCT | SEQ ID NO: 319 |
| 26 | TMHIV115 Q45R as | CCCAGGAGCTGTcGATCCTTTAGGTATC | SEQ ID NO: 320 |
| 27 | TMHIV115 Q45R s | GATACCTAAAGGATCgACAGCTCCTGGG | SEQ ID NO: 321 |
| 28 | TMHIV115 Q46R as | CAAATCCCCAGGAGCcGTTGATCCTTTAG | SEQ ID NO: 322 |
| 29 | TMHIV115 Q46R s | CTAAAGGATCAACgGCTCCTGGGGATTTG | SEQ ID NO: 323 |
| 30 | TMHIV115 L47R as | CAAATCCCCAGGcGCTGTTGATCCTTTAG | SEQ ID NO: 324 |
| 31 | TMHIV115 L47R s | CTAAAGGATCAACAGCgCCTGGGGATTTG | SEQ ID NO: 325 |
| 32 | TMHIV115 L48R as | CCCAAATCCCCcGGAGCTGTTG | SEQ ID NO: 326 |
| 33 | TMHIV115 L48R s | CAACAGCTCCgGGGGATTTGGG | SEQ ID NO: 327 |
| 34 | TMHIV115 G49R as | CCCCAAATCCgCAGGAGCTGTTG | SEQ ID NO: 328 |
| 35 | TMHIV115 G49R s | CAACAGCTCCTGcGGATTTGGGG | SEQ ID NO: 329 |
| 36 | TMHIV115 I50R as | GCAACCCCAtcTCCCCAGGAGCTG | SEQ ID NO: 330 |
| 37 | TMHIV115 I50R s | CAGCTCCTGGGGAgaTGGGGTTGC | SEQ ID NO: 331 |
| 38 | TMHIV115 W51R as | GCAACCCCAtcTCCCCAGGAGCTG | SEQ ID NO: 332 |
| 39 | TMHIV115 W51R s | CAGCTCCTGGGGAgaTGGGGTTGC | SEQ ID NO: 333 |

**HIV115 Y41 mutant construction:** To explore the amino acid sequence necessary for the loss of immunosuppression, HIV115 Y41 mutants were produced by PCR using the pDFG HIV115 WT plasmid as template and pairs of primers 1-42 and 43-2, 1-44 and 45-2, 1-46 and 47-2, 1-48 and 49-2, 1-50 and 51-2, or 1-52 and 53-2, to introduce the Y41K, Y41H, Y41A, Y41G, Y41F, or Y41L mutations respectively. The PCR products were then used as templates in subsequent PCR using primers 1-2. These PCR fragments were digested with SfiI and MluI and inserted into pDFG-ectoSyncytin-1 (Mangeney et al, 2007) opened with the same enzymes
All the constructions were sequenced before use.

| N° | Name | Primer sequence (5'-3') | SEQ ID NO |
|---|---|---|---|
| 42 | TMHIV115 Y41K as | GTTGATCCTTTAGtTtTCTTTCCACAGCCAG | SEQ ID NO: 334 |
| 43 | TMHIV115 Y41K s | CTGGCTGTGGAAAGAaAaCTAAAGGATCAAC | SEQ ID NO: 335 |
| 44 | TMHIV115 Y41H as | GTTGATCCTTTAGGTgTCTTTCCACAGCCAG | SEQ ID NO: 336 |
| 45 | TMHIV115 Y41H s | CTGGCTGTGGAAAGAcACCTAAAGGATCAAC | SEQ ID NO: 337 |
| 46 | TMHIV115 Y41A as | GTTGATCCTTTAGGgcTCTTTCCACAGCCAG | SEQ ID NO: 338 |
| 47 | TMHIV115 Y41A s | CTGGCTGTGGAAAGAgcCCTAAAGGATCAAC | SEQ ID NO: 339 |
| 48 | TMHIV 115 Y41G as | GTTGATCCTTTAGGccTCTTTCCACAGCCAG | SEQ ID NO: 340 |
| 49 | TMHIV115 Y41G s | CTGGCTGTGGAAAGAggCCTAAAGGATCAAC | SEQ ID NO: 341 |
| 50 | TMHIV115 Y41F as | GTTGATCCTTTAGGaATCTTTCCACAGCCAG | SEQ ID NO: 342 |
| 51 | TMHIV115 Y41F s | CTGGCTGTGGAAAGATtCCTAAAGGATCAAC | SEQ ID NO: 343 |
| 52 | TMHIV115 Y41L as | GTTGATCCTTTAGGagTCTTTCCACAGCCAG | SEQ ID NO: 344 |
| 53 | TMHIV115 Y41L s | CTGGCTGTGGAAAGActCCTAAAGGATCAAC | SEQ ID NO: 345 |

**HIV115 Y41 double mutants construction:** HIV115 Y41R K72A, G or S double mutants were produced by PCR using the pDFG HIV115 Y41R plasmid as template and pairs of primers 1-94 and 93-2, 1-96 and 95-2, or 1-98 and 97-2. The PCR products were then used as templates in subsequent PCR using primers 1-2. These PCR fragments were digested with SfiI and MluI and inserted into pDFG-ectoSyncytin-1 (Mangeney et al, 2007) opened with the same enzymes. HIV115 R40X Y41R double-mutants were produced by PCR using the pDFG HIV115 Y41R as template and pairs of primers 1-54 and 55-2, 1-56 and 57-2 to introduce the R40A and R40E mutations respectively. HIV115 R40X Y41A double-mutants were produced by PCR using the pDFG HIV115 Y41A as template and pairs of primers 1-58 and 59-2, 1-60 and 61-2, 1-62 and 63-2, 1-64 and 65-2 to introduce the R40A, R40E, R40S, R40T mutations respectively. HIV115 R40X simple mutants were also generated using pDFG HIV115 WT as template and pairs of primers 1-66 and 67-2, 1-68 and 69-2, 1-70 and 71-2, 1-72 and 73-2.

The PCR products were then used as templates in subsequent PCR using primers 1-2. These PCR fragments were digested with SfiI and MluI and inserted into pDFG-ectoSyncytin-1 (Mangeney et al, 2007) opened with the same enzyme.

| N° | Name | Primer sequence (5'-3') | SEQ ID |
|---|---|---|---|
| 54 | TM HIV115 R40AY41R-S | CTGGCTGTGGAAgcAcgCCTAAAGGATCAAC | SEQ ID NO: 378 |
| 55 | TM HIV115 R40AY41RAS | GTTGATCCTTTAGGcgTgcTTCCACAGCCAG | SEQ ID NO: 379 |
| 56 | TM HIV115 R40EY41R-S | CTGGCTGTGGAAgaAcgCCTAAAGGATCAAC | SEQ ID NO: 380 |
| 57 | TM HIV115 R40EY41RAS | GTTGATCCTTTAGGcgTtcTTCCACAGCCAG | SEQ ID NO: 381 |
| 58 | TM HIV115 R40AY41AS | CTGGCTGTGGAAgctgcCCTAAAGGATCAAC | SEQ ID NO: 382 |
| 59 | TM HIV115 R40AY41A-AS | GTTGATCCTTTAGGgcagcTTCCACAGCCAG | SEQ ID NO: 383 |
| 60 | TM HIV115 R40EY41AS | CTGGCTGTGGAAgaagcCCTAAAGGATCAAC | SEQ ID NO: 384 |
| 61 | TM HIV115 R40EY41A-AS | GTTGATCCTTTAGGgcttcTTCCACAGCCAG | SEQ ID NO: 385 |
| 62 | TM HIV115 R40SY41AS | CTGGCTGTGGAAagcgcCCTAAAGGATCAAC | SEQ ID NO: 386 |
| 63 | TM HIV115 R40SY41A-AS | GTTGATCCTTTAGGgcgctTTCCACAGCCAG | SEQ ID NO: 387 |
| 64 | TM HIV115 R40TY41AS | CTGGCTGTGGAAacagcCCTAAAGGATCAAC | SEQ ID NO: 388 |
| 65 | TM HIV115 R40TY41A-AS | GTTGATCCTTTAGGgctgtTTCCACAGCCAG | SEQ ID NO: 389 |
| 66 | TM HIV115 R40A-S | CTGGCTGTGGAAgctTACCT AAAGGATCAAC | SEQ ID NO: 390 |
| 67 | TM HIV115 R40A-AS | GTTGA TCCTTT AGGT AagcTTCCACAGCCAG | SEQ ID NO: 391 |
| 68 | TM HIV115 R40E-S | CTGGCTGTGGAAgaaT ACCTAAAGGATCAAC | SEQ ID NO: 392 |
| 69 | TM HIV115 R40E-AS | GTTGA TCCTTT AGGT AttcTTCCACAGCCAG | SEQ ID NO: 393 |
| 70 | TM HIV 115 R40S-S | CTGGCTGTGGAAagcT ACCTAAAGGATCAAC | SEQ ID NO: 394 |
| 71 | TM HIV 115 R40S-AS | GTTGA TCCTTT AGGT AgctTTCCACAGCCAG | SEQ ID NO: 395 |
| 72 | TM HIV 115 R40T-S | CTGGCTGTGGAAacaT ACCT AAAGGATCAAC | SEQ ID NO: 396 |
| 73 | TM HIV 115 R40T-AS | GTTGA TCCTTT AGGT AtgtTTCCACAGCCAG | SEQ ID NO: 397 |
| 93 | TMHIV115 K72A s | GCTAGTTGGAGTAATgcATCTCTGGAACAGATTTGG | SEQ ID NO: 398 |
| 94 | TMHIV115 K72A as | CCAAATCTGTTCCAGAGATgcATTACTCCAACTAGC | SEQ ID NO: 399 |
| 95 | TMHIV115 K72G s | GCTAGTTGGAGTAATggATCTCTGGAACAGATTTGG | SEQ ID NO: 400 |
| 96 | TMHIV115 K72G as | CCAAATCTGTTCCAGAGATccATTACTCCAACTAGC | SEQ ID NO: 401 |
| 97 | TMHIV115 K72S s | GCTAGTTGGAGTAATtcATCTCTGGAACAGATTTGG | SEQ ID NO: 402 |
| 98 | TMHIV115 K72S as | CCAAATCTGTTCCAGAGATgaATTACTCCAACTAGC | SEQ ID NO: 403 |

**Introduction of HIV115 mutations into an HIV Env expression vector:** To introduce these HIV envelope mutations into the HIVenv pTr712 expression vector (Schnierle et al PNAS 1997), a silent mutation was first generated to create an SfiI insertion site. PCR fragments were generated using pTr712as a template and primer pairs 38-39 and 40-41. The PCR products were then used as templates in subsequent PCR using primers 38-41.

The resulting PCR fragment was then digested with BsaBI and HindIII and inserted into the pTr712 plasmid opened with the same enzymes resulting in the pTr712-Sfi plasmid. PCR fragments of each HIV115 mutation were then generated by using primers 40-41 and the pDFG HIV115 mutant plasmids as templates, digestion with SfiI and HindIII and then insertion into the pTr712-Sfi plasmid opened with the same enzymes.

All the constructions were sequenced before use.

| N° | Name | Primer sequence (5'-3') | SEQ ID NO |
|---|---|---|---|
| 38 | HIV BH10 BsaBI s | acaaattagatgttcatcaaatattacaggg | SEQ ID NO: 346 |
| 39 | HIV BH10 SfiI mutsil as | | SEQ ID NO: 347 |
| 40 | HIV BH10 SfiI mutsil s | | SEQ ID NO: 348 |
| 41 | HIV BH10 HindIII as | gtgtattaagcttgtgtaattgttaatttctc | SEQ ID NO: 349 |
| 74 | EnvHIVTr712-Xho-Age-Kozak-S | | SEQ ID NO : 404 |
| 75 | EnvHIVTr712-Mlu-AS | | SEQ ID NO : 405 |
| 76 | EnvHIVWT-Mlu-AS | | SEQ ID NO : 406 |

**Establishment of *env*-expressing tumor cells and MCA205 tumor-rejection assay:** 7.5 x 10⁵ 293T cells were cotransfected with the env-expressing pDFG retroviral vector to be tested (1.75 µg) and expression vectors for the MLV proteins (0.55 µg for the amphotropic MLV *env* vector and 1.75 µg for the MLV *gag* and *pol* vector). 36 hours post-transfection, supernatants were harvested for infection of MCA205 tumor cells (2.5 ml of supernatant per 5 x 10⁵ cells with 8 µg/ml polybrene). Cells were maintained in selective medium (400 units/ml hygromycin) for 3 weeks, and then washed with PBS, trypsinized and inoculated subcutaneously in the shaved area of each mouse right flank as in Mangeney et al (1998, 2007). Tumor growth was monitored by palpation twice or thrice weekly and tumor area (mm²) determined by measuring perpendicular tumor diameters. The extent of "immunosuppression" was quantified by an index based on tumor size: (Aₑₙᵥ-Aₙₒₙₑ)/Aₙₒₙₑ, where Aₑₙᵥ and Aₙₒₙₑ are the mean areas at the peak of growth of tumors from Balb/c mice injected with env-expressing or control cells, respectively.

**Analysis of HIVenv expression :** 293T cells were transfected with 4mg of the expression vector for the HIV-1 envelope (pTr712) either wild-type or mutated at the indicated positions, by Calcium Phosphate precipitation. Cells are washed 16h later and then harvested 2 days post-transfection using PBS-EDTA 5mM. The 110-H monoclonal antibody (anti-V3 loop, gift from Hybridolab, Pasteur Institute) was used (1/200 dilution) to stain the HIV envelope. As a secondary antibody, the Inventors used the goat anti mouse IgG Alexa 488 (1/400) (Invitrogen). For intracellular HIVenv staining, 293T cells were fixed with a formaldehyde buffer and then permeabilized (BD cytofix /cytoperm, BD Biosciences). The isotype mouse anti IgGlKappa (BD Biosciences) was used to control non-specific staining. Fluorescence was acquired by flow cytometry using a FACS Calibur (BD Biosciences), and data analysed by the CellQuest software (BD Biosciences).

**Mutated Env pseudotyping and measure of viral titer:** 293T cells are triple transfected with 3mg of a reporter MLV vector carrying GFP (CNCG), 1.75mg Mo-MLV gag-pol vector and 0.55mg phCMV vector encoding HIV-1 envelope wt or mutated at the indicated positions. The infectivity of Mo-MLV virions pseudotyped with HIV-1 Env, harvested 48 hours post-transfection, is measured using U87 cells (CD4⁺, CXCR4⁺) as target cells. The infectivity of the enveloppes is analysed after 72h exposure diluted 0.45mm-filtered supernatant in presence of 4mg/mL polybrene. The fluorescence (GFP) is acquired by flow cytometry using a FACS Calibur (BD Biosciences). The results are analysed by the CellQuest software (BD Biosciences). The resulting titers (number of infected cells/mL) are calculated as the following : (% GFP⁺cells (infected) x plated cell number)/volume of supernatant x 1000.

**Env SIV mutants construction :** PCR fragments were generated using p239 SPE3' (the plasmid encoding the SIV half virus containing the envelope protein) as a template and primer pairs 77-79 and 78-80, 77-81 and 78-82, 77-83 and 78-84, 77-85 and 78-86, 77-87 and 78-88, 77-89 and 78-90 to introduce the E39R, K40R, Y41R, L42R, K43R, D44R mutations respectively. The PCR products were the used as templates in subsequent PCR using primers 77-78. The resulting PCR fragment was then digested with BmgBI and NheI and inserted into the p239 SPE3' plasmid opened with the same enzymes.
All the constructions were sequenced before use.

**Introduction of SIV mutants ectodomain into pDFG** : To generate the pDFG plasmids encoding the fragment of SIV envelope ectodomain-55, PCR fragments generated using p239 SPE3' WT and mutants as a template and primer pairs 91-92, were digested with SfiI and MluI and inserted into pDFG opened with the same enzymes.

| | | | |
|---|---|---|---|
| 77 | Env SIV S | ccgctcagtcccgaactttattggc | SEQ ID NO: 350 |
| 78 | Env SIV AS | ggtggggaagagaacactggcc | SEQ ID NO: 351 |
| 79 | SIV env E39R s | cagactagggtcactgccatcCGCaagtacttaaaggaccaggcg | SEQ ID NO: 352 |
| 80 | SIV env E39R as | cgcctggtcctttaagtacttGCGgatggcagtgaccctagtctg | SEQ ID NO: 353 |
| 81 | SIV env K40R s | actagggtcactgccatcgagCGCtacttaaaggaccaggcgcag | SEQ ID NO: 354 |
| 82 | SIV env K40R as | ctgcgcctggtcctttaagtaGCGctcgatggcagtgaccctagt | SEQ ID NO: 355 |
| 83 | SIV env Y41R s | GCCATCGAGAAGcgCTTAAAGGACCAGGCG | SEQ ID NO: 356 |
| 84 | SIV env Y41R as | CGCCTGGTCCTTTAAGcgCTTCTCGATGGC | SEQ ID NO: 357 |
| 85 | SIV env L42R s | gtcactgccatcgagaagtacCGCaaggaccaggcgcagctg | SEQ ID NO: 358 |
| 86 | SIV env L42R as | cagctgcgcctggtccttGCGgtacttctcgatggcagtgac | SEQ ID NO: 359 |
| 87 | SIV env K43R s | gccatcgagaagtacttaCGCgaccaggcgcagctgaatgcttgg | SEQ ID NO: 360 |
| 88 | SIV env K43R as | ccaagcattcagctgcgcctggtcGCGtaagtacttctcgatggc | SEQ ID NO: 361 |
| 89 | SIV env D44R s | gagaagtacttaaagCGCcaggcgcagctgaatgcttgg | SEQ ID NO: 362 |
| 90 | SIV env D44R as | attcagctgcgcctgGCGctttaagtacttctcgatggc | SEQ ID NO: 363 |
| 91 | TMSIV55 Sfi S | ACATggcccagccggccgctgggatagtgcagcaac | SEQ ID NO: 364 |
| 92 | TMSIV55 Mlu AS | GTATacgcgtTTAaaacgcacatccccaagcattc | SEQ ID NO: 365 |

**Comparative example: test of the *in vivo* effect of the mutation G49R, which corresponds to the mutation *"G19R"* in the international application** WO 2010/022,740.

WO 2010/022,740 discloses a consensus sequence of 50 amino acid of the HIV ENV protein. In this sequence, it is suggested that substitution of amino acids in positions 10, 19, 24, 34 and 40 affect the immunosuppressive properties of the HIV ENV protein.

These mutations are a transposition in lentivirus of the teaching of WO 2005/095,442 limited to endogenous or onco retroviruses. The authors of WO 2005/095,442 are also the authors of the present application and they early observed that such a transposition is not effective.

Despite the fact that any amino acids can be assigned to the positions 10, 19, 24, 34 or 40 in the consensus sequence of WO 2010/022,740, only one substitution (one residue for one position) was tested by *ex vivo* experiments in WO 2010/022,740. It is the mutation "*G19R",* which corresponds to the mutation G49R in the present figures 1 and 3.

Because the immune response of an individual involves different organs and different cellular and non-cellular components, *ex vivo* results have no predictive value concerning the *in vivo* immunosuppressive properties of a viral protein.

To determine if the mutations previously disclosed in WO 2010/022,740 are suitable for a medical use, the mutation G49R has been tested using the MCA205 tumor rejection *in vivo* assay, as defined in the section *"Establishment of env-expressing tumor cells and MCA205 tumor-rejection assay"* of the Example.

As shown in Figure 3, the peptide G49R remains almost as immunosuppressive *in vivo* as the wild type HIV 115 peptide, with an immunosuppression index which is higher than 0,5. Thus, the mutation G49R does not induce a significant decrease of the *in vivo* immunosuppressive properties of the HIV ENV protein, with a ratio of immunosuppression index of G49R mutated ENV HIV 115 peptide (i_{mutated env}) / immunosuppression index of wild type ENV HIV 115 peptide (i_{wild type env}), which is 0,7 (*i.e.* higher than 0,5).

This result demonstrates the insufficiently described teaching of WO 2010/022740 since the only one mutation tested in WO 2010/022,740, using an *ex vivo* test, does not significantly affect the *in vivo* immunosuppressive properties of the HIV ENV protein.

As a consequence, WO 2010/022,740 raises the same technical problem as the present invention but does not offer a technical solution.

## Claims

1. Pharmaceutical composition comprising as active substance :
a) an isolated mutated human or simian lentiviral ENV protein having substantially no immunosuppressive activity,
said mutated human or simian lentiviral ENV protein resulting from mutation of the transmembrane (TM) subunit of a wild type human or simian lentiviral ENV protein, said mutated human or simian lentiviral ENV protein having at least 90% identity to one sequence chosen from the group consisting of SEQ ID NO : 216, SEQ ID NO : 420 and SEQ ID NO : 421,
said mutated human or simian lentiviral ENV protein comprising a mutated immunosuppressive domain (ISU) containing the following amino acid sequence:
A-[I/V]-E-[K/R]-Xₐ-X_{b}-X-D-Q (SEQ ID NO: 416),
wherein,
X represents any amino acid,
and either
Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R,
said mutated human or simian lentiviral ENV protein retains the antigenic structure of the wild type human or simian lentiviral ENV protein
or
b) at least one fragment of said isolated mutated human or simian lentiviral ENV protein having substantially no immunosuppressive activity,
said fragment comprising at least 40 amino acids,
said fragment comprising a mutated immunosuppressive domain (ISU) containing the following amino acid sequence:
A-[I/V]-E-[K/R]-Xₐ-X_{b}-X-D-Q (SEQ ID NO: 416),
wherein,
X represents any amino acid,
and either
Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R,
said fragment retains the antigenic structure of the full length isolated mutated human or simian lentiviral ENV protein from which it derives
in association with a pharmaceutically acceptable carrier,
said substantial absence of immunosuppressive activity of the above mentioned mutated human or simian lentiviral ENV protein or of the above defined fragment being liable to be assessed by the fact that in an *in vivo* assay involving engrafted tumor cells rejection,
said tumor cells being transduced either so as to express said mutated ENV protein or said fragment (mutated ENV tumor cells),
or said tumor cells being transduced so as to express said wild type ENV protein or a fragment thereof (wild type ENV tumor cells),
or said tumor cells being not transduced (normal tumor cells),
the following ratio :
immunosuppression index of said mutated ENV protein or of said fragment (i_{mutated env}) / immunosuppression index of wild type ENV protein (i_{wild type env}) is less than 0.5,
i_{mutated env} being defined by : (maximum area reached by mutated ENV tumor cells - maximum area reached by normal tumor cells) / (maximum area reached by normal tumor cells), and
i_{wild type env} being defined by : (maximum area reached by wild type ENV tumor cells - maximum area reached by normal tumor cells) / (maximum area reached by normal tumor cells).

2. Pharmaceutical composition according to claim 1 comprising as active substance:
a) an isolated mutated human or simian lentiviral ENV protein having substantially no immunosuppressive activity,
said mutated human or simian lentiviral ENV protein resulting from mutation of the transmembrane (TM) subunit of a wild type human or simian lentiviral ENV protein, said mutated human or simian lentiviral ENV protein comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, F, G, L or R, and X_{b} is L, I, V, M or P, or
Xₐ is Y, I, H, C or T, and X_{b} is A, F, G or R, or
Xₐ is A, F, G, L or R, and X_{b} is A, F, G or R,
or
b) at least one fragment of said isolated mutated human or simian lentiviral ENV protein having substantially no immunosuppressive activity,
said fragment comprising at least 40 amino acids,
said fragment comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, F, G, L or R, and X_{b} is L, I, V, M or P, or
Xₐ is Y, I, H, C or T, and X_{b} is A, F, G or R, or
Xₐ is A, F, G, L or R, and X_{b} is A, F, G or R,
in association with a pharmaceutically acceptable carrier.

3. Pharmaceutical composition according to claim 1 comprising as active substance:
a) an isolated mutated human or simian lentiviral ENV protein having substantially no immunosuppressive activity,
said mutated human or simian lentiviral ENV protein resulting from mutation of the transmembrane (TM) subunit of a wild type human or simian lentiviral ENV protein,
said mutated human or simian lentiviral ENV protein comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, G, or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is F, G or R,
or
Xₐ is F or L, and X_{b} is F, G or R,
or
Xₐ is A, G or R, and X_{b} is A,
or
Xₐ is A, G or R, and X_{b} is F, G or R,
or
b) at least one fragment of said isolated mutated human or simian lentiviral ENV protein having substantially no immunosuppressive activity,
said fragment comprising at least 40 amino acids,
said fragment comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, G, or R, and X_{b} is C, D, E, H, I, K, L, M, N, P, Q, S, T, V, W or Y,
or
Xₐ is C, D, E, H, I, K, M, N, P, Q, S, T, V, W or Y, and X_{b} is F, G or R,
or
Xₐ is F or L, and X_{b} is F, G or R,
or
Xₐ is A, G or R, and X_{b} is A,
or
Xₐ is A, G or R, and X_{b} is F, G, R,
in association with a pharmaceutically acceptable carrier.

4. Pharmaceutical composition according to claim 1 to 3 comprising as active substance
a) an isolated mutated human or simian lentiviral ENV protein having substantially no immunosuppressive activity,
said mutated human or simian lentiviral ENV protein resulting from mutation of the transmembrane (TM) subunit of a wild type human or simian lentiviral ENV protein, said mutated human or simian lentiviral ENV protein comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, G, or R, and X_{b} is L, I, V, M or P,
or
Xₐ is Y, I, H, C or T, and X_{b} is F, G or R,
or
b) at least one fragment of said isolated mutated human or simian lentiviral ENV protein having substantially no immunosuppressive activity,
said fragment comprising at least 40 amino acids,
said fragment comprising a mutated immunosuppressive domain (ISU) containing the following amino acid sequence:
A-[I/V]-E-[K/R]-Xₐ-X_{b}-X-D-Q (SEQ ID NO: 416),
wherein
X represents any amino acid,
and either
Xₐ is A, G, or R, and X_{b} is L, I, V, M or P,
or
Xₐ is Y, I, H, C or T, and X_{b} is F, G or R,
in association with a pharmaceutically acceptable carrier.

5. Pharmaceutical composition according to anyone of claims 1, 3
wherein
Xₐ is R, and X_{b} is C, D, E, H, I, K, L, M, N, P, Q, S, T, V, W or Y, or
Xₐ is C, D, E, H, I, K, M, N, P, Q, S, T, V, W or Y, and X_{b} is R, or
Xₐ is R, and X_{b} is R, in particular wherein said isolated mutated human or simian lentiviral ENV protein or said fragment of said isolated mutated human or simian lentiviral ENV protein comprises one of the following amino acid sequences
A-I-E-K-Xₐ-X_{b}-X-DQ (SEQ ID NO: 422),
A-I-E-R-Xₐ-X_{b}-X-DQ (SEQ ID NO: 423),
A-V-E-K-Xₐ-X_{b}-X-DQ (SEQ ID NO: 424),
A-V-E-R-Xₐ-X_{b}-X-DQ (SEQ ID NO: 425).

6. Pharmaceutical composition according to anyone of claims 1 to 5, wherein said isolated mutated human or simian lentiviral ENV protein or said fragment of said isolated mutated human or simian lentiviral ENV protein comprises one of the amino acid sequences :
SEQ ID NO : 13, SEQ ID NO : 42, SEQ ID NO : 71,
SEQ ID NO : 9 to 12,
SEQ ID NO : 14 to 41,
SEQ ID NO : 43 to 70, and
SEQ ID NO : 72 to 95,
in particular wherein said fragment of said isolated mutated human or simian lentiviral ENV protein comprises one of the amino acid sequences : SEQ ID NO : 96 to 211, more particularely wherein said isolated mutated human or simian lentiviral ENV protein consists of one of the amino acid sequences : SEQ ID NO : 212 to 269.

7. Pharmaceutical composition according to anyone of claims 1 to 6, wherein said mutated protein comprises an additional mutation in one at least of the amino acids at positions 29, 36 and 37 of SEQ ID NO: 426 :
A[I/V]E[K/R]X**ₐ**X**_{b}**X₁DQX₂X₃LX₄X₅WGC[A/S][F/G]X₆X₇CVX₈TX₉VPX_{c}X₁₀Z₁Z ₂Z₃Z₄Z₅X**_{d}**X**ₑ**[S/T] (SEQ ID NO: 426)
wherein
X**ₐ** and X**_{b}** are as defined in anyone of claims 1 to 6,
X₁ to X₁₀ represent any amino acid,
Z₁ to Z₅ represent no amino acid or any amino acid, independently from each other such that
∘ X_{c} is A, C, D, E, F, G, H, I, K, L, M, N, P, Q, R, S, T, V or Y, preferably A, D, or N,
∘ X_{d} is A, C, D, E, F, G, H, I, L, M, N, P, Q, R, S, T, V, Y or W, preferably A, G, S or Y,
∘ Xₑ is A, C, D, E, F, G, H, I, K, L, M, N, P, Q, R, T, V, Y or W, preferably A, D or N,
in particular wherein said mutated protein consists of one of the amino acid sequences of the group consisting of SEQ ID NO : 271 to 283.

8. Pharmaceutical composition comprising as active substance a nucleic acid molecule coding for a mutated human or simian lentiviral ENV protein, or a fragment of said mutated human or simian lentiviral ENV protein, as defined in anyone of claims 1 to 7,
wherein said nucleic acid molecule being contained in a vector, said vector comprising means allowing the expression of the mutated human or simian lentiviral ENV protein, or a fragment of said mutated human or simian lentiviral ENV protein, as defined in anyone of claims 1 to 7.

9. Pharmaceutical composition according to claim 8, said vector is chosen among a measles vector, a canarypox vector, a pox vector, a fowlpox, an adenoviral vector, a lentiviral vector, a Sendai virus, a Cytomegalovirus vector or a Modified Vaccinia Ankara vector.

10. Pharmaceutical composition, according to claims 8 or 9, comprising at least one nucleic acid molecule coding for a GAG protein and/or a PRO protein and/or a POL protein and/or a mutated NEF protein, wherein
said mutated NEF protein is substantially devoid of immunosuppressive properties,
of a human or simian lentivirus, said lentivirus being preferably of the same origin as the mutated lentiviral ENV protein.

11. Pharmaceutical composition, according to claim 10, wherein said nucleic acid molecule coding for a mutated human or simian lentiviral ENV protein, or a fragment of said mutated human or simian lentiviral ENV protein, is contained in the same vector as the one which also contains said at least one nucleic acid molecule coding for a GAG protein and/or a PRO protein and/or a POL protein and/or a mutated NEF protein, wherein said mutated NEF protein is substantially devoid of immunosuppressive properties,
or wherein said nucleic acid molecule coding for a mutated human or simian lentiviral ENV protein, or a fragment of said mutated human or simian lentiviral ENV protein, is contained in the same vector as the one which also contains all said at least one nucleic acid molecule coding for a GAG protein and/or a PRO protein and/or a POL protein and/or a mutated NEF protein, wherein said mutated NEF protein is substantially devoid of immunosuppressive properties,
said vector being a measles vector or a canary pox vector.

12. Pharmaceutical composition according to anyone of claims 1 to 11, for its use for the prevention or the treatment of lentiviral infection, preferably HIV-1 infection, HIV-2 infection or SIV infection,
in particular for its use as a vaccine, in particular against HIV-1 infection, HIV-2 infection or SIV infection.

13. A method to obtain the active substance of a pharmaceutical composition, as defined in anyone of claims 1 to 12, consisting of modifying the immunosuppressive property of:
a wild-type human or simian lentiviral ENV protein,
or a fragment of said wild-type human or simian lentiviral ENV protein, said fragment comprising at least 40 amino acids,
said ENV protein or fragment thereof presenting a transmembrane subunit (TM) comprising an immunosuppressive domain (ISU) containing the following amino acid sequence :
A-[I/V]-E-[K/R]-X'**ₐ**-X'**_{b}**-X-D-Q (SEQ ID NO: 427),
wherein
X represents any amino acid,
X'a is C, D, E, H, I, K, M, N, P, Q, S, T, V, W or Y, and
X'**_{b}** is C, D, E, H, I, K, L, M, N, P, Q, S, T, V, W or Y,
said method comprising a step of introduction of at least one mutation of X'**ₐ** and/or X'**_{b}**,
to obtain:
an isolated mutated human or simian lentiviral ENV protein having substantially no immunosuppressive activity,
said mutated human or simian lentiviral ENV protein having at least 90% identity, to one sequence chosen from the group consisting of SEQ ID NO : 216, SEQ ID NO : 420 and SEQ ID NO : 421,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R ,
said substantial absence of immunosuppressive activity of the above mentioned mutated human or simian lentiviral ENV protein or of the above defined fragment being liable to be assessed by the fact that in an *in vivo* assay involving engrafted tumor cells rejection,
said tumor cells being transduced either so as to express said mutated ENV protein or said fragment (mutated ENV tumor cells),
or said tumor cells being transduced so as to express said wild type ENV protein or a fragment thereof (wild type ENV tumor cells),
or said tumor cells being not transduced (normal tumor cells),
the following ratio :
immunosuppression index of said mutated ENV protein or of said fragment (i_{mutated env})/immunosuppression index of wild type ENV protein (i_{wild type env}) is less than 0.5,
i_{mutated env} being defined by : (maximum area reached by mutated ENV tumor cells - maximum area reached by normal tumor cells) / (maximum area reached by normal tumor cells), and
i_{wild type env} being defined by : (maximum area reached by wild type ENV tumor cells - maximum area reached by normal tumor cells) / (maximum area reached by normal tumor cells).

14. A method to obtain the active substance of a pharmaceutical composition, as defined in anyone of claims 1 to 12, according to claim 13, consisting of modifying the immunosuppressive property of :
a wild-type human or simian lentiviral ENV protein,
or a fragment of said wild-type human or simian lentiviral ENV protein, said fragment comprising at least 40 amino acids,
said ENV protein or fragment thereof presenting a transmembrane subunit (TM) comprising an immunosuppressive domain (ISU) containing the following amino acid sequence :
A-[I/V]-E-[K/R]-Y-L-X-D-Q (SEQ ID NO : 1),
wherein
X represents any amino acid,
said method comprising a step of introduction of at least one mutation of Y in position 5 and/or L in position 6,
to obtain:
an isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, F, G, L or R, and X_{b} is C, D, E, H, I, K, L, M, N, P, Q, S, T, V, W or Y, or Xₐ is C, D, E, H, I, K, M, N, P, Q, S, T, V, W or Y, and X_{b} is A, F, G or R, or Xₐ is A, F, G, L or R, and X_{b} is A, F, G or R .

15. A method to obtain the active substance of a pharmaceutical composition, as defined in anyone of claims 1 to 12, according to claim 13 or 14,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising a mutated immunosuppressive domain (ISU) containing the following amino sequence :
A-[I/V]-E-[K/R]-Xₐ-X_{b}-X-D-Q (SEQ ID NO: 416),
wherein
X represents any amino acid,
and either
Xₐ is A, G, or R, and X_{b} is L, I, V, M or P, or
Xₐ is Y, I, H, C or T, and X_{b} is F, G or R, or
Xₐ is F or L, and X_{b} is F, G or R, or
Xₐ is A, G or R, and X_{b} is A, or
Xₐ is A, G or R, and X_{b} is F, G or R.

16. A method to obtain the active substance of a pharmaceutical composition, as defined in anyone of claims 1 to 12, according to anyone of claims 13 to 15,
wherein said isolated mutated human or simian lentiviral ENV protein having substantially no immunosuppressive activity,
or a fragment of said isolated mutated human or simian lentiviral ENV protein having substantially no immunosuppressive activity, said fragment comprising at least 40 amino acids,
said mutated ENV protein and fragment thereof comprising an additional mutation in one at least of the amino acids at positions, 29, 36 and 37 of SEQ ID NO: 426 :
A[I/V]E[K/R]XₐX_{b}X₁DQX₂X₃LX₄X₅WGC[A/S][F/G]X₆X₇CVX₈TX₉VPX_{c}X₁₀Z₁Z₂ Z₃Z₄Z₅X**_{d}**X**ₑ**[S/T] (SEQ ID NO: 426)
wherein
Xₐ and X_{b} are as defined in anyone of claims 1 to 14,
X₁ to X₁₀ represent any amino acid,
Z₁ to Z₅ represent no amino acid or any amino acid, independently from each other
such that
∘ X_{c} is A, C, D, E, F, G, H, I, K, L, M, N, P, Q, R, S, T, V or Y, preferably A, D, or N,
∘ X_{d} is A, C, D, E, F, G, H, I, L, M, N, P, Q, R, S, T, V, Y or W, preferably A, G, S or Y,
∘ Xₑ is A, C, D, E, F, G, H, I, K, L, M, N, P, Q, R, T, V, Y or W, preferably A, D or N,
in particular wherein fragment of said mutated ENV protein consists of one of the amino acid sequences of the group consisting of SEQ ID NO : 271 to 283.

## Patentansprüche

1. Pharmazeutische Zusammensetzung, die als Wirkstoff umfasst:
a) ein isoliertes mutiertes humanes oder simianes lentivirales ENV-Protein, das im Wesentlichen keine immunsuppressive Aktivität aufweist,
das mutierte humane oder simiane lentivirale ENV-Protein resultiert aus der Mutation der Transmembran (TM)-Untereinheit eines humanen oder simianen lentiviralen Wildtyp-ENV-Protein, das mutierte humane oder simiane lentivirale ENV-Protein weist mindestens 90% Identität mit einer Sequenz ausgewählt aus der Gruppe bestehend aus SEQ ID NO : 216, SEQ ID NO : 420 und SEQ ID NO : 421 auf,
das mutierte humane oder simiane lentivirale ENV-Protein umfasst eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei,
X eine Aminosäure darstellt,
und entweder
Xₐ A, F, G, L oder R ist, und X_{b} C, D, E, H, I, K, L, M, N, P, Q, S, T, V, W oder Y ist, oder
Xₐ C, D, E, H, I, K, M, N, P, Q, S, T, V, W oder Y ist, und X_{b} A, F, G oder R ist, oder
Xₐ A, F, G, L oder R ist, und X_{b} A, F, G oder R ist,
das mutierte humane oder simiane lentivirale ENV-Protein behält die antigene Struktur des humanen oder simianen lentiviralen Wildtyp-ENV-Protein bei
oder
b) mindestens ein Fragment des isolierten humanen oder simianen Antiviralen ENV-Protein, das im Wesentlichen keine immunsuppressive Aktivität aufweist,
das Fragment umfasst mindestens 40 Aminosäuren,
das Fragment umfasst eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei
X eine Aminosäure darstellt,
und entweder
Xₐ A, F, G, L oder R ist, und X_{b} C, D, E, H, I, K, L, M, N, P, Q, S, T, V, W oder Y ist, oder
Xₐ C, D, E, H, 1, K, M, N, P, Q, S, T, V, W oder Y ist, und X_{b} A, F, G oder R ist, oder
Xₐ A, F, G, L oder R ist, und X_{b} A, F, G oder R ist,
das Fragment behält die antigene Struktur des isolierten mutierten humanen oder simianen lentiviralen ENV-Proteins in voller Länge, von welchem es abgeleitet ist, bei,
in Gemeinschaft mit einem pharmazeutisch akzeptablen Träger,
die im Wesentlichen Abwesenheit der immunsuppressiven Aktivität des vorher erwähnten humanen oder simianen lentiviralen ENV-Proteins oder des vorher definierten Fragments wird verpflichtend durch die Tatsache festgestellt, dass in einer *in vivo*-Untersuchung, die die Abstoßung transplantierter Tumorzellen einbezieht,
die Tumorzellen entweder so transduziert werden, dass sie das mutierte ENV-Protein oder das Fragment exprimieren (mutierte ENV-Tumorzellen), oder die Tumorzellen so transduziert werden, dass sie das Wildtyp-ENV-Protein oder ein Fragment davon exprimieren (Wildtyp-ENV-Tumorzellen), oder die Tumorzellen nicht transduziert werden (normale Tumorzellen),
das folgende Verhältnis:
Immunsuppressionsindex des mutierten ENV-Proteins oder des Fragments (i_{mutated env})/Immunsuppressionsindex des Wildtyp-ENV-Proteins (i_{wild type env}) weniger als 0,5 ist,
i_{mutated env} ist definiert durch: (maximale Fläche, die durch mutierte ENV-Tumorzellen erreicht wird - maximale Fläche, die durch normale Tumorzellen erreicht wird)/(maximale Fläche, die durch normale Tumorzellen erreicht wird), und
i_{wild type env} definiert ist durch: (maximale Fläche, die durch Wildtyp-ENV-Tumorzellen erreicht wird - maximale Fläche, die durch normale Tumorzellen erreicht wird)/(maximale Fläche, die durch normale Tumorzellen erreicht wird).

2. Pharmazeutische Zusammensetzung nach Anspruch 1, die als Wirkstoff umfasst:
a) ein isoliertes mutiertes humanes oder simianes lentivirales ENV-Protein, das im Wesentlichen keine immunsuppressive Aktivität aufweist,
das mutierte humane oder simiane lentivirale ENV-Protein resultiert aus der Mutation der Transmembran (TM)-Untereinheit eines humanen oder simianen lentiviralen Wildtyp-ENV-Protein, das mutierte humane oder simiane lentivirale ENV-Protein umfasst eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei,
X eine Aminosäure darstellt,
und entweder
Xₐ A, F, G, L oder R ist, und X_{b} L, I, V, M oder P ist, oder
Xₐ Y, I, H, C oder T ist, und X_{b} A, F, G oder R ist, oder
Xₐ A, F, G, L oder R ist, und X_{b} A, F, G oder R ist,
oder
b) mindestens ein Fragment des isolierten mutierten humanen oder simianen lentiviralen ENV-Proteins, das im Wesentlichen keine immunsuppressive Aktivität aufweist,
das Fragment umfasst mindestens 40 Aminosäuren,
das Fragment umfasst eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei
X eine Aminosäure darstellt,
und entweder
Xₐ A, F, G, L oder R ist, und X_{b} L, I, V, M oder P ist, oder
Xₐ Y, I, H, C oder T ist, und X_{b} A, F, G oder R ist, oder
Xₐ A, F, G, L oder R ist, und X_{b} A, F, G oder R ist,
in Gemeinschaft mit einem pharmazeutisch akzeptablen Träger,

3. Pharmazeutische Zusammensetzung nach Anspruch 1, die als Wirkstoff umfasst:
a) ein isoliertes mutiertes humanes oder simianes lentivirales ENV-Protein, das im Wesentlichen keine immunsuppressive Aktivität aufweist,
das mutierte humane oder simiane lentivirale ENV-Protein resultiert aus der Mutation der Transmembran (TM)-Untereinheit eines humanen oder simianen lentiviralen Wildtyp-ENV-Protein, das mutierte humane oder simiane lentivirale ENV-Protein umfasst eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei,
X eine Aminosäure darstellt,
und entweder
X₃ A, G, oder R ist, und X_{b} C, D, E, H, I, K, L, M, N, P, Q, S, T, V, W oder Y ist, oder
Xₐ C, D, E, H, I, K, M, N, P, Q, S, T, V, W oder Y ist, und X_{b} F, G oder R ist, oder
Xₐ F oder L ist, und X_{b} F, G oder R ist,
oder
Xₐ A, G, oder R ist, und X_{b} A ist,
oder
Xₐ A, G oder R ist, und X_{b} F, G oder R ist,
oder
b) mindestens ein Fragment des isolierten mutierten humanen oder simianen Antiviralen ENV-Protein, das im Wesentlichen keine immunsuppressive Aktivität aufweist,
das Fragment umfasst mindestens 40 Aminosäuren,
das Fragment umfasst eine mutierte immunsuppressive Domäne (ISU), die die folgende Aminosäuresequenz enthält:
A-[I/V]-E-[K/R]-Xₐ-X_{b}X-D-Q (SEQ ID NO : 416),
wobei
X eine Aminosäure darstellt,
und entweder
Xₐ A, G, oder R ist, und X_{b} C, D, E, H, I, K, L, M, N, P, Q, S, T, V, W oder Y ist,
oder
Xₐ C, D, E, H, I, K, M, N, P, Q, S, T, V, W oder Y ist, und X_{b} F, G oder R ist, oder
Xₐ F oder L ist, und X_{b} F, G oder R ist,
oder
Xₐ A, G oder R ist, und X_{b} A ist,
oder
Xₐ A, G oder R ist, und X_{b} F, G oder R ist,
in Gemeinschaft mit einem pharmazeutisch akzeptablen Träger,

4. Pharmazeutische Zusammensetzung nach Anspruch 1 bis 3, die als Wirkstoff umfasst:
a) ein isoliertes mutiertes humanes oder simianes lentivirales ENV-Protein, das im Wesentlichen keine immunsuppressive Aktivität aufweist,
das mutierte humane oder simiane lentivirale ENV-Protein resultiert aus der Mutation der Transmembran (TM)-Untereinheit eines humanen oder simianen Antiviralen Wildtyp-ENV-Protein, das mutierte humane oder simiane lentivirale ENV-Protein umfasst eine mutierte immunsuppressive Domäne (ISU), die die folgende Aminosäuresequenz enthält:
A-[I/V]-E-[K/R]-XₐX_{b}-X-D-Q (SEQ ID NO : 416),
wobei,
X eine Aminosäure darstellt,
und entweder
Xₐ A, G, oder R ist, und X_{b} L, I, V, M oder P ist,
oder
Xₐ Y, I, H, C oder T ist, und X_{b} F, G oder R ist,
oder
b) mindestens ein Fragment des isolierten mutierten humanen oder simianen lentiviralen ENV-Protein, das im Wesentlichen keine immunsuppressive Aktivität aufweist,
das Fragment umfasst mindestens 40 Aminosäuren,
das Fragment umfasst eine mutierte immunsuppressive Domäne (ISU), die die folgende Aminosäuresequenz enthält:
A-[I/V-E-[K/R]-Xₐ-X_{b}-X-D-Q (SEQ ID NO : 416),
wobei
X eine Aminosäure darstellt,
und entweder
Xₐ A, G, oder R ist, und X_{b} L, I, V, M oder P ist,
oder
Xₐ Y, I, H, C oder T ist, und X_{b} F, G oder R ist,
in Gemeinschaft mit einem pharmazeutisch akzeptablen Träger,

5. Pharmazeutische Zusammensetzung nach einem der Ansprüche 1, 3,
wobei
Xₐ R ist, und X_{b} C, D, E, H, I, K, L, M, N, P, Q, S, T, V, W oder Y ist, oder
Xₐ C, D, E, H, I, K, M, N, P, Q, S, T, V, W oder Y ist, und X_{b} R ist, oder
Xₐ R ist und X_{b} R ist, besonders wobei das isolierte humane oder simiane lentivirale ENV-Protein oder das Fragment des isolierten mutierten humanen oder simianen lentiviralen ENV-Proteins eine der folgenden Aminosäuresequenzen umfasst:
A-I-E-K-Xₐ-X_{b}-X-DQ (SEQ ID NO: 422),
A-I-E-R-Xₐ-X_{b}-X-DQ (SEQ ID NO: 423),
A-V-E-K-Xₐ-X_{b}-X-DQ (SEQ ID NO: 424),
A-V-E-R-Xₐ-X_{b}-X-DQ (SEQ ID NO: 425).

6. Pharmazeutische Zusammensetzung nach einem der Ansprüche 1 bis 5, wobei das isolierte mutierte humane oder simiane lentivirale ENV-Protein oder das Fragment des isolierten mutierten humanen oder simianen lentiviralen ENV-Proteins eine der Aminosäurefrequenzen umfasst:
SEQ ID NO: 13, SEQ ID NO: 42, SEQ ID NO: 71,
SEQ ID NO: 9 bis 12,
SEQ ID NO: 14 bis 41,
SEQ ID NO: 43 bis 70, und
SEQ ID NO: 72 bis 95,
besonders wobei das Fragment des isolierten mutierten humanen oder simianen lentiviralen ENV-Proteins eine der Aminosäuresequenzen umfasst: SEQ ID NO: 96 bis 211, besonderer wobei das isolierte mutierte humane oder simiane lentivirale ENV-Protein aus einer der Aminosäuresequenzen besteht: SEQ ID NO: 212 bis 269.

7. Pharmazeutische Zusammensetzung nach einem der Ansprüche 1 bis 6, wobei das mutierte Protein eine zusätzliche Mutation in mindestens einer der Aminosäuren an Positionen 29, 36 und 37 der SEQ ID NO: 426 umfasst:
A[I/V]E[K/R]XₐX_{b}X₁DQX₂X₃LX₄X₅WGC[A/S][F/G]X₆X₇CVX₈TX₉VPX_{c}X₁₀Z₁Z₂Z₃Z ₄Z₅X_{d}Xₑ[S/T] (SEQ ID NO: 426)
wobei
Xₐ und X_{b} wie in einem der Ansprüche 1 bis 6 definiert sind,
X₁ bis X₁₀ eine Aminosäure darstellen,
Z₁ bis Z₅ unabhängig voneinander keine Aminosäure oder eine Aminosäure darstellen, sodass
∘ X_{c} A, C, D, E, F, G, H, I, K, L, M, N, P, Q, R, S, T, V oder Y, bevorzugt A, D oder N, ist,
∘ X_{d} A, C, D, E, F, G, H, I, L, M, N, P, Q, R, S, T,V, Y oder W, bevorzugt A, G, S oder Y, ist,
_{°} Xₑ A, C, D, E, F, G, H, I, K, L, M, N, P, Q, R, T, V, Y oder W, bevorzugt A, D oder N, ist,
besonders wobei das mutierte Protein aus einer der Aminosäuresequenzen der Gruppe bestehend aus SEQ ID NO: 271 bis 283 besteht.

8. Pharmazeutische Zusammensetzung, die als Wirkstoff ein Nukleinsäuremolekül umfasst, das für ein mutiertes humanes oder simianes lentivirales ENV-Protein oder ein Fragment des mutierten humanen oder simianen lentiviralen ENV-Proteins, wie in einem der Ansprüche 1 bis 7 definiert, kodiert,
wobei das Nukleinsäuremolekül in einem Vektor enthalten ist, der Vektor Einrichtungen umfasst, die die Expression des mutierten humanen oder simianen lentiviralen ENV-Proteins oder eines Fragments des mutierten humanen oder simianen lentiviralen ENV-Proteins, wie in einem der Ansprüche 1 bis 7 definiert, ermöglichen.

9. Pharmazeutische Zusammensetzung nach Anspruch 8, wobei der Vektor aus einem Masernvektor, einem Kanarienpockenvektor, einem Pockenvektor, einem Vogelpockenvektor, einem adenoviralen Vektor, einem lentiviralen Vektor, einem Sendaïvirus, einem Cytomegalievirusvektor oder einem Modified-Vaccinia-Ankara-Vektor ausgewählt ist.

10. Pharmazeutische Zusammensetzung nach Anspruch 8 oder 9, die mindestens ein Nukleinsäuremolekül umfasst, das für ein GAG-Protein und/oder ein PRO-Protein und/oder ein POL-Protein und/oder ein mutiertes NEF-Protein, wobei
das mutierte NEF-Protein im Wesentlichen frei von immunsuppressiven Eigenschaften ist,
eines humanen oder simianen Lentivirus kodiert, das Lentivirus ist bevorzugt vom gleichen Ursprung wie das mutierte lentivirale ENV-Protein,

11. Pharmazeutische Zusammensetzung nach Anspruch 10, wobei das Nukleinsäuremolekül, das für ein mutiertes humanes oder simianes lentivirales ENV-Protein kodiert, oder ein Fragment des mutierten humanen oder simianen lentiviralen ENV-Proteins, in dem gleichen Vektor wie dem enthalten ist, welcher ebenfalls mindestens ein Nukleinsäuremolekül enthält, das für ein GAG-Protein und/oder ein PRO-Protein und/oder ein POL-Protein und/oder ein mutiertes NEF-Protein, wobei das mutierte NEF-Protein im Wesentlichen frei von immunsuppressiven Eigenschaften ist, kodiert
oder wobei das Nukleinsäuremolekül, das für ein mutiertes humanes oder simianes lentivirales ENV-Protein kodiert, oder ein Fragment des mutierten humanen oder simianen lentiviralen ENV-Proteins, in dem gleichen Vektor wie dem enthalten ist, welcher ebenfalls alle des mindestens einen Nukleinsäuremoleküls enthält, das für ein GAG-Protein und/oder ein PRO-Protein und/oder ein POL-Protein und/oder ein mutiertes NEF-Protein, wobei das mutierte NEF-Protein im Wesentlichen frei von immunsuppressiven Eigenschaften ist, kodiert,
der Vektor ist ein Masernvektor oder ein Kanarienpockenvektor.

12. Pharmazeutische Zusammensetzung nach einem der Ansprüche 1 bis 11 für ihre Verwendung für die Prävention oder die Behandlung einer lentiviralen Infektion, bevorzugt einer HIV-1-Infektion, HIV-2-Infektion oder SIV-Infektion,
besonders für ihre Verwendung als ein Impfstoff, besonders gegen eine HIV-1-Infektion, HIV-2-Infektion oder SIV-Infektion.

13. Verfahren, um den Wirkstoff einer wie in einem der Ansprüche 1 bis 12 definierten pharmazeutischen Zusammensetzung zu erhalten, bestehend aus Modifizieren der immunsuppressiven Eigenschaft von:
einem humanen oder simianen lentiviralen Wildtyp-ENV-Protein,
oder einem Fragment des humanen oder simianen lentiviralen Wildtyp-ENV-Proteins, das Fragment umfasst mindestens 40 Aminosäuren,
das ENV-Protein oder das Fragment davon präsentiert eine Transmembranuntereinheit (TM), die eine immunsuppressive Domäne (ISU) umfasst, die die folgende Aminosäuresequenz enthält:
A-[I/V]-E-[K/R]-)X'ₐ-X'_{b}X-D-Q (SEQ ID NO : 427),
wobei,
X eine Aminosäure darstellt,
X'ₐ C, D, E, H, I, K, M, N, P, Q, S, T, V, W oder Y ist, und
X'_{b} C, D, E, H, I, K, L, M, N, P, Q, S, T, V, W oder Y ist,
das Verfahren umfasst einen Schritt des Einbringens mindestens einer Mutation von X'ₐ oder X'_{b},
um zu erhalten:
ein isoliertes mutiertes humanes oder simianes lentivirales ENV-Protein, das im Wesentlichen keine immunsuppressive Aktivität aufweist,
das mutierte humane oder slmiane lentivirale ENV-Protein weist mindestens 90% Identität mit einer Sequenz ausgewählt aus der Gruppe bestehend aus SEQ ID NO : 216, SEQ ID NO : 420 und SEQ ID NO : 421 auf,
oder ein Fragment des isolierten humanen oder simianen lentiviralen ENV-Protein, das im Wesentlichen keine immunsuppressive Aktivität aufweist, das Fragment umfasst mindestens 40 Aminosäuren,
das mutierte ENV-Protein und das Fragment davon umfassen eine mutierte immunsuppressive Domäne (ISU), die die folgende Aminosäuresequenz enthält:
A-[I/V]-E-[K/R]-Xₐ-X_{b}-X-D-Q (SEQ ID NO : 416),
wobei,
X eine Aminosäure darstellt,
und entweder
Xₐ A, F, G, L oder R ist, und X_{b} C, D, E, H, I, K, L, M, N, P, Q, S, T, V, W oder Y ist, oder
Xₐ C, D, E, H, I, K, M, N, P, Q, S, T, V, W oder Y ist, und X_{b} A, F, G oder R ist, oder
Xₐ A, F, G, L oder R ist, und X_{b} A, F, G oder R ist,
die im Wesentlichen Abwesenheit der immunsuppressiven Aktivität des vorher erwähnten humanen oder simlanen lentiviralen ENV-Proteins oder des vorher definierten Fragments wird verpflichtend durch die Tatsache festgestellt, dass in einer i*n vivo*-Untersuchung, die die Abstoßung transplantierter Tumorzellen einbezieht,
die Tumorzellen entweder so transduziert werden, dass sie das mutierte ENV-Protein oder das Fragment exprimieren (mutierte ENV-Tumorzellen), oder die Tumorzellen so transduziert werden, dass sie das Wildtyp-ENV-Protein oder ein Fragment davon exprimieren (Wildtyp-ENV-Tumorzellen), oder die Tumorzellen nicht transduziert werden (normale Tumorzellen), das folgende Verhältnis:
Immunsuppressionsindex des mutierten ENV-Proteins oder des Fragments (i_{mutated env})/Immunsuppressionsindex des Wildtyp-ENV-Proteins (i_{wlld type env}) weniger als 0,5 ist,
i_{mutated env} ist definiert durch: (maximale Fläche, die durch mutierte ENV-Tumorzellen erreicht wird - maximale Fläche, die durch normale Tumorzellen erreicht wird)/(maximale Fläche, die durch normale Tumorzellen erreicht wird), und
i_{wild type env} definiert ist durch: (maximale Fläche, die durch Wildtyp-ENV-Tumorzellen erreicht wird - maximale Fläche, die durch normale Tumorzellen erreicht wird)/(maximale Fläche, die durch normale Tumorzellen erreicht wird).

14. Verfahren nach Anspruch 13, um den Wirkstoff einer wie in einem der Ansprüche 1 bis 12 definierten pharmazeutischen Zusammensetzung zu erhalten, bestehend aus der Modifikation der immunsuppressiven Eigenschaft von:
einem humanen oder simianen lentiviralen Wildtyp-ENV-Protein,
oder einem Fragment des simianen lentiviralen Wildtyp-ENV-Proteins, das Fragment umfasst mindestens 40 Aminosäuren,
das ENV-Protein oder das Fragment davon präsentiert eine Transmembranuntereinheit (TM), die eine immunsuppressive Domäne (ISU) umfasst, die die folgende Aminosäuresequenz enthält:
A-[I/V]-E-[K/R]-Y-L-X-D-Q (SEQ ID NO: 1),
wobei,
X eine Aminosäure darstellt,
das Verfahren umfasst einen Schritt des Einbringens mindestens einer Mutation von Y in Position 5 und/oder L in Position 6,
um zu erhalten:
ein isoliertes mutiertes humanes oder simianes lentivirales ENV-Protein, das im Wesentlichen keine immunsuppressive Aktivität aufweist,
oder ein Fragment des humanen oder simianen lentiviralen Wildtyp-ENV-Proteins, das Fragment umfasst mindestens 40 Aminosäuren,
das mutierte humane oder simiane lentivirale ENV-Protein und das Fragment davon umfassen eine mutierte immunsuppressive Domäne (ISU),
die die folgende Aminosäuresequenz enthält:
A-[I/V]-E-[K/R]-Xₐ-X_{b}-X-D-Q (SEQ ID NO: 416),
wobei,
X eine Aminosäure darstellt,
und entweder
Xₐ A, F, G, L oder R ist, und X_{b} C, D, E, H, I, K, L, M, N, P, Q, S, T, V, W oder Y ist, oder
Xₐ C, D, E, H, I, K, M, N, P, Q, S, T, V, W oder Y ist, und X_{b} A, F, G oder R ist, oder
Xₐ A, F, G, L oder R ist, und X_{b} A, F, G oder R ist.

15. Verfahren nach Anspruch 13 oder 14, um den Wirkstoff einer wie in einem der Ansprüche 1 bis 12 definierten pharmazeutischen Zusammensetzung zu erhalten,
wobei das isolierte mutierte humane oder simiane lentivirale ENV-Protein, das im Wesentlichen keine immunsuppressive Aktivität aufweist, oder ein Fragment des isolierten mutierten humanen oder simianen lentiviralen ENV-Proteins, das im Wesentlichen keine immunsuppressive Aktivität aufweist, das Fragment umfasst mindestens 40 Aminosäuren,
das mutierte ENV-Protein und das Fragment davon umfassen eine mutierte immunsuppressive Domäne (ISU), die die folgende Aminosäuresequenz enthält:
A-[I/V]-E-[K/R]-Xₐ-X_{b}-X-D-Q (SEQ ID NO: 416),
wobei
X eine Aminosäure darstellt,
und entweder
Xₐ A, G, oder R ist, und X_{b} L, I, V, M oder P ist, oder
Xₐ Y, I, H, C oder T ist, und X_{b} F, G oder R ist, oder
Xₐ F oder L ist, und X_{b} F, G oder R ist, oder
X₃ A, G oder R ist, und X_{b} A ist, oder
Xₐ A, G oder R ist, und X_{b} F, G oder R ist.

16. Verfahren nach einem der Ansprüche 13 bis 15, um den Wirkstoff einer wie in einem der Ansprüche 1 bis 12 definierten pharmazeutischen Zusammensetzung zu erhalten,
wobei das isolierte mutierte humane oder simiane lentivirale ENV-Protein, das im Wesentlichen keine immunsuppressive Aktivität aufweist,
oder ein Fragment des isolierten mutierten humanen oder simianen lentiviralen ENV-Proteins, das im Wesentlichen keine immunsuppressive Aktivität aufweist, das Fragment umfasst mindestens 40 Aminosäuren,
das mutierte ENV-Protein und das Fragment davon eine zusätzliche Mutation in mindestens einer der Aminosäuren an Positionen 29, 36 und 37 der SEQ ID NO: 426 umfassen:
A[I/V]E[K/R]XₐX_{b}X₁DQX₂X₃LX₄X₅WGC[A/S][F/G]X₆X₇CVX₈TX₉VPX_{c}X₁₀Z₁ Z₂Z₃Z₄Z₅X_{d}Xₑ[S/T] (SEQ ID NO: 426) wob
ei
Xₐ und X_{b} wie in einem der Ansprüche 1 bis 14 definiert sind,
X₁ bis X₁₀ eine Aminosäure darstellen
Z₁ bis Z₅ unabhängig voneinander keine Aminosäure oder eine Aminosäure darstellen, sodass
∘ X_{c} A, C, D, E, F, G, H, I, K, L, M, N, P, Q, R, S, T, V oder Y, bevorzugt A, D oder N, ist,
∘ X_{d} A, C, D, E, F, G, H, I, L, M, N, P, Q, R, S, T, V, Y oder W, bevorzugt A, G, S oder Y, ist,
° Xₑ A, C, D, E, F, G, H, I, K, L, M, N, P, Q, R, T, V, Y oder W, bevorzugt A, D oder N, ist,
besonders wobei das mutierte Protein aus einer der Aminosäuresequenzen der Gruppe bestehend aus SEQ ID NO: 271 bis 283 besteht.

## Revendications

1. Composition pharmaceutique comprenant en tant que substance active :
a) Une protéine ENV lentivirale humaine ou simienne isolée mutée n'ayant sensiblement pas d'activité immunosuppressive,
ladite protéine ENV lentivirale humaine ou simienne mutée résultant de la mutation de la sous-unité transmembranaire (TM) d'une protéine ENV lentivirale humaine ou simienne de type sauvage
ladite protéine ENV lentivirale humaine ou simienne mutée ayant au moins 90% d'identité avec une séquence choisie parmi le groupe consistant en SEQ ID NO : 216, SEQ ID NO: 420 et SEQ ID NO : 421,
ladite protéine ENV lentivirale humaine ou simienne mutée comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, F, G, L ou R et Xb est C, D, E, H, I, K, L, M, N, P, Q, S, T, V, W ou Y, ou Xa est C, D, E, H, I, K, M, N, P, Q, S, T, V, W ou Y et Xb est A, F, G ou R, ou Xa est A, F, G, L ou R, et Xb est A, F, G ou R,
ladite protéine ENV lentivirale humaine ou simienne mutée conserve la structure antigénique de la protéine ENV lentivirale humaine ou simienne de type sauvage,
ou
b) au moins un fragment de ladite protéine ENV lentivirale humaine ou simienne isolée mutée n'ayant sensiblement pas d'activité immunosuppressive,
ledit fragment comprenant au moins 40 acides aminés,
ledit fragment comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, F, G, L ou R et Xb est C, D, E, H, I, K, L, M, N, P, Q, S, T, V, W ou Y, ou
Xa est C, D, E, H, I, K, M, N, P, Q, S, T, V, W ou Y et Xb est A, F, G ou R, ou
Xa est A, F, G, L ou R, et Xb est A, F, G ou R,
ledit fragment conserve la structure antigénique de la protéine ENV lentivirale humaine ou simienne mutée isolée complète dont il dérive
en association avec un excipient pharmaceutiquement acceptable,
ladite sensible absence d'activité immunosuppressive de la protéine ENV lentivirale humaine ou simienne mutée mentionnée ci-dessus ou du fragment défini ci-dessus pouvant être évaluée par le fait que dans un test in vivo impliquant le rejet de cellules tumorales greffées,
lesdites cellules tumorales étant transduites soit pour exprimer ladite protéine ENV mutée, soit ledit fragment (cellules tumorales ENV mutées),
ou lesdites cellules tumorales étant transduites de manière à exprimer ladite protéine ENV de type sauvage ou un fragment de celle-ci (cellules tumorales ENV de type sauvage),
ou lesdites cellules tumorales n'étant pas transduites (cellules tumorales normales), le ratio suivant:
indice d'immunosuppression de ladite protéine ENV mutée ou dudit fragment (i_{env mutée}) / indice d'immunosuppression de la protéine ENV de type sauvage (i_{env sauvage}) est inférieur à 0,5,
i_{env mutée} étant défini par: (aire maximale atteinte par les cellules tumorales ENV mutées - aire maximale atteinte par les cellules tumorales normales) / (aire maximale atteinte par les cellules tumorales normales), et
i_{env sauvage} étant défini par: (aire maximale atteinte par les cellules tumorales ENV de type sauvage - aire maximale atteinte par les cellules tumorales normales) / (aire maximale atteinte par les cellules tumorales normales).

2. Composition pharmaceutique selon la revendication 1 comprenant en tant que substance active :
a) Une protéine ENV lentivirale humaine ou simienne isolée mutée n'ayant sensiblement pas d'activité immunosuppressive,
ladite protéine ENV lentivirale humaine ou simienne mutée résultant de la mutation de la sous-unité transmembranaire (TM) d'une protéine ENV lentivirale humaine ou simienne de type sauvage,
ladite protéine ENV lentivirale humaine ou simienne mutée comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, F, G, L ou R, et Xb est L, I, V, M ou P, ou
Xa est Y, I, H, C ou T, et Xb est A, F, G ou R, ou
Xa est A, F, G, L ou R, et Xb est A, F, G ou R,
ou
b) au moins un fragment de ladite protéine ENV lentivirale humaine ou simienne isolée mutée n'ayant sensiblement pas d'activité immunosuppressive,
ledit fragment comprenant au moins 40 acides aminés,
ledit fragment comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, F, G, L ou R, et Xb est L, I, V, M ou P, ou
Xa est Y, I, H, C ou T, et Xb est A, F, G ou R, ou
Xa est A, F, G, L ou R, et Xb est A, F, G ou R,
en association avec un excipient pharmaceutiquement acceptable.

3. Composition pharmaceutique selon la revendication 1 comprenant en tant que substance active :
a) Une protéine ENV lentivirale humaine ou simienne isolée mutée n'ayant sensiblement pas d'activité immunosuppressive,
ladite protéine ENV lentivirale humaine ou simienne mutée résultant de la mutation de la sous-unité transmembranaire (TM) d'une protéine ENV lentivirale humaine ou simienne de type sauvage,
ladite protéine ENV lentivirale humaine ou simienne mutée comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, G ou R, et Xb est C, D, E, H, I, K, L, M, N, P, Q, S, T, V, W ou Y, ou
Xa est C, D, E, H, I, K, M, N, P, Q, S, T, V, W ou Y et Xb est F, G ou R, ou
Xa est F ou L, et Xb est F, G ou R, ou
Xa est A, G ou R, et Xb est A, ou
Xa est A, G ou R, et Xb est F, G ou R
ou
b) au moins un fragment de ladite protéine ENV lentivirale humaine ou simienne isolée mutée n'ayant sensiblement pas d'activité immunosuppressive,
ledit fragment comprenant au moins 40 acides aminés,
ledit fragment comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, G ou R, et Xb est C, D, E, H, I, K, L, M, N, P, Q, S, T, V, W ou Y, ou
Xa est C, D, E, H, I, K, M, N, P, Q, S, T, V, W ou Y et Xb est F, G ou R, ou
Xa est F ou L, et Xb est F, G ou R, ou
Xa est A, G ou R, et Xb est A, ou
Xa est A, G ou R, et Xb est F, G ou R
en association avec un excipient pharmaceutiquement acceptable.

4. Composition pharmaceutique selon les revendications 1 à 3 comprenant en tant que substance active :
a) Une protéine ENV lentivirale humaine ou simienne isolée mutée n'ayant sensiblement pas d'activité immunosuppressive,
ladite protéine ENV lentivirale humaine ou simienne mutée résultant de la mutation de la sous-unité transmembranaire (TM) d'une protéine ENV lentivirale humaine ou simienne de type sauvage,
ladite protéine ENV lentivirale humaine ou simienne mutée comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, G ou R, et Xb est L, I, V, M ou P, ou
Xa est Y, 1, H, C ou T, et Xb est F, G ou R,
ou
b) au moins un fragment de ladite protéine ENV lentivirale humaine ou simienne isolée mutée n'ayant sensiblement pas d'activité immunosuppressive,
ledit fragment comprenant au moins 40 acides aminés,
ledit fragment comprenant un domaine immunosuppresseur (ISU) muté contenant la séquence d'acides aminés suivante:
A-[I/V]-E-[K/R]-Xa-Xb-X-D-Q (SEQ ID NO: 416),
où,
X représente n'importe quel acide aminé,
et soit
Xa est A, G ou R, et Xb est L, I, V, M ou P, ou
Xa est Y, I, H, C ou T, et Xb est F, G ou R,
en association avec un excipient pharmaceutiquement acceptable.

5. Composition pharmaceutique selon l'une quelconque des revendications 1, 3 où
Xa est R et Xb est C, D, E, H, I, K, L, M, N, P, Q, S, T, V, W ou Y, ou
Xa est C, D, E, H, I, K, M, N, P, Q, S, T, V, W ou Y et Xb est R, ou
Xa est R et Xb est R, en particulier dans laquelle ladite protéine ENV lentivirale humaine ou simienne isolée mutée ou ledit fragment de ladite protéine ENV lentivirale humaine ou simienne isolée mutée comprend l'une des séquences d'acides aminés suivantes:
A-I-E-K-Xa-Xb-X-DQ (SEQ ID NO: 422),
A-I-E-R-Xa-Xb-X-DQ (SEQ ID NO: 423),
A-V-E-K-Xa-Xb-X-DQ (SEQ ID NO: 424),
A-V-E-R-Xa-Xb-X-DQ (SEQ ID NO: 425).

6. Composition pharmaceutique selon l'une quelconque des revendications 1 à 5, dans laquelle ladite protéine ENV lentivirale humaine ou simienne isolée mutée ou ledit fragment de ladite protéine ENV lentivirale humaine ou simienne isolée mutée comprend l'une des séquences d'acides aminés :
SEQ ID NO : 13, SEQ ID NO : 42, SEQ ID NO : 71,
SEQ ID NO : 9 à 12,
SEQ ID NO: 14 à 41,
SEQ ID NO : 43 à 70 et
SEQ ID NO : 72 à 95,
en particulier dans lequel ledit fragment de ladite protéine ENV lentivirale humaine ou simienne isolée mutée comprend l'une des séquences d'acides aminés: SEQ ID NO: 96 à 211, plus particulièrement dans laquelle ladite protéine ENV lentivirale humaine ou simienne isolée mutée consiste en l'un des acides aminés séquences: SEQ ID NO: 212 à 269.

7. Composition pharmaceutique selon l'une quelconque des revendications 1 à 6, dans laquelle ladite protéine mutée comprend une mutation supplémentaire de l'un au moins des acides aminés aux positions 29, 36 et 37 de la SEQ ID NO: 426:
A[I/V]E[K/R]XₐX_{b}X₁DQX₂X₃LX₄X₅WGC[A/S][F/G]X₆X₇CVX₈TX₉VPX_{c}X₁₀Z₁Z₂Z₃ Z₄Z₅X_{d}Xₑ[S/T] (SEQ ID NO: 426)
où
Xa et Xb sont tels que définis dans l'une quelconque des revendications 1 à 6,
X₁ à X₁₀ représentent n'importe quel acide aminé,
Z₁ à Z₅ ne représentent aucun acide aminé ou n'importe quel acide aminé, indépendamment l'un de l'autre
tel que
∘ Xc est A, C, D, E, F, G, H, I, K, L, M, N, P, Q, R, S, T, V ou Y, de préférence A, D ou N,
∘ Xd est A, C, D, E, F, G, H, I, L, M, N, P, Q, R, S, T, V, Y ou W, de préférence A, G, S ou Y,
∘ Xe est A, C, D, E, F, G, H, I, K, L, M, N, P, Q, R, T, V, Y ou W, de préférence A, D ou N,
en particulier dans laquelle ladite protéine mutée consiste en l'une des séquences d'acides aminés du groupe consistant en SEQ ID NO: 271 à 283.

8. Composition pharmaceutique comprenant en tant que substance active une molécule d'acide nucléique codant pour une protéine ENV lentivirale humaine ou simienne mutée, ou un fragment de ladite protéine ENV lentivirale humaine ou simienne mutée, tel que défini dans l'une quelconque des revendications 1 à 7,
dans laquelle ladite molécule d'acide nucléique étant contenue dans un vecteur, ledit vecteur comprenant des moyens permettant l'expression de la protéine ENV lentivirale humaine ou simienne mutée, ou un fragment de ladite protéine ENV lentivirale humaine ou simienne mutée, tel que défini dans l'une quelconque des revendications 1 à 7.

9. Composition pharmaceutique selon la revendication 8, ledit vecteur étant choisi parmi un vecteur de la rougeole, un vecteur du virus de la variole du canari, un vecteur du virus de la variole, du virus de la variole aviaire, un vecteur adénoviral, un vecteur lentiviral, un virus Sendaï, un vecteur Cytomégalovirus ou un vecteur du virus Ankara de la vaccine modifié.

10. Composition pharmaceutique selon la revendication 8 ou 9, comprenant au moins une molécule d'acide nucléique codant pour une protéine GAG et/ou une protéine PRO et/ou une protéine POL et/ou une protéine NEF mutée, dans laquelle ladite protéine NEF mutée est sensiblement dépourvue de propriétés immunosuppressives,
d'un lentivirus humain ou simien, ledit lentivirus étant de préférence de même origine que la protéine ENV lentivirale mutée.

11. Composition pharmaceutique selon la revendication 10, dans laquelle ladite molécule d'acide nucléique codant pour une protéine ENV lentivirale humaine ou simienne mutée, ou un fragment de ladite protéine ENV lentivirale humaine ou simienne mutée, est contenue dans le même vecteur que celui contenant également ladite au moins une molécule d'acide nucléique codant pour une protéine GAG et/ou une protéine PRO et/ou une protéine POL et/ou une protéine NEF mutée, ladite protéine NEF mutée étant sensiblement dépourvu de propriétés immunosuppressives,
ou dans laquelle ladite molécule d'acide nucléique codant pour une protéine ENV lentivirale humaine ou simienne mutée, ou un fragment de ladite protéine ENV lentivirale humaine ou simienne mutée, est contenue dans le même vecteur que celui contenant également toutes lesdites au moins une molécule d'acide nucléique codant pour une protéine GAG et/ou une protéine PRO et/ou une protéine POL et/ou une protéine NEF mutée, ladite protéine NEF mutée étant sensiblement dépourvue de propriétés immunosuppressives,
ledit vecteur étant un vecteur de la rougeole ou un vecteur de la variole du canari.

12. Composition pharmaceutique selon l'une quelconque des revendications 1 à 11, pour son utilisation pour la prévention ou le traitement d'une infection lentivirale, de préférence une infection par le VIH-1, une infection par le VIH-2 ou une infection par le SIV,
en particulier pour son utilisation en tant que vaccin, en particulier contre l'infection par le VIH-1, l'infection par le VIH-2 ou l'infection par le SIV.

13. Méthode pour obtenir la substance active d'une composition pharmaceutique, telle que définie dans l'une quelconque des revendications 1 à 12, consistant en la modification de la propriété immunosuppressive de:
une protéine ENV lentivirale humaine ou simienne de type sauvage,
ou un fragment de ladite protéine ENV lentivirale humaine ou simienne de type sauvage, ledit fragment comprenant au moins 40 acides aminés,
ladite protéine ENV ou fragment de celle-ci présentant une sous-unité transmembranaire (TM) comprenant un domaine immunosuppresseur (ISU) contenant la séquence d'acides aminés suivante:
A-[I/V]-E-[K/R.]-X'a-X'b-X-D-Q (SEQ ID NO: 427),
où
X représente n'importe quel acide aminé,
X'a est C, D, E, H, I, K, M, N, P, Q, S, T, V, W ou Y et
X'b est C, D, E, H, I, K, L, M, N, P, Q, S, T, V, W ou Y,
ladite méthode comprenant une étape d'introduction d'au moins une mutation de X'a et/ou X'b,
pour obtenir:
une protéine ENV lentivirale humaine ou simienne isolée mutée n'ayant sensiblement pas d'activité immunosuppressive,
ladite protéine ENV lentivirale humaine ou simienne mutée ayant au moins 90% d'identité avec une séquence choisie parmi le groupe consistant en SEQ ID NO: 216, SEQ ID NO : 420 et SEQ ID NO : 421,
ou un fragment de ladite protéine ENV lentivirale humaine ou simienne isolée mutée n'ayant sensiblement pas d'activité immunosuppressive, ledit fragment comprenant au moins 40 acides aminés,
ladite protéine ENV mutée et fragment de celle-ci comprenant un domaine immunosuppresseur (ISU) muté contenant la séquence amino suivante:
A-[I/V]-E-[K/R]-Xa-Xb-X-D-Q (SEQ ID NO: 416),
où
X représente n'importe quel acide aminé,
et soit
Xa est A, F, G, L ou R et Xb est C, D, E, H, I, K, L, M, N, P, Q, S, T, V, W ou Y, ou
Xa est C, D, E, H, I, K, M, N, P, Q, S, T, V, W ou Y et Xb est A, F, G ou R, ou
Xa est A, F, G, L ou R, et Xb est A, F, G ou R,
ladite sensible absence d'activité immunosuppressive de la protéine ENV lentivirale humaine ou simienne mutée mentionnée ci-dessus ou du fragment défini ci-dessus pouvant être évaluée par le fait que dans un test in vivo impliquant le rejet de cellules tumorales greffées,
lesdites cellules tumorales étant transduites soit de manière à exprimer ladite protéine ENV mutée, soit ledit fragment (cellules tumorales ENV mutées),
ou lesdites cellules tumorales étant transduites de manière à exprimer ladite protéine ENV de type sauvage ou un fragment de celle-ci (cellules tumorales ENV de type sauvage),
ou lesdites cellules tumorales n'étant pas transduites (cellules tumorales normales),
le ratio suivant:
indice d'immunosuppression de ladite protéine ENV mutée ou dudit fragment (i _{env mutée}) / indice d'immunosuppression de la protéine ENV de type sauvage (i _{env sauvage}) est inférieur à 0,5,
i_{env mutée} étant défini par: (aire maximale atteinte par les cellules tumorales ENV mutées - aire maximale atteinte par les cellules tumorales normales) / (aire maximale atteinte par les cellules tumorales normales), et
i_{env sauvage} étant défini par: (aire maximale atteinte par les cellules tumorales ENV de type sauvage - aire maximale atteinte par les cellules tumorales normales) / (aire maximale atteinte par les cellules tumorales normales).

14. Méthode pour obtenir la substance active d'une composition pharmaceutique, telle que définie dans l'une quelconque des revendications 1 à 12, consistant en la modification de la propriété immunosuppressive de:
une protéine ENV lentivirale humaine ou simienne de type sauvage,
ou un fragment de ladite protéine ENV lentivirale humaine ou simienne de type sauvage, ledit fragment comprenant au moins 40 acides aminés,
ladite protéine ENV ou fragment de celle-ci présentant une sous-unité transmembranaire (TM) comprenant un domaine immunosuppresseur (ISU) contenant la séquence d'acides aminés suivante:
A-[I/V]-E-[K/R]-Y-L-X-D-Q (SEQ ID NO : 1),
où
X représente n'importe quel acide aminé,
ladite méthode comprenant une étape d'introduction d'au moins une mutation de Y en position 5 et/ou L en position 6,
pour obtenir:
une protéine ENV lentivirale humaine ou simienne isolée mutée n'ayant sensiblement pas d'activité immunosuppressive,
ou un fragment de ladite protéine ENV lentivirale humaine ou simienne isolée mutée n'ayant sensiblement pas d'activité immunosuppressive, ledit fragment comprenant au moins 40 acides aminés,
ladite protéine ENV mutée et fragment de celle-ci comprenant un domaine immunosuppresseur (ISU) muté contenant la séquence amino suivante:
A-[I/V]-E-[K/R]-Xa-Xb-X-D-Q (SEQ ID NO: 416),
où
X représente n'importe quel acide aminé,
et soit
Xa est A, F, G, L ou R, et Xb est C, D, E, H, I, K, L, M, N, P, Q, S, T, V, W ou Y, ou
Xa est C, D, E, H, I, K, M, N, P, Q, S, T, V, W ou Y, et Xb est A, F, G ou R, ou
Xa est A, F, G, L ou R, et Xb est A, F, G ou R.

15. Méthode pour obtenir la substance active d'une composition pharmaceutique, telle que définie dans l'une quelconque des revendications 1 à 12, selon la revendication 13 ou 14,
dans laquelle ladite protéine ENV lentivirale humaine ou simienne isolée mutée n'ayant sensiblement pas d'activité immunosuppressive,
ou un fragment de ladite protéine ENV lentivirale humaine ou simienne isolée mutée n'ayant sensiblement pas d'activité immunosuppressive, ledit fragment comprenant au moins 40 acides aminés,
ladite protéine ENV mutée et fragment de celle-ci comprenant un domaine immunosuppresseur (ISU) muté contenant la séquence amino suivante:
A-[I/V]-E-[K/R]-Xa-Xb-X-D-Q (SEQ ID NO: 416),
où
X représente n'importe quel acide aminé,
et soit
Xa est A, G ou R et Xb est L, I, V, M ou P, ou
Xa est Y, I, H, C ou T et Xb est F, G ou R, ou
Xa est F ou L et Xb est F, G ou R, ou
Xa est A, G ou R et Xb est A ou
Xa est A, G ou R et Xb est F, G ou R.

16. Méthode pour obtenir la substance active d'une composition pharmaceutique, telle que définie dans l'une quelconque des revendications 1 à 12, selon l'une quelconque des revendications 13 à 15,
dans laquelle ladite protéine ENV lentivirale humaine ou simienne isolée mutée n'ayant sensiblement pas d'activité immunosuppressive,
ou un fragment de ladite protéine ENV lentivirale humaine ou simienne isolée mutée n'ayant sensiblement pas d'activité immunosuppressive, ledit fragment comprenant au moins 40 acides aminés,
ladite protéine ENV mutée et fragment de celle-ci comprenant une mutation supplémentaire dans l'un au moins des acides aminés aux positions 29, 36 et 37 de la SEQ ID NO: 426:
A[I/V]E[K/R]XₐX_{b}X₁DQX₂X₃LX₄X₅WGC[A/S][F/G]X₆X₇CVX₈TX₉VPX_{c}X₁₀Z₁Z₂Z₃ Z₄Z₅X_{d}Xₑ[S/T] (SEQ ID NO: 426)
où
Xa et Xb sont tels que définis dans l'une quelconque des revendications 1 à 14,
X1 à X10 représentent n'importe quel acide aminé,
Z1 à Z5 ne représentent aucun acide aminé n'importe quel acide aminé, indépendamment l'un de l'autre
tel que
∘ Xc est A, C, D, E, F, G, H, I, K, L, M, N, P, Q, R, S, T, V ou Y, de préférence A, D ou N,
∘ Xd est A, C, D, E, F, G, H, I, L, M, N, P, Q, R, S, T, V, Y ou W, de préférence A, G, S ou Y,
∘ Xe est A, C, D, E, F, G, H, I, K, L, M, N, P, Q, R, T, V, Y ou W, de préférence A, D ou N,
en particulier dans laquelle le fragment de ladite protéine ENV mutée consiste en l'une des séquences d'acides aminés du groupe constitué de SEQ ID NO: 271 à 283.


### SEQUENCE LISTING

<110> CENTRE NATIONAL DE LA RECHERCHE SCIENTIFIQUE
   INSTITUT GUSTAVE ROUSSY
   UNIVERSITE PARIS SUD XI
   VIROXIS SAS
<120> MUTATED LENTIVIRAL ENV PROTEINS AND THEIR USE AS DRUGS
<130> WOB 10 VIR ARGI
<160> 428
<170> PatentIn version 3.5
<210> 1
   <211> 9
   <212> PRT
   <213> Artificial Sequence
<220>
   <223> fragment of lentiviral ENV ISU
<220>
   <221> MISC_FEATURE
   <222> (2)..(2)
   <223> Xaa = Ile or Val
<220>
   <221> MISC_FEATURE
   <222> (4)..(4)
   <223> Xaa = K or R
<220>
   <221> misc_feature
   <222> (7)..(7)
   <223> Xaa can be any naturally occurring amino acid
<400> 1
   Ala Xaa Glu Xaa Tyr Leu Xaa Asp Gln
   1 5
<210> 2
   <211> 9
   <212> PRT
   <213> Artificial Sequence
<220>
   <223> fragement of lentiviral ENV ISU
<220>
   <221> misc_feature
   <222> (7)..(7)
   <223> Xaa can be any naturally occurring amino acid
<400> 2
   Ala Ile Glu Lys Tyr Leu Xaa Asp Gln
   1 5
<210> 3
   <211> 9
   <212> PRT
   <213> Artificial Sequence
<220>
   <223> fragement of lentiviral ENV ISU
<220>
   <221> misc_feature
   <222> (7)..(7)
   <223> Xaa can be any naturally occurring amino acid
<400> 3
   Ala Ile Glu Arg Tyr Leu Xaa Asp Gln
   1 5
<210> 4
   <211> 9
   <212> PRT
   <213> Artificial Sequence
<220>
   <223> fragment of lentiviral ENV ISU
<220>
   <221> misc_feature
   <222> (7)..(7)
   <223> Xaa can be any naturally occurring amino acid
<400> 4
   Ala Val Glu Lys Tyr Leu Xaa Asp Gln
   1               5
<210> 5
   <211> 9
   <212> PRT
   <213> Artificial Sequence
<220>
   <223> fragment of lentiviral ENV ISU
<220>
   <221> misc_feature
   <222> (7)..(7)
   <223> Xaa can be any naturally occurring amino acid
<400> 5
   Ala Val Glu Arg Tyr Leu Xaa Asp Gln
   1               5
<210> 6
   <211> 49
   <212> PRT
   <213> Artificial Sequence
<220>
   <223> fragment of lentiviral ENV ISU
<220>
   <221> misc_feature
   <222> (1)..(1)
   <223> Xaa can be any naturally occurring amino acid
<220>
   <221> misc_feature
   <222> (8)..(9)
   <223> Xaa can be any naturally occurring amino acid
<220>
   <221> misc_feature
   <222> (12)..(17)
   <223> Xaa can be any naturally occurring amino acid
<220>
   <221> misc_feature
   <222> (19)..(20)
   <223> Xaa can be any naturally occurring amino acid
<220>
   <221> misc_feature
   <222> (22)..(22)
   <223> Xaa can be any naturally occurring amino acid
<220>
   <221> misc_feature
   <222> (24)..(25)
   <223> Xaa can be any naturally occurring amino acid
<220>
   <221> misc_feature
   <222> (28)..(28)
   <223> Xaa can be any naturally occurring amino acid
<220>
   <221> misc_feature
   <222> (30)..(30)
   <223> Xaa can be any naturally occurring amino acid
<220>
   <221> misc_feature
   <222> (33)..(33)
   <223> Xaa can be any naturally occurring amino acid
<220>
   <221> misc_feature
   <222> (35)..(36)
   <223> Xaa can be any naturally occurring amino acid
<220>
   <221> MISC_FEATURE
   <222> (38)..(38)
   <223> Xaa = I or V
<220>
   <221> MISC_FEATURE
   <222> (40)..(40)
   <223> Xaa = R or K
<220>
   <221> misc_feature
   <222> (43)..(43)
   <223> Xaa can be any naturally occurring amino acid
<220>
   <221> misc_feature
   <222> (46)..(47)
   <223> Xaa can be any naturally occurring amino acid
<220>
   <221> misc_feature
   <222> (49)..(49)
   <223> Xaa can be any naturally occurring amino acid
<400> 6
   Xaa Gly Ile Val Gln Gln Gln Xaa Xaa Leu Leu Xaa Xaa Xaa Xaa Xaa
   1 5 10 15
   Xaa Gln Xaa Xaa Leu Xaa Leu Xaa Xaa Trp Gly Xaa Lys Xaa Leu Gln
   20 25 30
   Xaa Arg Xaa Xaa Ala Xaa Glu Xaa Tyr Leu Xaa Asp Gln Xaa Xaa Leu
   35 40 45
   Xaa
<210> 7
   <211> 9
   <212> PRT
   <213> Artificial Sequence
<220>
   <223> fragment of lentiviral ENV ISU
<400> 7
   Ala Val Glu Arg Tyr Leu Lys Asp Gln
   1 5
<210> 8
   <211> 9
   <212> PRT
   <213> Artificial Sequence
<220>
   <223> fragment of lentiviral ENV ISU
<400> 8
   Ala Ile Glu Lys Tyr Leu Lys Asp Gln
   1 5
<210> 9
   <211> 8
   <212> PRT
   <213> Artificial Sequence
<220>
   <223> fragment of lentiviral ENV ISU
<400> 9
   Ala Val Glu Arg Ala Leu Lys Asp
   1 5
<210> 10
   <211> 8
   <212> PRT
   <213> Artificial sequence
<220>
   <223> fragment of lentiviral ENV ISU
<400> 10
   Ala Val Glu Arg Phe Leu Lys Asp
   1               5
<210> 11
   <211> 8
   <212> PRT
   <213> Artificial sequence
<220>
   <223> fragment of lentiviral ENV ISU
<400> 11
   Ala Val Glu Arg Gly Leu Lys Asp
   1               5
<210> 12
   <211> 8
   <212> PRT
   <213> Artificial sequence
<220>
   <223> fragment of lentiviral ENV ISU
<400> 12
   Ala Val Glu Arg Leu Leu Lys Asp
   1               5
<210> 13
   <211> 8
   <212> PRT
   <213> Artificial sequence
<220>
   <223> fragment of lentiviral ENV ISU
<400> 13
   Ala Val Glu Arg Arg Leu Lys Asp
   1               5
<210> 14
   <211> 8
   <212> PRT
   <213> Artificial sequence
<220>
   <223> fragment of lentiviral ENV ISU
<400> 14
   Ala Val Glu Arg Tyr Ala Lys Asp
   1 5
<210> 15
   <211> 8
   <212> PRT
   <213> Artificial sequence
<220>
   <223> fragment of lentiviral ENV ISU
<400> 15
   Ala Val Glu Arg Tyr Phe Lys Asp
   1 5
<210> 16
   <211> 8
   <212> PRT
   <213> Artificial sequence
<220>
   <223> fragment of lentiviral ENV ISU
<400> 16
   Ala Val Glu Arg Tyr Gly Lys Asp
   1 5
<210> 17
   <211> 8
   <212> PRT
   <213> Artificial sequence
<220>
   <223> fragment of lentiviral ENV ISU
<400> 17
   Ala Val Glu Arg Tyr Arg Lys Asp
   1 5
<210> 18
   <211> 8
   <212> PRT
   <213> Artificial sequence
<220>
   <223> fragment of lentiviral ENV ISU
<400> 18
   Ala Val Glu Arg Ala Ala Lys Asp
   1 5
<210> 19
   <211> 8
   <212> PRT
   <213> Artificial sequence
<220>
   <223> fragment of lentiviral ENV ISU
<400> 19
   Ala Val Glu Arg Ala Phe Lys Asp
   1 5
<210> 20
   <211> 8
   <212> PRT
   <213> Artificial sequence
<220>
   <223> fragment of lentiviral ENV ISU
<400> 20
   Ala Val Glu Arg Ala Gly Lys Asp
   1 5
<210> 21
   <211> 8
   <212> PRT
   <213> Artificial sequence
<220>
   <223> fragment of lentiviral ENV ISU
<400> 21
   Ala Val Glu Arg Ala Arg Lys Asp
   1 5
<210> 22
   <211> 8
   <212> PRT
   <213> Artificial sequence
<220>
   <223> fragment of lentiviral ENV ISU
<400> 22
   Ala Val Glu Arg Phe Ala Lys Asp
   1 5
<210> 23
   <211> 8
   <212> PRT
   <213> Artificial sequence
<220>
   <223> fragment of lentiviral ENV ISU
<400> 23
   Ala Val Glu Arg Phe Phe Lys Asp
   1 5
<210> 24
   <211> 8
   <212> PRT
   <213> Artificial sequence
<220>
   <223> fragment of lentiviral ENV ISU
<400> 24
   Ala Val Glu Arg Phe Gly Lys Asp
   1 5
<210> 25
   <211> 8
   <212> PRT
   <213> Artificial sequence
<220>
   <223> fragment of lentiviral ENV ISU
<400> 25
   Ala Val Glu Arg Phe Arg Lys Asp
   1 5
<210> 26
   <211> 8
   <212> PRT
   <213> Artificial sequence
<220>
   <223> fragment of lentiviral ENV ISU
<400> 26
   Ala Val Glu Arg Gly Ala Lys Asp
   1 5
<210> 27
   <211> 8
   <212> PRT
   <213> Artificial sequence
<220>
   <223> fragment of lentiviral ENV ISU
<400> 27
   Ala Val Glu Arg Gly Phe Lys Asp
   1               5
<210> 28
   <211> 8
   <212> PRT
   <213> Artificial sequence
<220>
   <223> fragment of lentiviral ENV ISU
<400> 28
   Ala Val Glu Arg Gly Gly Lys Asp
   1               5
<210> 29
   <211> 8
   <212> PRT
   <213> Artificial sequence
<220>
   <223> fragment of lentiviral ENV ISU
<400> 29
   Ala Val Glu Arg Gly Arg Lys Asp
   1               5
<210> 30
   <211> 8
   <212> PRT
   <213> Artificial sequence
<220>
   <223> fragment of lentiviral ENV ISU
<400> 30
   Ala Val Glu Arg Leu Ala Lys Asp
   1               5
<210> 31
   <211> 8
   <212> PRT
   <213> Artificial sequence
<220>
   <223> fragment of lentiviral ENV ISU
<400> 31
   Ala Val Glu Arg Leu Phe Lys Asp
   1               5
<210> 32
   <211> 8
   <212> PRT
   <213> Artificial sequence
<220>
   <223> fragment of lentiviral ENV ISU
<400> 32
   Ala Val Glu Arg Leu Gly Lys Asp
   1 5
<210> 33
   <211> 8
   <212> PRT
   <213> Artificial sequence
<220>
   <223> fragment of lentiviral ENV ISU
<400> 33
   Ala Val Glu Arg Leu Arg Lys Asp
   1 5
<210> 34
   <211> 8
   <212> PRT
   <213> Artificial sequence
<220>
   <223> fragment of lentiviral ENV ISU
<400> 34
   Ala Val Glu Arg Arg Ala Lys Asp
   1 5
<210> 35
   <211> 8
   <212> PRT
   <213> Artificial sequence
<220>
   <223> fragment of lentiviral ENV ISU
<400> 35
   Ala Val Glu Arg Arg Phe Lys Asp
   1 5
<210> 36
   <211> 8
   <212> PRT
   <213> Artificial sequence
<220>
   <223> fragment of lentiviral ENV ISU
<400> 36
   Ala Val Glu Arg Arg Gly Lys Asp
   1 5
<210> 37
   <211> 8
   <212> PRT
   <213> Artificial sequence
<220>
   <223> fragment of lentiviral ENV ISU
<400> 37
   Ala Val Glu Arg Arg Arg Lys Asp
   1 5
<210> 38
   <211> 8
   <212> PRT
   <213> Artificial sequence
<220>
   <223> fragement of lentiviral ENV ISU
<400> 38
   Ala Ile Glu Lys Ala Leu Lys Asp
   1 5
<210> 39
   <211> 8
   <212> PRT
   <213> Artificial sequence
<220>
   <223> fragement of lentiviral ENV ISU
<400> 39
   Ala Ile Glu Lys Phe Leu Lys Asp
   1 5
<210> 40
   <211> 8
   <212> PRT
   <213> Artificial sequence
<220>
   <223> fragement of lentiviral ENV ISU
<400> 40
   Ala Ile Glu Lys Gly Leu Lys Asp
   1 5
<210> 41
   <211> 8
   <212> PRT
   <213> Artificial sequence
<220>
   <223> fragement of lentiviral ENV ISU
<400> 41
   Ala Ile Glu Lys Leu Leu Lys Asp
   1 5
<210> 42
   <211> 8
   <212> PRT
   <213> Artificial sequence
<220>
   <223> fragement of lentiviral ENV ISU
<400> 42
   Ala Ile Glu Lys Arg Leu Lys Asp
   1 5
<210> 43
   <211> 8
   <212> PRT
   <213> Artificial sequence
<220>
   <223> fragement of lentiviral ENV ISU
<400> 43
   Ala Ile Glu Lys Tyr Ala Lys Asp
   1 5
<210> 44
   <211> 8
   <212> PRT
   <213> Artificial sequence
<220>
   <223> fragement of lentiviral ENV ISU
<400> 44
   Ala Ile Glu Lys Tyr Phe Lys Asp
   1 5
<210> 45
   <211> 8
   <212> PRT
   <213> Artificial sequence
<220>
   <223> fragement of lentiviral ENV ISU
<400> 45
   Ala Ile Glu Lys Tyr Gly Lys Asp
   1               5
<210> 46
   <211> 8
   <212> PRT
   <213> Artificial sequence
<220>
   <223> fragement of lentiviral ENV ISU
<400> 46
   Ala Ile Glu Lys Tyr Arg Lys Asp
   1               5
<210> 47
   <211> 8
   <212> PRT
   <213> Artificial sequence
<220>
   <223> fragement of lentiviral ENV ISU
<400> 47
   Ala Ile Glu Lys Ala Ala Lys Asp
   1               5
<210> 48
   <211> 8
   <212> PRT
   <213> Artificial sequence
<220>
   <223> fragement of lentiviral ENV ISU
<400> 48
   Ala Ile Glu Lys Ala Phe Lys Asp
   1               5
<210> 49
   <211> 8
   <212> PRT
   <213> Artificial sequence
<220>
   <223> fragement of lentiviral ENV ISU
<400> 49
   Ala Ile Glu Lys Ala Gly Lys Asp
   1 5
<210> 50
   <211> 8
   <212> PRT
   <213> Artificial sequence
<220>
   <223> fragement of lentiviral ENV ISU
<400> 50
   Ala Ile Glu Lys Ala Arg Lys Asp
   1 5
<210> 51
   <211> 8
   <212> PRT
   <213> Artificial sequence
<220>
   <223> fragement of lentiviral ENV ISU
<400> 51
   Ala Ile Glu Lys Phe Ala Lys Asp
   1 5
<210> 52
   <211> 8
   <212> PRT
   <213> Artificial sequence
<220>
   <223> fragement of lentiviral ENV ISU
<400> 52
   Ala Ile Glu Lys Phe Phe Lys Asp
   1 5
<210> 53
   <211> 8
   <212> PRT
   <213> Artificial sequence
<220>
   <223> fragement of lentiviral ENV ISU
<400> 53
   Ala Ile Glu Lys Phe Gly Lys Asp
   1 5
<210> 54
   <211> 8
   <212> PRT
   <213> Artificial sequence
<220>
   <223> fragement of lentiviral ENV ISU
<400> 54
   Ala Ile Glu Lys Phe Arg Lys Asp
   1 5
<210> 55
   <211> 8
   <212> PRT
   <213> Artificial sequence
<220>
   <223> fragement of lentiviral ENV ISU
<400> 55
   Ala Ile Glu Lys Gly Ala Lys Asp
   1 5
<210> 56
   <211> 8
   <212> PRT
   <213> Artificial sequence
<220>
   <223> fragement of lentiviral ENV ISU
<400> 56
   Ala Ile Glu Lys Gly Phe Lys Asp
   1 5
<210> 57
   <211> 8
   <212> PRT
   <213> Artificial sequence
<220>
   <223> fragement of lentiviral ENV ISU
<400> 57
   Ala Ile Glu Lys Gly Gly Lys Asp
   1 5
<210> 58
   <211> 8
   <212> PRT
   <213> Artificial sequence
<220>
   <223> fragement of lentiviral ENV ISU
<400> 58
   Ala Ile Glu Lys Gly Arg Lys Asp
   1 5
<210> 59
   <211> 8
   <212> PRT
   <213> Artificial sequence
<220>
   <223> fragement of lentiviral ENV ISU
<400> 59
   Ala Ile Glu Lys Leu Ala Lys Asp
   1 5
<210> 60
   <211> 8
   <212> PRT
   <213> Artificial sequence
<220>
   <223> fragement of lentiviral ENV ISU
<400> 60
   Ala Ile Glu Lys Leu Phe Lys Asp
   1 5
<210> 61
   <211> 8
   <212> PRT
   <213> Artificial sequence
<220>
   <223> fragement of lentiviral ENV ISU
<400> 61
   Ala Ile Glu Lys Leu Gly Lys Asp
   1 5
<210> 62
   <211> 8
   <212> PRT
   <213> Artificial sequence
<220>
   <223> fragement of lentiviral ENV ISU
<400> 62
   Ala Ile Glu Lys Leu Arg Lys Asp
   1 5
<210> 63
   <211> 8
   <212> PRT
   <213> Artificial sequence
<220>
   <223> fragement of lentiviral ENV ISU
<400> 63
   Ala Ile Glu Lys Arg Ala Lys Asp
   1 5
<210> 64
   <211> 8
   <212> PRT
   <213> Artificial sequence
<220>
   <223> fragement of lentiviral ENV ISU
<400> 64
   Ala Ile Glu Lys Arg Phe Lys Asp
   1 5
<210> 65
   <211> 8
   <212> PRT
   <213> Artificial sequence
<220>
   <223> fragement of lentiviral ENV ISU
<400> 65
   Ala Ile Glu Lys Arg Gly Lys Asp
   1 5
<210> 66
   <211> 8
   <212> PRT
   <213> Artificial Sequence
<220>
   <223> fragement of lentiviral ENV ISU
<400> 66
   Ala Ile Glu Lys Arg Arg Lys Asp
   1 5
<210> 67
   <211> 8
   <212> PRT
   <213> Artificial sequence
<220>
   <223> fragement of lentiviral ENV ISU
<400> 67
   Ala Ile Glu Arg Ala Leu Lys Asp
   1 5
<210> 68
   <211> 8
   <212> PRT
   <213> Artificial sequence
<220>
   <223> fragement of lentiviral ENV ISU
<400> 68
   Ala Ile Glu Arg Phe Leu Lys Asp
   1 5
<210> 69
   <211> 8
   <212> PRT
   <213> Artificial sequence
<220>
   <223> fragement of lentiviral ENV ISU
<400> 69
   Ala Ile Glu Arg Gly Leu Lys Asp
   1 5
<210> 70
   <211> 8
   <212> PRT
   <213> Artificial sequence
<220>
   <223> fragement of lentiviral ENV ISU
<400> 70
   Ala Ile Glu Arg Leu Leu Lys Asp
   1 5
<210> 71
   <211> 8
   <212> PRT
   <213> Artificial sequence
<220>
   <223> fragement of lentiviral ENV ISU
<400> 71
   Ala Ile Glu Arg Arg Leu Lys Asp
   1 5
<210> 72
   <211> 8
   <212> PRT
   <213> Artificial sequence
<220>
   <223> fragement of lentiviral ENV ISU
<400> 72
   Ala Ile Glu Arg Tyr Ala Lys Asp
   1 5
<210> 73
   <211> 8
   <212> PRT
   <213> Artificial sequence
<220>
   <223> fragement of lentiviral ENV ISU
<400> 73
   Ala Ile Glu Arg Tyr Phe Lys Asp
   1 5
<210> 74
   <211> 8
   <212> PRT
   <213> Artificial sequence
<220>
   <223> fragement of lentiviral ENV ISU
<400> 74
   Ala Ile Glu Arg Tyr Gly Lys Asp
   1 5
<210> 75
   <211> 8
   <212> PRT
   <213> Artificial sequence
<220>
   <223> fragement of lentiviral ENV ISU
<400> 75
   Ala Ile Glu Arg Tyr Arg Lys Asp
   1 5
<210> 76
   <211> 8
   <212> PRT
   <213> Artificial sequence
<220>
   <223> fragement of lentiviral ENV ISU
<400> 76
   Ala Ile Glu Arg Ala Ala Lys Asp
   1 5
<210> 77
   <211> 8
   <212> PRT
   <213> Artificial sequence
<220>
   <223> fragement of lentiviral ENV ISU
<400> 77
   Ala Ile Glu Arg Ala Phe Lys Asp
   1 5
<210> 78
   <211> 8
   <212> PRT
   <213> Artificial sequence
<220>
   <223> fragement of lentiviral ENV ISU
<400> 78
   Ala Ile Glu Arg Ala Gly Lys Asp
   1 5
<210> 79
   <211> 8
   <212> PRT
   <213> Artificial sequence
<220>
   <223> fragement of lentiviral ENV ISU
<400> 79
   Ala Ile Glu Arg Ala Arg Lys Asp
   1 5
<210> 80
   <211> 8
   <212> PRT
   <213> Artificial sequence
<220>
   <223> fragement of lentiviral ENV ISU
<400> 80
   Ala Ile Glu Arg Phe Ala Lys Asp
   1 5
<210> 81
   <211> 8
   <212> PRT
   <213> Artificial sequence
<220>
   <223> fragement of lentiviral ENV ISU
<400> 81
   Ala Ile Glu Arg Phe Phe Lys Asp
   1 5
<210> 82
   <211> 8
   <212> PRT
   <213> Artificial sequence
<220>
   <223> fragement of lentiviral ENV ISU
<400> 82
   Ala Ile Glu Arg Phe Gly Lys Asp
   1 5
<210> 83
   <211> 8
   <212> PRT
   <213> Artificial sequence
<220>
   <223> fragement of lentiviral ENV ISU
<400> 83
   Ala Ile Glu Arg Phe Arg Lys Asp
   1 5
<210> 84
   <211> 8
   <212> PRT
   <213> Artificial sequence
<220>
   <223> fragement of lentiviral ENV ISU
<400> 84
   Ala Ile Glu Arg Gly Ala Lys Asp
   1 5
<210> 85
   <211> 8
   <212> PRT
   <213> Artificial sequence
<220>
   <223> fragement of lentiviral ENV ISU
<400> 85
   Ala Ile Glu Arg Gly Phe Lys Asp
   1 5
<210> 86
   <211> 8
   <212> PRT
   <213> Artificial sequence
<220>
   <223> fragement of lentiviral ENV ISU
<400> 86
   Ala Ile Glu Arg Gly Gly Lys Asp
   1 5
<210> 87
   <211> 8
   <212> PRT
   <213> Artificial sequence
<220>
   <223> fragement of lentiviral ENV ISU
<400> 87
   Ala Ile Glu Arg Gly Arg Lys Asp
   1 5
<210> 88
   <211> 8
   <212> PRT
   <213> Artificial sequence
<220>
   <223> fragement of lentiviral ENV ISU
<400> 88
   Ala Ile Glu Arg Leu Ala Lys Asp
   1 5
<210> 89
   <211> 8
   <212> PRT
   <213> Artificial sequence
<220>
   <223> fragement of lentiviral ENV ISU
<400> 89
   Ala Ile Glu Arg Leu Phe Lys Asp
   1 5
<210> 90
   <211> 8
   <212> PRT
   <213> Artificial sequence
<220>
   <223> fragement of lentiviral ENV ISU
<400> 90
   Ala Ile Glu Arg Leu Gly Lys Asp
   1 5
<210> 91
   <211> 8
   <212> PRT
   <213> Artificial sequence
<220>
   <223> fragement of lentiviral ENV ISU
<400> 91
   Ala Ile Glu Arg Leu Arg Lys Asp
   1 5
<210> 92
   <211> 8
   <212> PRT
   <213> Artificial sequence
<220>
   <223> fragement of lentiviral ENV ISU
<400> 92
   Ala Ile Glu Arg Arg Ala Lys Asp
   1 5
<210> 93
   <211> 8
   <212> PRT
   <213> Artificial sequence
<220>
   <223> fragement of lentiviral ENV ISU
<400> 93
   Ala Ile Glu Arg Arg Phe Lys Asp
   1               5
<210> 94
   <211> 8
   <212> PRT
   <213> Artificial sequence
<220>
   <223> fragement of lentiviral ENV ISU
<400> 94
   Ala Ile Glu Arg Arg Gly Lys Asp
   1               5
<210> 95
   <211> 8
   <212> PRT
   <213> Artificial Sequence
<220>
   <223> fragement of lentiviral ENV ISU
<400> 95
   Ala Ile Glu Arg Arg Arg Lys Asp
   1               5
<210> 96
   <211> 49
   <212> PRT
   <213> Artificial Sequence
<220>
   <223> fragment of HIV1 ENV ISU
<400> 96
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Ala Leu Lys Asp Gln Gln Leu Leu
   35 40 45
   Gly
<210> 97
   <211> 49
   <212> PRT
   <213> Artificial sequence
<220>
   <223> fragment of HIV1 ENV ISU
<400> 97
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Phe Leu Lys Asp Gln Gln Leu Leu
   35 40 45
   Gly
<210> 98
   <211> 49
   <212> PRT
   <213> Artificial sequence
<220>
   <223> fragment of HIV1 ENV ISU
<400> 98
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Gly Leu Lys Asp Gln Gln Leu Leu
           35                  40                  45
   Gly
<210> 99
   <211> 49
   <212> PRT
   <213> Artificial sequence
<220>
   <223> fragment of HIV1 ENV ISU
<400> 99
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Leu Leu Lys Asp Gln Gln Leu Leu
           35                  40                  45
   Gly
<210> 100
   <211> 49
   <212> PRT
   <213> Artificial sequence
<220>
   <223> fragment of HIV1 ENV ISU
<400> 100
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Arg Leu Lys Asp Gln Gln Leu Leu
   35 40 45
   Gly
<210> 101
   <211> 49
   <212> PRT
   <213> Artificial sequence
<220>
   <223> fragment of HIV1 ENV ISU
<400> 101
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Tyr Ala Lys Asp Gln Gln Leu Leu
   35 40 45
   Gly
<210> 102
   <211> 49
   <212> PRT
   <213> Artificial sequence
<220>
   <223> fragment of HIV1 ENV ISU
<400> 102
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Tyr Phe Lys Asp Gln Gln Leu Leu
           35                  40                  45
   Gly
<210> 103
   <211> 49
   <212> PRT
   <213> Artificial sequence
<220>
   <223> fragment of HIV1 ENV ISU
<400> 103
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Tyr Gly Lys Asp Gln Gln Leu Leu
           35                  40                  45
   Gly
<210> 104
   <211> 49
   <212> PRT
   <213> Artificial sequence
<220>
   <223> fragment of HIV1 ENV ISU
<400> 104
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Tyr Arg Lys Asp Gln Gln Leu Leu
           35                  40                  45
   Gly
<210> 105
   <211> 49
   <212> PRT
   <213> Artificial sequence
<220>
   <223> fragment of HIV1 ENV ISU
<400> 105
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Ala Ala Lys Asp Gln Gln Leu Leu
           35                  40                  45
   Gly
<210> 106
   <211> 49
   <212> PRT
   <213> Artificial sequence
<220>
   <223> fragment of HIV1 ENV ISU
<400> 106
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Ala Phe Lys Asp Gln Gln Leu Leu
           35                  40                  45
   Gly
<210> 107
   <211> 49
   <212> PRT
   <213> Artificial sequence
<220>
   <223> fragment of HIV1 ENV ISU
<400> 107
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Ala Gly Lys Asp Gln Gln Leu Leu
           35                  40                  45
   Gly
<210> 108
   <211> 49
   <212> PRT
   <213> Artificial sequence
<220>
   <223> fragment of HIV1 ENV ISU
<400> 108
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Ala Arg Lys Asp Gln Gln Leu Leu
           35                  40                  45
   Gly
<210> 109
   <211> 49
   <212> PRT
   <213> Artificial sequence
<220>
   <223> fragment of HIV1 ENV ISU
<400> 109
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Phe Ala Lys Asp Gln Gln Leu Leu
   35 40 45
   Gly
<210> 110
   <211> 49
   <212> PRT
   <213> Artificial sequence
<220>
   <223> fragment of HIV1 ENV ISU
<400> 110
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Phe Phe Lys Asp Gln Gln Leu Leu
   35 40 45
   Gly
<210> 111
   <211> 49
   <212> PRT
   <213> Artificial sequence
<220>
   <223> fragment of HIV1 ENV ISU
<400> 111
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Phe Gly Lys Asp Gln Gln Leu Leu
   35 40 45
   Gly
<210> 112
   <211> 49
   <212> PRT
   <213> Artificial sequence
<220>
   <223> fragment of HIV1 ENV ISU
<400> 112
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Phe Arg Lys Asp Gln Gln Leu Leu
   35 40 45
   Gly
<210> 113
   <211> 49
   <212> PRT
   <213> Artificial sequence
<220>
   <223> fragment of HIV1 ENV ISU
<400> 113
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Gly Ala Lys Asp Gln Gln Leu Leu
   35 40 45
   Gly
<210> 114
   <211> 49
   <212> PRT
   <213> Artificial sequence
<220>
   <223> fragment of HIV1 ENV ISU
<400> 114
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Gly Phe Lys Asp Gln Gln Leu Leu
   35 40 45
   Gly
<210> 115
   <211> 49
   <212> PRT
   <213> Artificial sequence
<220>
   <223> fragment of HIV1 ENV ISU
<400> 115
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Gly Gly Lys Asp Gln Gln Leu Leu
           35                  40                  45
   Gly
<210> 116
   <211> 49
   <212> PRT
   <213> Artificial sequence
<220>
   <223> fragment of HIV1 ENV ISU
<400> 116
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Gly Arg Lys Asp Gln Gln Leu Leu
           35                  40                  45
   Gly
<210> 117
   <211> 49
   <212> PRT
   <213> Artificial sequence
<220>
   <223> fragment of HIV1 ENV ISU
<400> 117
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Leu Ala Lys Asp Gln Gln Leu Leu
   35 40 45
   Gly
<210> 118
   <211> 49
   <212> PRT
   <213> Artificial sequence
<220>
   <223> fragment of HIV1 ENV ISU
<400> 118
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Leu Phe Lys Asp Gln Gln Leu Leu
   35 40 45
   Gly
<210> 119
   <211> 49
   <212> PRT
   <213> Artificial sequence
<220>
   <223> fragment of HIV1 ENV ISU
<400> 119
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Leu Gly Lys Asp Gln Gln Leu Leu
           35                  40                  45
   Gly
<210> 120
   <211> 49
   <212> PRT
   <213> Artificial sequence
<220>
   <223> fragment of HIV1 ENV ISU
<400> 120
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Leu Arg Lys Asp Gln Gln Leu Leu
           35                  40                  45
   Gly
<210> 121
   <211> 49
   <212> PRT
   <213> Artificial sequence
<220>
   <223> fragment of HIV1 ENV ISU
<400> 121
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Arg Ala Lys Asp Gln Gln Leu Leu
   35 40 45
   Gly
<210> 122
   <211> 49
   <212> PRT
   <213> Artificial sequence
<220>
   <223> fragment of HIV1 ENV ISU
<400> 122
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Arg Phe Lys Asp Gln Gln Leu Leu
   35 40 45
   Gly
<210> 123
   <211> 49
   <212> PRT
   <213> Artificial sequence
<220>
   <223> fragment of HIV1 ENV ISU
<400> 123
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Arg Gly Lys Asp Gln Gln Leu Leu
   35 40 45
   Gly
<210> 124
   <211> 49
   <212> PRT
   <213> Artificial sequence
<220>
   <223> fragment of HIV1 ENV ISU
<400> 124
   Ser Gly Ile Val Gln Gln Gln Ser Asn Leu Leu Arg Ala Ile Gln Ala
   1               5                   10                  15
   Arg Gln His Met Leu Gln Leu Thr Val Trp Gly Ile Lys Gln Leu Gln
               20                  25                  30
   Ala Arg Val Leu Ala Val Glu Arg Arg Arg Lys Asp Gln Gln Leu Leu
           35                  40                  45
   Gly
<210> 125
   <211> 57
   <212> PRT
   <213> Artificial sequence
<220>
   <223> fragment of HIV2 ENV ISU
<400> 125
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Ala Leu Lys Asp Gln Ala Gln Leu
           35                  40                  45
   Asn Ser Trp Gly Cys Ala Phe Arg Gln
       50                  55
<210> 126
   <211> 57
   <212> PRT
   <213> Artificial sequence
<220>
   <223> fragment of HIV2 ENV ISU
<400> 126
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Phe Leu Lys Asp Gln Ala Gln Leu
           35                  40                  45
   Asn Ser Trp Gly Cys Ala Phe Arg Gln
       50                  55
<210> 127
   <211> 57
   <212> PRT
   <213> Artificial sequence
<220>
   <223> fragment of HIV2 ENV ISU
<400> 127
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Gly Leu Lys Asp Gln Ala Gln Leu
           35                  40                  45
   Asn Ser Trp Gly Cys Ala Phe Arg Gln
       50                  55
<210> 128
   <211> 57
   <212> PRT
   <213> Artificial sequence
<220>
   <223> fragment of HIV2 ENV ISU
<400> 128
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Leu Leu Lys Asp Gln Ala Gln Leu
           35                  40                  45
   Asn Ser Trp Gly Cys Ala Phe Arg Gln
       50                  55
<210> 129
   <211> 57
   <212> PRT
   <213> Artificial sequence
<220>
   <223> fragment of HIV2 ENV ISU
<400> 129
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Arg Leu Lys Asp Gln Ala Gln Leu
           35                  40                  45
   Asn Ser Trp Gly Cys Ala Phe Arg Gln
       50                  55
<210> 130
   <211> 57
   <212> PRT
   <213> Artificial sequence
<220>
   <223> fragment of HIV2 ENV ISU
<400> 130
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Tyr Ala Lys Asp Gln Ala Gln Leu
   35 40 45
   Asn Ser Trp Gly Cys Ala Phe Arg Gln
   50 55
<210> 131
   <211> 57
   <212> PRT
   <213> Artificial sequence
<220>
   <223> fragment of HIV2 ENV ISU
<400> 131
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Tyr Phe Lys Asp Gln Ala Gln Leu
   35 40 45
   Asn Ser Trp Gly Cys Ala Phe Arg Gln
   50 55
<210> 132
   <211> 57
   <212> PRT
   <213> Artificial sequence
<220>
   <223> fragment of HIV2 ENV ISU
<400> 132
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Tyr Gly Lys Asp Gln Ala Gln Leu
   35 40 45
   Asn Ser Trp Gly Cys Ala Phe Arg Gln
   50 55
<210> 133
   <211> 57
   <212> PRT
   <213> Artificial sequence
<220>
   <223> fragment of HIV2 ENV ISU
<400> 133
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Tyr Arg Lys Asp Gln Ala Gln Leu
   35 40 45
   Asn Ser Trp Gly Cys Ala Phe Arg Gln
   50 55
<210> 134
   <211> 57
   <212> PRT
   <213> Artificial sequence
<220>
   <223> fragment of HIV2 ENV ISU
<400> 134
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Ala Ala Lys Asp Gln Ala Gln Leu
           35                  40                  45
   Asn Ser Trp Gly Cys Ala Phe Arg Gln
       50                  55
<210> 135
   <211> 57
   <212> PRT
   <213> Artificial sequence
<220>
   <223> fragment of HIV2 ENV ISU
<400> 135
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Ala Phe Lys Asp Gln Ala Gln Leu
           35                  40                  45
   Asn Ser Trp Gly Cys Ala Phe Arg Gln
       50                  55
<210> 136
   <211> 57
   <212> PRT
   <213> Artificial sequence
<220>
   <223> fragment of HIV2 ENV ISU
<400> 136
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Ala Gly Lys Asp Gln Ala Gln Leu
   35 40 45
   Asn Ser Trp Gly Cys Ala Phe Arg Gln
   50 55
<210> 137
   <211> 57
   <212> PRT
   <213> Artificial sequence
<220>
   <223> fragment of HIV2 ENV ISU
<400> 137
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Ala Arg Lys Asp Gln Ala Gln Leu
   35 40 45
   Asn Ser Trp Gly Cys Ala Phe Arg Gln
   50 55
<210> 138
   <211> 57
   <212> PRT
   <213> Artificial sequence
<220>
   <223> fragment of HIV2 ENV ISU
<400> 138
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Phe Ala Lys Asp Gln Ala Gln Leu
   35 40 45
   Asn Ser Trp Gly Cys Ala Phe Arg Gln
   50 55
<210> 139
   <211> 57
   <212> PRT
   <213> Artificial sequence
<220>
   <223> fragment of HIV2 ENV ISU
<400> 139
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Phe Phe Lys Asp Gln Ala Gln Leu
   35 40 45
   Asn Ser Trp Gly Cys Ala Phe Arg Gln
   50 55
<210> 140
   <211> 57
   <212> PRT
   <213> Artificial sequence
<220>
   <223> fragment of HIV2 ENV ISU
<400> 140
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Phe Gly Lys Asp Gln Ala Gln Leu
   35 40 45
   Asn Ser Trp Gly Cys Ala Phe Arg Gln
   50 55
<210> 141
   <211> 57
   <212> PRT
   <213> Artificial sequence
<220>
   <223> fragment of HIV2 ENV ISU
<400> 141
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Phe Arg Lys Asp Gln Ala Gln Leu
   35 40 45
   Asn Ser Trp Gly Cys Ala Phe Arg Gln
   50 55
<210> 142
   <211> 57
   <212> PRT
   <213> Artificial sequence
<220>
   <223> fragment of HIV2 ENV ISU
<400> 142
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Gly Ala Lys Asp Gln Ala Gln Leu
   35 40 45
   Asn Ser Trp Gly Cys Ala Phe Arg Gln
   50 55
<210> 143
   <211> 57
   <212> PRT
   <213> Artificial sequence
<220>
   <223> fragment of HIV2 ENV ISU
<400> 143
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Gly Phe Lys Asp Gln Ala Gln Leu
           35                  40                  45
   Asn Ser Trp Gly Cys Ala Phe Arg Gln
       50                  55
<210> 144
   <211> 57
   <212> PRT
   <213> Artificial sequence
<220>
   <223> fragment of HIV2 ENV ISU
<400> 144
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Gly Gly Lys Asp Gln Ala Gln Leu
           35                  40                  45
   Asn Ser Trp Gly Cys Ala Phe Arg Gln
       50                  55
<210> 145
   <211> 57
   <212> PRT
   <213> Artificial sequence
<220>
   <223> fragment of HIV2 ENV ISU
<400> 145
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Gly Arg Lys Asp Gln Ala Gln Leu
   35 40 45
   Asn Ser Trp Gly Cys Ala Phe Arg Gln
   50 55
<210> 146
   <211> 57
   <212> PRT
   <213> Artificial sequence
<220>
   <223> fragment of HIV2 ENV ISU
<400> 146
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Leu Ala Lys Asp Gln Ala Gln Leu
   35 40 45
   Asn Ser Trp Gly Cys Ala Phe Arg Gln
   50 55
<210> 147
   <211> 57
   <212> PRT
   <213> Artificial sequence
<220>
   <223> fragment of HIV2 ENV ISU
<400> 147
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Leu Phe Lys Asp Gln Ala Gln Leu
   35 40 45
   Asn Ser Trp Gly Cys Ala Phe Arg Gln
   50 55
<210> 148
   <211> 57
   <212> PRT
   <213> Artificial sequence
<220>
   <223> fragment of HIV2 ENV ISU
<400> 148
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Leu Gly Lys Asp Gln Ala Gln Leu
   35 40 45
   Asn Ser Trp Gly Cys Ala Phe Arg Gln
   50 55
<210> 149
   <211> 57
   <212> PRT
   <213> Artificial sequence
<220>
   <223> fragment of HIV2 ENV ISU
<400> 149
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Leu Arg Lys Asp Gln Ala Gln Leu
           35                  40                  45
   Asn Ser Trp Gly Cys Ala Phe Arg Gln
       50                  55
<210> 150
   <211> 57
   <212> PRT
   <213> Artificial sequence
<220>
   <223> fragment of HIV2 ENV ISU
<400> 150
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Arg Ala Lys Asp Gln Ala Gln Leu
           35                  40                  45
   Asn Ser Trp Gly Cys Ala Phe Arg Gln
       50                  55
<210> 151
   <211> 57
   <212> PRT
   <213> Artificial sequence
<220>
   <223> fragment of HIV2 ENV ISU
<400> 151
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Arg Phe Lys Asp Gln Ala Gln Leu
   35 40 45
   Asn Ser Trp Gly Cys Ala Phe Arg Gln
   50 55
<210> 152
   <211> 57
   <212> PRT
   <213> Artificial sequence
<220>
   <223> fragment of HIV2 ENV ISU
<400> 152
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Arg Gly Lys Asp Gln Ala Gln Leu
   35 40 45
   Asn Ser Trp Gly Cys Ala Phe Arg Gln
   50 55
<210> 153
   <211> 57
   <212> PRT
   <213> Artificial Sequence
<220>
   <223> fragment of HIV2 ENV ISU
<400> 153
   Ala Gly Ile Val Gln Gln Gln Gln Gln Leu Leu Asp Val Val Lys Arg
   1 5 10 15
   Gln Gln Glu Met Leu Arg Leu Thr Val Trp Gly Thr Lys Asn Leu Gln
   20 25 30
   Ala Arg Val Thr Ala Ile Glu Lys Arg Arg Lys Asp Gln Ala Gln Leu
   35 40 45
   Asn Ser Trp Gly Cys Ala Phe Arg Gln
   50 55
<210> 154
   <211> 57
   <212> PRT
   <213> Artificial sequence
<220>
   <223> fragment of HIV2 ENV ISU
<400> 154
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Ala Leu Gln Asp Gln Ala Arg Leu
   35 40 45
   Asn Ser Trp Gly Cys Ala Phe Arg Gln
   50 55
<210> 155
   <211> 57
   <212> PRT
   <213> Artificial sequence
<220>
   <223> fragment of HIV2 ENV ISU
<400> 155
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Phe Leu Gln Asp Gln Ala Arg Leu
   35 40 45
   Asn Ser Trp Gly Cys Ala Phe Arg Gln
   50 55
<210> 156
   <211> 57
   <212> PRT
   <213> Artificial sequence
<220>
   <223> fragment of HIV2 ENV ISU
<400> 156
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Gly Leu Gln Asp Gln Ala Arg Leu
   35 40 45
   Asn Ser Trp Gly Cys Ala Phe Arg Gln
   50 55
<210> 157
   <211> 57
   <212> PRT
   <213> Artificial sequence
<220>
   <223> fragment of HIV2 ENV ISU
<400> 157
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Leu Leu Gln Asp Gln Ala Arg Leu
   35 40 45
   Asn Ser Trp Gly Cys Ala Phe Arg Gln
   50 55
<210> 158
   <211> 57
   <212> PRT
   <213> Artificial sequence
<220>
   <223> fragment of HIV2 ENV ISU
<400> 158
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Arg Leu Gln Asp Gln Ala Arg Leu
           35                  40                  45
   Asn Ser Trp Gly Cys Ala Phe Arg Gln
       50                  55
<210> 159
   <211> 57
   <212> PRT
   <213> Artificial sequence
<220>
   <223> fragment of HIV2 ENV ISU
<400> 159
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Tyr Ala Gln Asp Gln Ala Arg Leu
           35                  40                  45
   Asn Ser Trp Gly Cys Ala Phe Arg Gln
       50                  55
<210> 160
   <211> 57
   <212> PRT
   <213> Artificial sequence
<220>
   <223> fragment of HIV2 ENV ISU
<400> 160
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Tyr Phe Gln Asp Gln Ala Arg Leu
   35 40 45
   Asn Ser Trp Gly Cys Ala Phe Arg Gln
   50 55
<210> 161
   <211> 57
   <212> PRT
   <213> Artificial sequence
<220>
   <223> fragment of HIV2 ENV ISU
<400> 161
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Tyr Gly Gln Asp Gln Ala Arg Leu
   35 40 45
   Asn Ser Trp Gly Cys Ala Phe Arg Gln
   50 55
<210> 162
   <211> 57
   <212> PRT
   <213> Artificial sequence
<220>
   <223> fragment of HIV2 ENV ISU
<400> 162
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Tyr Arg Gln Asp Gln Ala Arg Leu
   35 40 45
   Asn Ser Trp Gly Cys Ala Phe Arg Gln
   50 55
<210> 163
   <211> 57
   <212> PRT
   <213> Artificial sequence
<220>
   <223> fragment of HIV2 ENV ISU
<400> 163
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Ala Ala Gln Asp Gln Ala Arg Leu
   35 40 45
   Asn Ser Trp Gly Cys Ala Phe Arg Gln
   50 55
<210> 164
   <211> 57
   <212> PRT
   <213> Artificial sequence
<220>
   <223> fragment of HIV2 ENV ISU
<400> 164
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Ala Phe Gln Asp Gln Ala Arg Leu
   35 40 45
   Asn Ser Trp Gly Cys Ala Phe Arg Gln
   50 55
<210> 165
   <211> 57
   <212> PRT
   <213> Artificial sequence
<220>
   <223> fragment of HIV2 ENV ISU
<400> 165
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Ala Gly Gln Asp Gln Ala Arg Leu
   35 40 45
   Asn Ser Trp Gly Cys Ala Phe Arg Gln
   50 55
<210> 166
   <211> 57
   <212> PRT
   <213> Artificial sequence
<220>
   <223> fragment of HIV2 ENV ISU
<400> 166
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Ala Arg Gln Asp Gln Ala Arg Leu
   35 40 45
   Asn Ser Trp Gly Cys Ala Phe Arg Gln
   50 55
<210> 167
   <211> 57
   <212> PRT
   <213> Artificial sequence
<220>
   <223> fragment of HIV2 ENV ISU
<400> 167
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Phe Ala Gln Asp Gln Ala Arg Leu
   35 40 45
   Asn Ser Trp Gly Cys Ala Phe Arg Gln
   50 55
<210> 168
   <211> 57
   <212> PRT
   <213> Artificial sequence
<220>
   <223> fragment of HIV2 ENV ISU
<400> 168
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Phe Phe Gln Asp Gln Ala Arg Leu
   35 40 45
   Asn Ser Trp Gly Cys Ala Phe Arg Gln
   50 55
<210> 169
   <211> 57
   <212> PRT
   <213> Artificial sequence
<220>
   <223> fragment of HIV2 ENV ISU
<400> 169
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Phe Gly Gln Asp Gln Ala Arg Leu
   35 40 45
   Asn Ser Trp Gly Cys Ala Phe Arg Gln
   50 55
<210> 170
   <211> 57
   <212> PRT
   <213> Artificial sequence
<220>
   <223> fragment of HIV2 ENV ISU
<400> 170
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Phe Arg Gln Asp Gln Ala Arg Leu
   35 40 45
   Asn Ser Trp Gly Cys Ala Phe Arg Gln
   50 55
<210> 171
   <211> 57
   <212> PRT
   <213> Artificial sequence
<220>
   <223> fragment of HIV2 ENV ISU
<400> 171
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Gly Ala Gln Asp Gln Ala Arg Leu
   35 40 45
   Asn Ser Trp Gly Cys Ala Phe Arg Gln
   50 55
<210> 172
   <211> 57
   <212> PRT
   <213> Artificial sequence
<220>
   <223> fragment of HIV2 ENV ISU
<400> 172
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Gly Phe Gln Asp Gln Ala Arg Leu
   35 40 45
   Asn Ser Trp Gly Cys Ala Phe Arg Gln
   50 55
<210> 173
   <211> 57
   <212> PRT
   <213> Artificial sequence
<220>
   <223> fragment of HIV2 ENV ISU
<400> 173
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Gly Gly Gln Asp Gln Ala Arg Leu
   35 40 45
   Asn Ser Trp Gly Cys Ala Phe Arg Gln
   50 55
<210> 174
   <211> 57
   <212> PRT
   <213> Artificial sequence
<220>
   <223> fragment of HIV2 ENV ISU
<400> 174
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Gly Arg Gln Asp Gln Ala Arg Leu
   35 40 45
   Asn Ser Trp Gly Cys Ala Phe Arg Gln
   50 55
<210> 175
   <211> 57
   <212> PRT
   <213> Artificial sequence
<220>
   <223> fragment of HIV2 ENV ISU
<400> 175
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Leu Ala Gln Asp Gln Ala Arg Leu
           35                  40                  45
   Asn Ser Trp Gly Cys Ala Phe Arg Gln
       50                  55
<210> 176
   <211> 57
   <212> PRT
   <213> Artificial sequence
<220>
   <223> fragment of HIV2 ENV ISU
<400> 176
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Leu Phe Gln Asp Gln Ala Arg Leu
           35                  40                  45
   Asn Ser Trp Gly Cys Ala Phe Arg Gln
       50                  55
<210> 177
   <211> 57
   <212> PRT
   <213> Artificial sequence
<220>
   <223> fragment of HIV2 ENV ISU
<400> 177
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Leu Gly Gln Asp Gln Ala Arg Leu
   35 40 45
   Asn Ser Trp Gly Cys Ala Phe Arg Gln
   50 55
<210> 178
   <211> 57
   <212> PRT
   <213> Artificial sequence
<220>
   <223> fragment of HIV2 ENV ISU
<400> 178
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Ala Arg Val Thr Ala Ile Glu Lys Leu Arg Gln Asp Gln Ala Arg Leu
   35 40 45
   Asn Ser Trp Gly Cys Ala Phe Arg Gln
   50 55
<210> 179
   <211> 57
   <212> PRT
   <213> Artificial sequence
<220>
   <223> fragment of HIV2 ENV ISU
<400> 179
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Arg Ala Gln Asp Gln Ala Arg Leu
           35                  40                  45
   Asn Ser Trp Gly Cys Ala Phe Arg Gln
   50                  55
<210> 180
   <211> 57
   <212> PRT
   <213> Artificial sequence
<220>
   <223> fragment of HIV2 ENV ISU
<400> 180
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Arg Phe Gln Asp Gln Ala Arg Leu
           35                  40                  45
   Asn Ser Trp Gly Cys Ala Phe Arg Gln
       50                  55
<210> 181
   <211> 57
   <212> PRT
   <213> Artificial sequence
<220>
   <223> fragment of HIV2 ENV ISU
<400> 181
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Arg Gly Gln Asp Gln Ala Arg Leu
           35                  40                  45
   Asn Ser Trp Gly Cys Ala Phe Arg Gln
       50                  55
<210> 182
   <211> 57
   <212> PRT
   <213> Artificial Sequence
<220>
   <223> fragment of HIV2 ENV ISU
<400> 182
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Ala Arg Val Thr Ala Ile Glu Lys Arg Arg Gln Asp Gln Ala Arg Leu
           35                  40                  45
   Asn Ser Trp Gly Cys Ala Phe Arg Gln
       50                  55
<210> 183
   <211> 57
   <212> PRT
   <213> Artificial sequence
<220>
   <223> fragment of SIV ENV ISU
<400> 183
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Ala Leu Lys Asp Gln Ala Gln Leu
   35 40 45
   Asn Ala Trp Gly Cys Ala Phe Arg Gln
   50 55
<210> 184
   <211> 57
   <212> PRT
   <213> Artificial sequence
<220>
   <223> fragment of SIV ENV ISU
<400> 184
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Phe Leu Lys Asp Gln Ala Gln Leu
   35 40 45
   Asn Ala Trp Gly Cys Ala Phe Arg Gln
   50 55
<210> 185
   <211> 57
   <212> PRT
   <213> Artificial sequence
<220>
   <223> fragment of SIV ENV ISU
<400> 185
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Gly Leu Lys Asp Gln Ala Gln Leu
   35 40 45
   Asn Ala Trp Gly Cys Ala Phe Arg Gln
   50 55
<210> 186
   <211> 57
   <212> PRT
   <213> Artificial sequence
<220>
   <223> fragment of SIV ENV ISU
<400> 186
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Leu Leu Lys Asp Gln Ala Gln Leu
   35 40 45
   Asn Ala Trp Gly Cys Ala Phe Arg Gln
   50 55
<210> 187
   <211> 57
   <212> PRT
   <213> Artificial sequence
<220>
   <223> fragment of SIV ENV ISU
<400> 187
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Arg Leu Lys Asp Gln Ala Gln Leu
           35                  40                  45
   Asn Ala Trp Gly Cys Ala Phe Arg Gln
       50                  55
<210> 188
   <211> 57
   <212> PRT
   <213> Artificial sequence
<220>
   <223> fragment of SIV ENV ISU
<400> 188
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Tyr Ala Lys Asp Gln Ala Gln Leu
           35                  40                  45
   Asn Ala Trp Gly Cys Ala Phe Arg Gln
       50                  55
<210> 189
   <211> 57
   <212> PRT
   <213> Artificial sequence
<220>
   <223> fragment of SIV ENV ISU
<400> 189
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
                   20                  25                  30
   Thr Arg Val Thr Ala Ile Glu Lys Tyr Phe Lys Asp Gln Ala Gln Leu
           35                  40                  45
   Asn Ala Trp Gly Cys Ala Phe Arg Gln
       50                  55
<210> 190
   <211> 57
   <212> PRT
   <213> Artificial sequence
<220>
   <223> fragment of SIV ENV ISU
<400> 190
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Tyr Gly Lys Asp Gln Ala Gln Leu
           35                  40                  45
   Asn Ala Trp Gly Cys Ala Phe Arg Gln
       50                  55
<210> 191
   <211> 57
   <212> PRT
   <213> Artificial sequence
<220>
   <223> fragment of SIV ENV ISU
<400> 191
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Tyr Arg Lys Asp Gln Ala Gln Leu
   35 40 45
   Asn Ala Trp Gly Cys Ala Phe Arg Gln
   50 55
<210> 192
   <211> 57
   <212> PRT
   <213> Artificial sequence
<220>
   <223> fragment of SIV ENV ISU
<400> 192
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Ala Ala Lys Asp Gln Ala Gln Leu
   35 40 45
   Asn Ala Trp Gly Cys Ala Phe Arg Gln
   50 55
<210> 193
   <211> 57
   <212> PRT
   <213> Artificial sequence
<220>
   <223> fragment of SIV ENV ISU
<400> 193
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Ala Phe Lys Asp Gln Ala Gln Leu
           35                  40                  45
   Asn Ala Trp Gly Cys Ala Phe Arg Gln
       50                  55
<210> 194
   <211> 57
   <212> PRT
   <213> Artificial sequence
<220>
   <223> fragment of SIV ENV ISU
<400> 194
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Ala Gly Lys Asp Gln Ala Gln Leu
           35                  40                  45
   Asn Ala Trp Gly Cys Ala Phe Arg Gln
       50                  55
<210> 195
   <211> 57
   <212> PRT
   <213> Artificial sequence
<220>
   <223> fragment of SIV ENV ISU
<400> 195
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Ala Arg Lys Asp Gln Ala Gln Leu
           35                  40                  45
   Asn Ala Trp Gly Cys Ala Phe Arg Gln
       50                  55
<210> 196
   <211> 57
   <212> PRT
   <213> Artificial sequence
<220>
   <223> fragment of SIV ENV ISU
<400> 196
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Phe Ala Lys Asp Gln Ala Gln Leu
           35                  40                  45
   Asn Ala Trp Gly Cys Ala Phe Arg Gln
       50                  55
<210> 197
   <211> 57
   <212> PRT
   <213> Artificial sequence
<220>
   <223> fragment of SIV ENV ISU
<400> 197
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Phe Phe Lys Asp Gln Ala Gln Leu
           35                  40                  45
   Asn Ala Trp Gly Cys Ala Phe Arg Gln
       50                  55
<210> 198
   <211> 57
   <212> PRT
   <213> Artificial sequence
<220>
   <223> fragment of SIV ENV ISU
<400> 198
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Phe Gly Lys Asp Gln Ala Gln Leu
           35                  40                  45
   Asn Ala Trp Gly Cys Ala Phe Arg Gln
       50                  55
<210> 199
   <211> 57
   <212> PRT
   <213> Artificial sequence
<220>
   <223> fragment of SIV ENV ISU
<400> 199
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Phe Arg Lys Asp Gln Ala Gln Leu
           35                  40                  45
   Asn Ala Trp Gly Cys Ala Phe Arg Gln
       50                  55
<210> 200
   <211> 57
   <212> PRT
   <213> Artificial sequence
<220>
   <223> fragment of SIV ENV ISU
<400> 200
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Gly Ala Lys Asp Gln Ala Gln Leu
           35                  40                  45
   Asn Ala Trp Gly Cys Ala Phe Arg Gln
       50                  55
<210> 201
   <211> 57
   <212> PRT
   <213> Artificial sequence
<220>
   <223> fragment of SIV ENV ISU
<400> 201
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Gly Phe Lys Asp Gln Ala Gln Leu
   35 40 45
   Asn Ala Trp Gly Cys Ala Phe Arg Gln
   50 55
<210> 202
   <211> 57
   <212> PRT
   <213> Artificial sequence
<220>
   <223> fragment of SIV ENV ISU
<400> 202
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Gly Gly Lys Asp Gln Ala Gln Leu
   35 40 45
   Asn Ala Trp Gly Cys Ala Phe Arg Gln
   50 55
<210> 203
   <211> 57
   <212> PRT
   <213> Artificial sequence
<220>
   <223> fragment of SIV ENV ISU
<400> 203
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Gly Arg Lys Asp Gln Ala Gln Leu
   35 40 45
   Asn Ala Trp Gly Cys Ala Phe Arg Gln
   50 55
<210> 204
   <211> 57
   <212> PRT
   <213> Artificial sequence
<220>
   <223> fragment of SIV ENV ISU
<400> 204
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Leu Ala Lys Asp Gln Ala Gln Leu
           35                  40                  45
   Asn Ala Trp Gly Cys Ala Phe Arg Gln
       50                  55
<210> 205
   <211> 57
   <212> PRT
   <213> Artificial sequence
<220>
   <223> fragment of SIV ENV ISU
<400> 205
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Leu Phe Lys Asp Gln Ala Gln Leu
           35                  40                  45
   Asn Ala Trp Gly Cys Ala Phe Arg Gln
       50                  55
<210> 206
   <211> 57
   <212> PRT
   <213> Artificial sequence
<220>
   <223> fragment of SIV ENV ISU
<400> 206
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Leu Gly Lys Asp Gln Ala Gln Leu
           35                  40                  45
   Asn Ala Trp Gly Cys Ala Phe Arg Gln
       50                  55
<210> 207
   <211> 57
   <212> PRT
   <213> Artificial sequence
<220>
   <223> fragment of SIV ENV ISU
<400> 207
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Leu Arg Lys Asp Gln Ala Gln Leu
           35                  40                  45
   Asn Ala Trp Gly Cys Ala Phe Arg Gln
       50                  55
<210> 208
   <211> 57
   <212> PRT
   <213> Artificial sequence
<220>
   <223> fragment of SIV ENV ISU
<400> 208
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Arg Ala Lys Asp Gln Ala Gln Leu
           35                  40                  45
   Asn Ala Trp Gly Cys Ala Phe Arg Gln
       50                  55
<210> 209
   <211> 57
   <212> PRT
   <213> Artificial sequence
<220>
   <223> fragment of SIV ENV ISU
<400> 209
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Arg Phe Lys Asp Gln Ala Gln Leu
           35                  40                  45
   Asn Ala Trp Gly Cys Ala Phe Arg Gln
       50                  55
<210> 210
   <211> 57
   <212> PRT
   <213> Artificial sequence
<220>
   <223> fragment of SIV ENV ISU
<400> 210
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Arg Gly Lys Asp Gln Ala Gln Leu
   35 40 45
   Asn Ala Trp Gly Cys Ala Phe Arg Gln
   50 55
<210> 211
   <211> 57
   <212> PRT
   <213> Artificial Sequence
<220>
   <223> fragment of SIV ENV ISU
<400> 211
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Thr Ala Ile Glu Lys Arg Arg Lys Asp Gln Ala Gln Leu
   35 40 45
   Asn Ala Trp Gly Cys Ala Phe Arg Gln
   50 55
<210> 212
   <211> 861
   <212> PRT
   <213> Human immunodeficiency virus type 1
<400> 212
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Ala Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 213
   <211> 861
   <212> PRT
   <213> Human immunodeficiency virus type 1
<400> 213
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Phe Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 214
   <211> 861
   <212> PRT
   <213> Human immunodeficiency virus type 1
<400> 214
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Gly Leu
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 215
   <211> 861
   <212> PRT
   <213> Human immunodeficiency virus type 1
<400> 215
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Leu Leu
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 216
   <211> 861
   <212> PRT
   <213> Human immunodeficiency virus type 1
<400> 216
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 217
   <211> 861
   <212> PRT
   <213> Human immunodeficiency virus type 1
<400> 217
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Tyr Ala
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 218
   <211> 861
   <212> PRT
   <213> Human immunodeficiency virus type 1
<400> 218
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Tyr Phe
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 219
   <211> 861
   <212> PRT
   <213> Human immunodeficiency virus type 1
<400> 219
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Tyr Gly
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 220
   <211> 861
   <212> PRT
   <213> Human immunodeficiency virus type 1
<400> 220
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Tyr Arg
               580                 585                 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 221
   <211> 861
   <212> PRT
   <213> Human immunodeficiency virus type 1
<400> 221
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Ala Ala
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 222
   <211> 861
   <212> PRT
   <213> Human immunodeficiency virus type 1
<400> 222
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Ala Phe
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 223
   <211> 861
   <212> PRT
   <213> Human immunodeficiency virus type 1
<400> 223
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Ala Gly
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 224
   <211> 861
   <212> PRT
   <213> Human immunodeficiency virus type 1
<400> 224
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Ala Arg
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 225
   <211> 861
   <212> PRT
   <213> Human immunodeficiency virus type 1
<400> 225
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Phe Ala
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 226
   <211> 861
   <212> PRT
   <213> Human immunodeficiency virus type 1
<400> 226
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Phe Phe
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 227
   <211> 861
   <212> PRT
   <213> Human immunodeficiency virus type 1
<400> 227
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Phe Gly
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 228
   <211> 861
   <212> PRT
   <213> Human immunodeficiency virus type 1
<400> 228
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Phe Arg
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 229
   <211> 861
   <212> PRT
   <213> Human immunodeficiency virus type 1
<400> 229
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Gly Ala
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 230
   <211> 861
   <212> PRT
   <213> Human immunodeficiency virus type 1
<400> 230
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Gly Phe
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 231
   <211> 861
   <212> PRT
   <213> Human immunodeficiency virus type 1
<400> 231
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Gly Gly
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 232
   <211> 861
   <212> PRT
   <213> Human immunodeficiency virus type 1
<400> 232
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Gly Arg
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 233
   <211> 861
   <212> PRT
   <213> Human immunodeficiency virus type 1
<400> 233
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Leu Ala
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 234
   <211> 861
   <212> PRT
   <213> Human immunodeficiency virus type 1
<400> 234
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Leu Phe
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 235
   <211> 861
   <212> PRT
   <213> Human immunodeficiency virus type 1
<400> 235
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Leu Gly
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 236
   <211> 861
   <212> PRT
   <213> Human immunodeficiency virus type 1
<400> 236
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Leu Arg
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
                   675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 237
   <211> 861
   <212> PRT
   <213> Human immunodeficiency virus type 1
<400> 237
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Arg Ala
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 238
   <211> 861
   <212> PRT
   <213> Human immunodeficiency virus type 1
<400> 238
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Phe
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 239
   <211> 861
   <212> PRT
   <213> Human immunodeficiency virus type 1
<400> 239
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Gly
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 240
   <211> 861
   <212> PRT
   <213> Human immunodeficiency virus type 1
<400> 240
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Arg Arg
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 241
   <211> 856
   <212> PRT
   <213> Human immunodeficiency virus type 1
<400> 241
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Ala Leu Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 242
   <211> 856
   <212> PRT
   <213> Human immunodeficiency virus type 1
<400> 242
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Phe Leu Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 243
   <211> 856
   <212> PRT
   <213> Human immunodeficiency virus type 1
<400> 243
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Gly Leu Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 244
   <211> 856
   <212> PRT
   <213> Human immunodeficiency virus type 1
<400> 244
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Leu Leu Lys Asp Gln Gln Leu
   580 585 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
   850 855
<210> 245
   <211> 856
   <212> PRT
   <213> Human immunodeficiency virus type 1
<400> 245
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Arg Leu Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 246
   <211> 856
   <212> PRT
   <213> Human immunodeficiency virus type 1
<400> 246
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Tyr Ala Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 247
   <211> 856
   <212> PRT
   <213> Human immunodeficiency virus type 1
<400> 247
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Tyr Phe Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
               690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 248
   <211> 856
   <212> PRT
   <213> Human immunodeficiency virus type 1
<400> 248
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Tyr Gly Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 249
   <211> 856
   <212> PRT
   <213> Human immunodeficiency virus type 1
<400> 249
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Tyr Arg Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
               675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 250
   <211> 856
   <212> PRT
   <213> Human immunodeficiency virus type 1
<400> 250
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Ala Ala Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 251
   <211> 856
   <212> PRT
   <213> Human immunodeficiency virus type 1
<400> 251
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Ala Phe Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 252
   <211> 856
   <212> PRT
   <213> Human immunodeficiency virus type 1
<400> 252
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
               515                 520                 525
   Thr Met Gly Ala Ala Ser Met Thr Leu Thr Val Gln Ala Arg Gln Leu
           530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Ala Gly Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 253
   <211> 856
   <212> PRT
   <213> Human immunodeficiency virus type 1
<400> 253
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Ala Arg Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 254
   <211> 856
   <212> PRT
   <213> Human immunodeficiency virus type 1
<400> 254
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Phe Ala Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 255
   <211> 856
   <212> PRT
   <213> Human immunodeficiency virus type 1
<400> 255
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Phe Phe Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 256
   <211> 856
   <212> PRT
   <213> Human immunodeficiency virus type 1
<400> 256
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Phe Gly Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 257
   <211> 856
   <212> PRT
   <213> Human immunodeficiency virus type 1
<400> 257
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Phe Arg Lys Asp Gln Gln Leu
   580 585 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
   850 855
<210> 258
   <211> 856
   <212> PRT
   <213> Human immunodeficiency virus type 1
<400> 258
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Gly Ala Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 259
   <211> 856
   <212> PRT
   <213> Human immunodeficiency virus type 1
<400> 259
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Gly Phe Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 260
   <211> 856
   <212> PRT
   <213> Human immunodeficiency virus type 1
<400> 260
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Gly Gly Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 261
   <211> 856
   <212> PRT
   <213> Human immunodeficiency virus type 1
<400> 261
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Gly Arg Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 262
   <211> 856
   <212> PRT
   <213> Human immunodeficiency virus type 1
<400> 262
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Leu Ala Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 263
   <211> 856
   <212> PRT
   <213> Human immunodeficiency virus type 1
<400> 263
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
           530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Leu Phe Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 264
   <211> 856
   <212> PRT
   <213> Human immunodeficiency virus type 1
<400> 264
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
                   485                 490                 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Leu Gly Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 265
   <211> 856
   <212> PRT
   <213> Human immunodeficiency virus type 1
<400> 265
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Leu Arg Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 266
   <211> 856
   <212> PRT
   <213> Human immunodeficiency virus type 1
<400> 266
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Arg Ala Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 267
   <211> 856
   <212> PRT
   <213> Human immunodeficiency virus type 1
<400> 267
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
               500                 505                 510
   Val Gly Ile Gly Ala Leu Phe Leu Gly Phe Leu Gly Ala Ala Gly Ser
           515                 520                 525
   Thr Met Gly Ala Ala Ser Met Thr Leu Thr Val Gln Ala Arg Gln Leu
       530                 535                 540
   Leu Ser Gly Ile Val Gln Gln Gln Asn Asn Leu Leu Arg Ala Ile Glu
   545                 550                 555                 560
   Ala Gln Gln His Leu Leu Gln Leu Thr Val Trp Gly Ile Lys Gln Leu
                   565                 570                 575
   Gln Ala Arg Ile Leu Ala Val Glu Arg Arg Phe Lys Asp Gln Gln Leu
               580                 585                 590
   Leu Gly Ile Trp Gly Cys Ser Gly Lys Leu Ile Cys Thr Thr Ala Val
           595                 600                 605
   Pro Trp Asn Ala Ser Trp Ser Asn Lys Ser Leu Glu Gln Ile Trp Asn
       610                 615                 620
   Asn Met Thr Trp Met Glu Trp Asp Arg Glu Ile Asn Asn Tyr Thr Ser
   625                 630                 635                 640
   Leu Ile His Ser Leu Ile Glu Glu Ser Gln Asn Gln Gln Glu Lys Asn
                   645                 650                 655
   Glu Gln Glu Leu Leu Glu Leu Asp Lys Trp Ala Ser Leu Trp Asn Trp
               660                 665                 670
   Phe Asn Ile Thr Asn Trp Leu Trp Tyr Ile Lys Leu Phe Ile Met Ile
           675                 680                 685
   Val Gly Gly Leu Val Gly Leu Arg Ile Val Phe Ala Val Leu Ser Val
       690                 695                 700
   Val Asn Arg Val Arg Gln Gly Tyr Ser Pro Leu Ser Phe Gln Thr His
   705                 710                 715                 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 268
   <211> 856
   <212> PRT
   <213> Human immunodeficiency virus type 1
<400> 268
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Thr
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Ala Asn Phe Thr Asp Asn Ala Lys Thr Ile Ile Val Gln Leu
   275 280 285
   Asn Gln Ser Val Glu Ile Asn Cys Thr Arg Pro Asn Asn Asn Thr Arg
   290 295 300
   Lys Ser Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Asp Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Lys Gly Ser Asn Asn Thr Glu Gly Ser Asp Thr Ile Thr Leu
   405 410 415
   Pro Cys Arg Ile Lys Gln Ile Ile Asn Met Trp Gln Glu Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Arg Gly Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Tyr Arg Ala Ile Arg His Ile Pro Arg Arg Ile Arg
   835 840 845
   Gln Gly Leu Glu Arg Ile Leu Leu
   850 855
<210> 269
   <211> 856
   <212> PRT
   <213> Human immunodeficiency virus type 1
<400> 269
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Thr
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
       210                 215                 220
   Ile Leu Lys Cys Asn Asn Lys Thr Phe Asn Gly Thr Gly Pro Cys Thr
   225                 230                 235                 240
   Asn Val Ser Thr Val Gln Cys Thr His Gly Ile Arg Pro Val Val Ser
                   245                 250                 255
   Thr Gln Leu Leu Leu Asn Gly Ser Leu Ala Glu Glu Glu Val Val Ile
               260                 265                 270
   Arg Ser Ala Asn Phe Thr Asp Asn Ala Lys Thr Ile Ile Val Gln Leu
           275                 280                 285
   Asn Gln Ser Val Glu Ile Asn Cys Thr Arg Pro Asn Asn Asn Thr Arg
       290                 295                 300
   Lys Ser Ile Arg Ile Gln Arg Gly Pro Gly Arg Ala Phe Val Thr Ile
   305                 310                 315                 320
   Gly Lys Ile Gly Asn Met Arg Gln Ala His Cys Asn Ile Ser Arg Ala
                   325                 330                 335
   Lys Trp Asn Asn Thr Leu Lys Gln Ile Asp Ser Lys Leu Arg Glu Gln
               340                 345                 350
   Phe Gly Asn Asn Lys Thr Ile Ile Phe Lys Gln Ser Ser Gly Gly Asp
           355                 360                 365
   Pro Glu Ile Val Thr His Ser Phe Asn Cys Gly Gly Glu Phe Phe Tyr
       370                 375                 380
   Cys Asn Ser Thr Gln Leu Phe Asn Ser Thr Trp Phe Asn Ser Thr Trp
   385                 390                 395                 400
   Ser Thr Lys Gly Ser Asn Asn Thr Glu Gly Ser Asp Thr Ile Thr Leu
                   405                 410                 415
   Pro Cys Arg Ile Lys Gln Ile Ile Asn Met Trp Gln Glu Val Gly Lys
               420                 425                 430
   Ala Met Tyr Ala Pro Pro Ile Ser Gly Gln Ile Arg Cys Ser Ser Asn
           435                 440                 445
   Ile Thr Gly Leu Leu Leu Thr Arg Asp Gly Gly Asn Ser Asn Asn Glu
       450                 455                 460
   Ser Glu Ile Phe Arg Pro Gly Gly Gly Asp Met Arg Asp Asn Trp Arg
   465                 470                 475                 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Arg Arg Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   Asn Met Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Val
   690 695 700
   Val Asn Arg Val Arg Gln Gly Tyr Ser Pro Leu Ser Phe Gln Thr His
   705 710 715 720
   Leu Pro Ile Pro Arg Gly Pro Asp Arg Pro Glu Gly Ile Glu Glu Glu
                   725                 730                 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Tyr Arg Ala Ile Arg His Ile Pro Arg Arg Ile Arg
           835                 840                 845
   Gln Gly Leu Glu Arg Ile Leu Leu
       850                 855
<210> 270
   <211> 32
   <212> PRT
   <213> Artificial Sequence
<220>
   <223> fragemnt of lentiviral ENV protein
<220>
   <221> misc_feature
   <222> (1)..(1)
   <223> Xaa can be any naturally occurring amino acid
<220>
   <221> misc_feature
   <222> (4)..(5)
   <223> Xaa can be any naturally occurring amino acid
<220>
   <221> misc_feature
   <222> (7)..(8)
   <223> Xaa can be any naturally occurring amino acid
<220>
   <221> MISC_FEATURE
   <222> (12)..(12)
   <223> Xaa = A or S
<220>
   <221> misc_feature
   <222> (13)..(15)
   <223> Xaa can be any naturally occurring amino acid
<220>
   <221> misc_feature
   <222> (18)..(18)
   <223> Xaa can be any naturally occurring amino acid
<220>
   <221> misc_feature
   <222> (20)..(20)
   <223> Xaa can be any naturally occurring amino acid
<220>
   <221> misc_feature
   <222> (24)..(24)
   <223> Xaa can be any naturally occurring amino acid
<220>
   <221> MISC_FEATURE
   <222> (25)..(29)
   <223> Xaa = no or any amino acid
<220>
   <221> MISC_FEATURE
   <222> (30)..(30)
   <223> Xaa = N or S
<220>
   <221> MISC_FEATURE
   <222> (31)..(31)
   <223> Xaa = E, D, N or A
<220>
   <221> MISC_FEATURE
   <222> (32)..(32)
   <223> Xaa = S or T
<400> 270
   Xaa Asp Gln Xaa Xaa Leu Xaa Xaa Trp Gly Cys Xaa Xaa Xaa Xaa Cys
   1               5                   10                  15
   Val Xaa Thr Xaa Val Pro Trp Xaa Xaa Xaa Xaa Xaa Xaa Xaa Xaa Xaa
               20                  25                  30
<210> 271
   <211> 115
   <212> PRT
   <213> Artificial sequence
<220>
   <223> derived from lentiviral ENV ISU
<400> 271
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Trp Asn Ala Ser Trp Ser Asn Ala Ser Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 272
   <211> 115
   <212> PRT
   <213> Artificial sequence
<220>
   <223> derived from lentiviral ENV ISU
<400> 272
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Trp Asn Ala Ser Trp Ser Asn Gly Ser Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 273
   <211> 115
   <212> PRT
   <213> Artificial sequence
<220>
   <223> derived from lentiviral ENV ISU
<400> 273
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Trp Asn Ala Ser Trp Ser Asn Ser Ser Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 274
   <211> 115
   <212> PRT
   <213> Artificial sequence
<220>
   <223> derived from lentiviral ENV ISU
<400> 274
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Asp Asn Ala Ser Trp Ser Asn Lys Ser Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 275
   <211> 115
   <212> PRT
   <213> Artificial sequence
<220>
   <223> derived from lentiviral ENV ISU
<400> 275
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Ala Asn Ala Ser Trp Ser Asn Lys Ser Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
   100 105 110
   Gln Glu Leu
   115
<210> 276
   <211> 115
   <212> PRT
   <213> Artificial sequence
<220>
   <223> derived from lentiviral ENV ISU
<400> 276
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Asn Asn Ala Ser Trp Ser Asn Lys Ser Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 277
   <211> 115
   <212> PRT
   <213> Artificial sequence
<220>
   <223> derived from lentiviral ENV ISU
<400> 277
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Trp Asn Ala Ser Trp Ser Asn Lys Asp Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 278
   <211> 115
   <212> PRT
   <213> Artificial sequence
<220>
   <223> derived from lentiviral ENV ISU
<400> 278
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Arg Leu Lys Asp Gln Gln Leu Leu
           35                  40                  45
   Gly Ile Trp Gly Cys Ser Gly Lys Leu Ile Cys Thr Thr Ala Val Pro
       50                  55                  60
   Trp Asn Ala Ser Trp Ser Asn Lys Ala Leu Glu Gln Ile Trp Asn Asn
   65                  70                  75                  80
   Met Thr Trp Met Glu Trp Asp Arg Glu Ile Asn Asn Tyr Thr Ser Leu
                   85                  90                  95
   Ile His Ser Leu Ile Glu Glu Ser Gln Asn Gln Gln Glu Lys Asn Glu
               100                 105                 110
   Gln Glu Leu
           115
<210> 279
   <211> 115
   <212> PRT
   <213> Artificial sequence
<220>
   <223> derived from lentiviral ENV ISU
<400> 279
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Trp Asn Ala Ser Trp Ser Asn Lys Asn Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 280
   <211> 115
   <212> PRT
   <213> Artificial sequence
<220>
   <223> derived from lentiviral ENV ISU
<400> 280
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Trp Asn Ala Ser Trp Ser Asn Tyr Ser Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 281
   <211> 115
   <212> PRT
   <213> Artificial sequence
<220>
   <223> derived from lentiviral ENV ISU
<400> 281
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Ala Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Trp Asn Ala Ser Trp Ser Asn Ala Ser Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 282
   <211> 115
   <212> PRT
   <213> Artificial sequence
<220>
   <223> derived from lentiviral ENV ISU
<400> 282
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Trp Asn Ala Ser Trp Ser Asn Ala Ala Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 283
   <211> 115
   <212> PRT
   <213> Artificial Sequence
<220>
   <223> derived from lentiviral ENV ISU
<400> 283
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Arg Leu Lys Asp Gln Gln Leu Leu
   35 40 45
   Gly Ile Trp Gly Cys Ser Gly Lys Leu Ile Cys Thr Thr Ala Val Pro
   50 55 60
   Ala Asn Ala Ser Trp Ser Asn Ala Ala Leu Glu Gln Ile Trp Asn Asn
   65 70 75 80
   Met Thr Trp Met Glu Trp Asp Arg Glu Ile Asn Asn Tyr Thr Ser Leu
   85 90 95
   Ile His Ser Leu Ile Glu Glu Ser Gln Asn Gln Gln Glu Lys Asn Glu
   100 105 110
   Gln Glu Leu
   115
<210> 284
   <211> 861
   <212> PRT
   <213> Human immunodeficiency virus type 1
<400> 284
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 285
   <211> 861
   <212> PRT
   <213> Human immunodeficiency virus type 1
<400> 285
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Ala Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 286
   <211> 861
   <212> PRT
   <213> Human immunodeficiency virus type 1
<400> 286
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Arg Leu
               580                 585                 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Gly Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 287
   <211> 861
   <212> PRT
   <213> Human immunodeficiency virus type 1
<400> 287
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Arg Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Ser Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 288
   <211> 861
   <212> PRT
   <213> Human immunodeficiency virus type 1
<400> 288
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Gly Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 289
   <211> 861
   <212> PRT
   <213> Human immunodeficiency virus type 1
<400> 289
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Leu Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 290
   <211> 861
   <212> PRT
   <213> Human immunodeficiency virus type 1
<400> 290
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Ala Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 291
   <211> 861
   <212> PRT
   <213> Human immunodeficiency virus type 1
<400> 291
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Phe Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 292
   <211> 861
   <212> PRT
   <213> Human immunodeficiency virus type 1
<400> 292
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
   325 330 335
   Asn Ile Ser Arg Ala Lys Trp Asn Ala Thr Leu Lys Gln Ile Ala Ser
   340 345 350
   Lys Leu Arg Glu Gln Phe Gly Asn Asn Lys Thr Ile Ile Phe Lys Gln
   355 360 365
   Ser Ser Gly Gly Asp Pro Glu Ile Val Thr His Ser Phe Asn Cys Gly
   370 375 380
   Gly Glu Phe Phe Tyr Cys Asn Ser Thr Gln Leu Phe Asn Ser Thr Trp
   385 390 395 400
   Phe Asn Ser Thr Trp Ser Thr Glu Gly Ser Asn Asn Thr Glu Gly Ser
   405 410 415
   Asp Thr Ile Thr Leu Pro Cys Arg Ile Lys Gln Phe Ile Asn Met Trp
   420 425 430
   Gln Glu Val Gly Lys Ala Met Tyr Ala Pro Pro Ile Ser Gly Gln Ile
   435 440 445
   Arg Cys Ser Ser Asn Ile Thr Gly Leu Leu Leu Thr Arg Asp Gly Gly
   450 455 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Tyr Arg
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
           835                 840                 845
   Pro Arg Arg Ile Arg Gln Gly Leu Glu Arg Ile Leu Leu
       850                 855                 860
<210> 293
   <211> 881
   <212> PRT
   <213> Simian immunodeficiency virus
<220>
   <221> misc_feature
   <222> (736)..(736)
   <223> Xaa can be any naturally occurring amino acid
<400> 293
   Met Gly Cys Leu Gly Asn Gln Leu Leu Ile Ala Ile Leu Leu Leu Ser
   1               5                   10                  15
   Val Tyr Gly Ile Tyr Cys Thr Gln Tyr Val Thr Val Phe Tyr Gly Val
               20                  25                  30
   Pro Ala Trp Arg Asn Ala Thr Ile Pro Leu Phe Cys Ala Thr Lys Asn
           35                  40                  45
   Arg Asp Thr Trp Gly Thr Thr Gln Cys Leu Pro Asp Asn Gly Asp Tyr
       50                  55                  60
   Ser Glu Leu Ala Leu Asn Val Thr Glu Ser Phe Asp Ala Trp Glu Asn
   65                  70                  75                  80
   Thr Val Thr Glu Gln Ala Ile Glu Asp Val Trp Gln Leu Phe Glu Thr
                   85                  90                  95
   Ser Ile Lys Pro Cys Val Lys Leu Ser Pro Leu Cys Ile Thr Met Arg
               100                 105                 110
   Cys Asn Lys Ser Glu Thr Asp Arg Trp Gly Leu Thr Lys Ser Ser Thr
   115 120 125
   Thr Ile Thr Thr Ala Ala Pro Thr Ser Ala Pro Val Ser Glu Lys Ile
   130 135 140
   Asp Met Val Asn Glu Thr Ser Ser Cys Ile Ala Gln Asn Asn Cys Thr
   145 150 155 160
   Gly Leu Glu Gln Glu Gln Met Ile Ser Cys Lys Phe Thr Met Thr Gly
   165 170 175
   Leu Lys Arg Asp Lys Thr Lys Glu Tyr Asn Glu Thr Trp Tyr Ser Thr
   180 185 190
   Asp Leu Val Cys Glu Gln Gly Asn Ser Thr Asp Asn Glu Ser Arg Cys
   195 200 205
   Tyr Met Asn His Cys Asn Thr Ser Val Ile Gln Glu Ser Cys Asp Lys
   210 215 220
   His Tyr Trp Asp Thr Ile Arg Phe Arg Tyr Cys Ala Pro Pro Gly Tyr
   225 230 235 240
   Ala Leu Leu Arg Cys Asn Asp Thr Asn Tyr Ser Gly Phe Met Pro Lys
   245 250 255
   Cys Ser Lys Val Val Val Ser Ser Cys Thr Arg Met Met Glu Thr Gln
   260 265 270
   Thr Ser Thr Trp Phe Gly Phe Asn Gly Thr Arg Ala Glu Asn Arg Thr
   275 280 285
   Tyr Ile Tyr Trp His Gly Arg Asp Asn Arg Thr Ile Ile Ser Leu Asn
   290 295 300
   Lys Tyr Tyr Asn Leu Thr Met Lys Cys Arg Arg Pro Gly Asn Lys Thr
   305 310 315 320
   Val Leu Pro Val Thr Ile Met Ser Gly Leu Val Phe His Ser Gln Pro
   325 330 335
   Ile Asn Asp Arg Pro Lys Gln Ala Trp Cys Trp Phe Gly Gly Lys Trp
   340 345 350
   Lys Asp Ala Ile Lys Glu Val Lys Gln Thr Ile Val Lys His Pro Arg
   355 360 365
   Tyr Thr Gly Thr Asn Asn Thr Asp Lys Ile Asn Leu Thr Ala Pro Gly
       370                 375                 380
   Gly Gly Asp Pro Glu Val Thr Phe Met Trp Thr Asn Cys Arg Gly Glu
   385                 390                 395                 400
   Phe Leu Tyr Cys Lys Met Asn Trp Phe Leu Asn Trp Val Glu Asp Arg
                   405                 410                 415
   Asp Val Thr Thr Gln Arg Pro Lys Glu Arg His Arg Arg Asn Tyr Val
               420                 425                 430
   Pro Cys His Ile Arg Gln Ile Ile Asn Thr Trp His Lys Val Gly Lys
           435                 440                 445
   Asn Val Tyr Leu Pro Pro Arg Glu Gly Asp Leu Thr Cys Asn Ser Thr
       450                 455                 460
   Val Thr Ser Leu Ile Ala Asn Ile Asp Trp Thr Asp Gly Asn Gln Thr
   465                 470                 475                 480
   Ser Ile Thr Met Ser Ala Glu Val Ala Glu Leu Tyr Arg Leu Glu Leu
                   485                 490                 495
   Gly Asp Tyr Lys Leu Val Glu Ile Thr Pro Ile Gly Leu Ala Pro Thr
               500                 505                 510
   Asp Val Lys Arg Tyr Thr Thr Gly Gly Thr Ser Arg Asn Lys Arg Gly
           515                 520                 525
   Val Phe Val Leu Gly Phe Leu Gly Phe Leu Ala Thr Ala Gly Ser Ala
       530                 535                 540
   Met Gly Ala Ala Ser Leu Thr Leu Thr Ala Gln Ser Arg Thr Leu Leu
   545                 550                 555                 560
   Ala Gly Ile Val Gln Gln Gln Gln Gln Leu Leu Asp Val Val Lys Arg
                   565                 570                 575
   Gln Gln Glu Leu Leu Arg Leu Thr Val Trp Gly Thr Lys Asn Leu Gln
               580                 585                 590
   Thr Arg Val Thr Ala Ile Glu Lys Tyr Arg Lys Asp Gln Ala Gln Leu
           595                 600                 605
   Asn Ala Trp Gly Cys Ala Phe Arg Gln Val Cys His Thr Thr Val Pro
       610                 615                 620
   Trp Pro Asn Ala Ser Leu Thr Pro Asp Trp Asn Asn Asp Thr Trp Gln
   625 630 635 640
   Glu Trp Glu Arg Lys Val Asp Phe Leu Glu Glu Asn Ile Thr Ala Leu
   645 650 655
   Leu Glu Glu Ala Gln Ile Gln Gln Glu Lys Asn Met Tyr Glu Leu Gln
   660 665 670
   Lys Leu Asn Ser Trp Asp Val Phe Gly Asn Trp Phe Asp Leu Ala Ser
   675 680 685
   Trp Ile Lys Tyr Ile Gln Tyr Gly Ile Tyr Val Val Val Gly Val Ile
   690 695 700
   Leu Leu Arg Ile Val Ile Tyr Ile Val Gln Met Leu Ala Lys Leu Arg
   705 710 715 720
   Gln Gly Tyr Arg Pro Val Phe Ser Ser Pro Pro Ser Tyr Phe Gln Xaa
   725 730 735
   Thr His Thr Gln Gln Asp Pro Ala Leu Pro Thr Arg Glu Gly Lys Glu
   740 745 750
   Gly Asp Gly Gly Glu Gly Gly Gly Asn Ser Ser Trp Pro Trp Gln Ile
   755 760 765
   Glu Tyr Ile His Phe Leu Ile Arg Gln Leu Ile Arg Leu Leu Thr Trp
   770 775 780
   Leu Phe Ser Asn Cys Arg Thr Leu Leu Ser Arg Ala Tyr Gln Ile Leu
   785 790 795 800
   Gln Pro Ile Leu Gln Arg Leu Ser Ala Thr Leu Arg Arg Val Arg Glu
   805 810 815
   Val Leu Arg Thr Glu Leu Thr Tyr Leu Gln Tyr Gly Trp Ser Tyr Phe
   820 825 830
   His Glu Ala Val Gln Ala Gly Trp Arg Ser Ala Thr Glu Thr Leu Ala
   835 840 845
   Gly Ala Trp Arg Asp Leu Trp Glu Thr Leu Arg Arg Gly Gly Arg Trp
   850 855 860
   Ile Leu Ala Ile Pro Arg Arg Ile Arg Gln Gly Leu Glu Leu Thr Leu
   865 870 875 880
   Leu
<210> 294
   <211> 860
   <212> PRT
   <213> Human immunodeficiency virus type 2
<400> 294
   Met Glu Pro Gly Arg Asn Gln Leu Phe Val Val Ile Leu Leu Thr Ser
   1               5                   10                  15
   Ala Cys Leu Val Tyr Cys Ser Gln Tyr Val Thr Val Phe Tyr Gly Ile
               20                  25                  30
   Pro Ala Trp Lys Asn Ala Ser Ile Pro Leu Phe Cys Ala Thr Lys Asn
           35                  40                  45
   Arg Asp Thr Trp Gly Thr Ile Gln Cys Leu Pro Asp Asn Asp Asp Tyr
       50                  55                  60
   Gln Glu Ile Ile Leu Asn Val Thr Glu Ala Phe Asp Ala Trp Asn Asn
   65                  70                  75                  80
   Thr Val Thr Glu Gln Ala Val Glu Asp Val Trp His Leu Phe Glu Thr
                   85                  90                  95
   Ser Ile Lys Pro Cys Val Lys Leu Thr Pro Leu Cys Val Ala Met Asn
               100                 105                 110
   Cys Ser Arg Val Gln Gly Asn Thr Thr Thr Pro Asn Pro Arg Thr Ser
           115                 120                 125
   Ser Ser Thr Thr Ser Arg Pro Pro Thr Ser Ala Ala Ser Ile Ile Asn
       130                 135                 140
   Glu Thr Ser Asn Cys Ile Glu Asn Asn Thr Cys Ala Gly Leu Gly Tyr
   145                 150                 155                 160
   Glu Glu Met Met Gln Cys Glu Phe Asn Met Lys Gly Leu Glu Gln Asp
                   165                 170                 175
   Lys Lys Arg Arg Tyr Lys Asp Thr Trp Tyr Leu Glu Asp Val Val Cys
               180                 185                 190
   Asp Asn Thr Thr Ala Gly Thr Cys Tyr Met Arg His Cys Asn Thr Ser
           195                 200                 205
   Ile Ile Lys Glu Ser Cys Asp Lys His Tyr Trp Asp Ala Met Arg Phe
       210                 215                 220
   Arg Tyr Cys Ala Pro Pro Gly Phe Ala Leu Leu Arg Cys Asn Asp Thr
   225                 230                 235                 240
   Asn Tyr Ser Gly Phe Glu Pro Lys Cys Thr Lys Val Val Ala Ala Ser
                   245                 250                 255
   Cys Thr Arg Met Met Glu Thr Gln Thr Ser Thr Trp Phe Gly Phe Asn
               260                 265                 270
   Gly Thr Arg Ala Glu Asn Arg Thr Tyr Ile Tyr Trp His Gly Arg Asp
           275                 280                 285
   Asn Arg Thr Ile Ile Ser Leu Asn Lys Tyr Tyr Asn Leu Thr Met Arg
       290                 295                 300
   Cys Lys Arg Pro Gly Asn Lys Thr Val Leu Pro Ile Thr Leu Met Ser
   305                 310                 315                 320
   Gly Leu Val Phe His Ser Gln Pro Ile Asn Thr Arg Pro Arg Gln Ala
                   325                 330                 335
   Trp Cys Arg Phe Gly Gly Arg Trp Arg Glu Ala Met Gln Glu Val Lys
               340                 345                 350
   Gln Thr Leu Val Gln His Pro Arg Tyr Lys Gly Ile Asn Asp Thr Gly
           355                 360                 365
   Lys Ile Asn Phe Thr Lys Pro Gly Ala Gly Ser Asp Pro Glu Val Ala
       370                 375                 380
   Phe Met Trp Thr Asn Cys Arg Gly Glu Phe Leu Tyr Cys Asn Met Thr
   385                 390                 395                 400
   Trp Phe Leu Asn Trp Val Glu Asp Lys Asn Gln Thr Arg Arg Asn Tyr
                   405                 410                 415
   Cys His Ile Lys Gln Ile Ile Asn Thr Trp His Lys Val Gly Lys Asn
               420                 425                 430
   Val Tyr Leu Pro Pro Arg Glu Gly Glu Leu Ala Cys Glu Ser Thr Val
           435                 440                 445
   Thr Ser Ile Ile Ala Asn Ile Asp Ile Asp Lys Asn Arg Thr His Thr
   450 455 460
   Asn Ile Thr Phe Ser Ala Glu Val Ala Glu Leu Tyr Arg Leu Glu Leu
   465 470 475 480
   Gly Asp Tyr Lys Leu Ile Glu Ile Thr Pro Ile Gly Phe Ala Pro Thr
   485 490 495
   Asp Gln Arg Arg Tyr Ser Ser Thr Pro Val Arg Asn Lys Arg Gly Val
   500 505 510
   Phe Val Leu Gly Phe Leu Gly Phe Leu Ala Thr Ala Gly Ser Ala Met
   515 520 525
   Gly Ala Arg Ser Leu Thr Leu Ser Ala Gln Ser Arg Thr Leu Leu Ala
   530 535 540
   Gly Ile Val Gln Gln Gln Gln Gln Leu Leu Asp Val Val Lys Arg Gln
   545 550 555 560
   Gln Glu Met Leu Arg Leu Thr Val Trp Gly Thr Lys Asn Leu Gln Ala
   565 570 575
   Arg Val Thr Ala Ile Glu Lys Tyr Arg Lys His Gln Ala Gln Leu Asn
   580 585 590
   Ser Trp Gly Cys Ala Phe Arg Gln Val Cys His Thr Thr Val Pro Trp
   595 600 605
   Val Asn Asp Ser Leu Ser Pro Asp Trp Lys Asn Met Thr Trp Gln Glu
   610 615 620
   Trp Glu Lys Gln Val Arg Tyr Leu Glu Ala Asn Ile Ser Gln Ser Leu
   625 630 635 640
   Glu Glu Ala Gln Ile Gln Gln Glu Lys Asn Met Tyr Glu Leu Gln Lys
   645 650 655
   Leu Asn Ser Trp Asp Ile Leu Gly Asn Trp Phe Asp Leu Thr Ser Trp
   660 665 670
   Val Lys Tyr Ile Gln Tyr Gly Val His Ile Val Val Gly Ile Ile Ala
   675 680 685
   Leu Arg Ile Ala Ile Tyr Val Val Gln Leu Leu Ser Arg Phe Arg Lys
   690 695 700
   Gly Tyr Arg Pro Val Phe Ser Ser Pro Pro Gly Tyr Leu Gln Gln Ile
   705                 710                 715                 720
   His Ile His Lys Asp Arg Gly Gln Pro Ala Asn Glu Gly Thr Glu Glu
                   725                 730                 735
   Asp Val Gly Gly Asp Ser Gly Tyr Asp Leu Trp Pro Trp Pro Ile Asn
               740                 745                 750
   Tyr Val Gln Phe Leu Ile His Leu Leu Thr Arg Leu Leu Ile Gly Leu
           755                 760                 765
   Tyr Asn Ile Cys Arg Asp Leu Leu Ser Lys Asn Ser Pro Thr Arg Arg
       770                 775                 780
   Leu Ile Ser Gln Ser Leu Thr Ala Ile Arg Asp Trp Leu Arg Leu Lys
   785                 790                 795                 800
   Ala Ala Gln Leu Gln Tyr Gly Cys Glu Trp Ile Gln Glu Ala Phe Gln
                   805                 810                 815
   Ala Phe Ala Arg Thr Thr Arg Glu Thr Leu Ala Gly Ala Trp Gly Trp
               820                 825                 830
   Leu Trp Glu Ala Ala Arg Arg Ile Gly Arg Gly Ile Leu Ala Val Pro
           835                 840                 845
   Arg Arg Ile Arg Gln Gly Ala Glu Leu Ala Leu Leu
       850                 855                 860
<210> 295
   <211> 36
   <212> DNA
   <213> Artificial sequence
<220>
   <223> oligonucleotide for introducing mutation within the ISU of lentiviral ENV protein
<400> 295
   acatggccca gccggcctct ggtatagtgc agcagc 36
<210> 296
   <211> 34
   <212> DNA
   <213> Artificial sequence
<220>
   <223> oligonucleotide for introducing mutation within the ISU of lentiviral ENV protein
<400> 296
   gtatacgcgt ttataattct tgttcattct tttc 34
<210> 297
   <211> 35
   <212> DNA
   <213> Artificial sequence
<220>
   <223> oligonucleotide for introducing mutation within the ISU of lentiviral ENV protein
<400> 297
   gtatacgcgt ttacatgtta ttccaaatct gttcc 35
<210> 298
   <211> 31
   <212> DNA
   <213> Artificial sequence
<220>
   <223> oligonucleotide for introducing mutation within the ISU of lentiviral ENV protein
<400> 298
   gtatacgcgt ttaagcattc caaggcacag c 31
<210> 299
   <211> 33
   <212> DNA
   <213> Artificial sequence
<220>
   <223> oligonucleotide for introducing mutation within the ISU of lentiviral ENV protein
<400> 299
   gtatacgcgt ttatccagag caaccccaaa tcc 33
<210> 300
   <211> 32
   <212> DNA
   <213> Artificial sequence
<220>
   <223> oligonucleotide for introducing mutation within the ISU of lentiviral ENV protein
<400> 300
   gtatacgcgt ttaccccagg agctgttgat cc 32
<210> 301
   <211> 34
   <212> DNA
   <213> Artificial sequence
<220>
   <223> oligonucleotide for introducing mutation within the ISU of lentiviral ENV protein
<400> 301
   gtatacgcgt ttactttagg tatctttcca cagc 34
<210> 302
   <211> 34
   <212> DNA
   <213> Artificial sequence
<220>
   <223> oligonucleotide for introducing mutation within the ISU of lentiviral ENV protein
<400> 302
   gtatacgcgt ttaagccagg attcttgcct ggag 34
<210> 303
   <211> 31
   <212> DNA
   <213> Artificial sequence
<220>
   <223> oligonucleotide for introducing mutation within the ISU of lentiviral ENV protein
<400> 303
   gtatacgcgt ttactgcttg atgccccaga c 31
<210> 304
   <211> 26
   <212> DNA
   <213> Artificial sequence
<220>
   <223> oligonucleotide for introducing mutation within the ISU of lentiviral ENV protein
<400> 304
   ctttccacag cccggattct tgcctg 26
<210> 305
   <211> 26
   <212> DNA
   <213> Artificial sequence
<220>
   <223> oligonucleotide for introducing mutation within the ISU of lentiviral ENV protein
<400> 305
   caggcaagaa tccgggctgt ggaaag 26
<210> 306
   <211> 28
   <212> DNA
   <213> Artificial sequence
<220>
   <223> oligonucleotide for introducing mutation within the ISU of lentiviral ENV protein
<400> 306
   gtatctttcc acacgcagga ttcttgcc 28
<210> 307
   <211> 28
   <212> DNA
   <213> Artificial sequence
<220>
   <223> oligonucleotide for introducing mutation within the ISU of lentiviral ENV protein
<400> 307
   ggcaagaatc ctgcgtgtgg aaagatac 28
<210> 308
   <211> 34
   <212> DNA
   <213> Artificial sequence
<220>
   <223> oligonucleotide for introducing mutation within the ISU of lentiviral ENV protein
<400> 308
   ctttaggtat ctttccctag ccaggattct tgcc 34
<210> 309
   <211> 34
   <212> DNA
   <213> Artificial sequence
<220>
   <223> oligonucleotide for introducing mutation within the ISU of lentiviral ENV protein
<400> 309
   ggcaagaatc ctggctaggg aaagatacct aaag 34
<210> 310
   <211> 30
   <212> DNA
   <213> Artificial sequence
<220>
   <223> oligonucleotide for introducing mutation within the ISU of lentiviral ENV protein
<400> 310
   cctttaggta tcttctcaca gccaggattc 30
<210> 311
   <211> 30
   <212> DNA
   <213> Artificial sequence
<220>
   <223> oligonucleotide for introducing mutation within the ISU of lentiviral ENV protein
<400> 311
   gaatcctggc tgtgagaaga tacctaaagg 30
<210> 312
   <211> 31
   <212> DNA
   <213> Artificial sequence
<220>
   <223> oligonucleotide for introducing mutation within the ISU of lentiviral ENV protein
<400> 312
   gttgatcctt taggcgtctt tccacagcca g 31
<210> 313
   <211> 31
   <212> DNA
   <213> Artificial sequence
<220>
   <223> oligonucleotide for introducing mutation within the ISU of lentiviral ENV protein
<400> 313
   ctggctgtgg aaagacgcct aaaggatcaa c 31
<210> 314
   <211> 35
   <212> DNA
   <213> Artificial sequence
<220>
   <223> oligonucleotide for introducing mutation within the ISU of lentiviral ENV protein
<400> 314
   ggagctgttg atcctttcgg tatctttcca cagcc 35
<210> 315
   <211> 35
   <212> DNA
   <213> Artificial sequence
<220>
   <223> oligonucleotide for introducing mutation within the ISU of lentiviral ENV protein
<400> 315
   ggctgtggaa agataccgaa aggatcaaca gctcc 35
<210> 316
   <211> 30
   <212> DNA
   <213> Artificial sequence
<220>
   <223> oligonucleotide for introducing mutation within the ISU of lentiviral ENV protein
<400> 316
   gagctgttga tcccttaggt atctttccac 30
<210> 317
   <211> 30
   <212> DNA
   <213> Artificial sequence
<220>
   <223> oligonucleotide for introducing mutation within the ISU of lentiviral ENV protein
<400> 317
   gtggaaagat acctaaggga tcaacagctc 30
<210> 318
   <211> 28
   <212> DNA
   <213> Artificial sequence
<220>
   <223> oligonucleotide for introducing mutation within the ISU of lentiviral ENV protein
<400> 318
   aggagctgtt gacgctttag gtatcttt 28
<210> 319
   <211> 28
   <212> DNA
   <213> Artificial sequence
<220>
   <223> oligonucleotide for introducing mutation within the ISU of lentiviral ENV protein
<400> 319
   aaagatacct aaagcgtcaa cagctcct 28
<210> 320
   <211> 28
   <212> DNA
   <213> Artificial sequence
<220>
   <223> oligonucleotide for introducing mutation within the ISU of lentiviral ENV protein
<400> 320
   cccaggagct gtcgatcctt taggtatc 28
<210> 321
   <211> 28
   <212> DNA
   <213> Artificial sequence
<220>
   <223> oligonucleotide for introducing mutation within the ISU of lentiviral ENV protein
<400> 321
   gatacctaaa ggatcgacag ctcctggg 28
<210> 322
   <211> 29
   <212> DNA
   <213> Artificial sequence
<220>
   <223> oligonucleotide for introducing mutation within the ISU of lentiviral ENV protein
<400> 322
   caaatcccca ggagccgttg atcctttag 29
<210> 323
   <211> 29
   <212> DNA
   <213> Artificial sequence
<220>
   <223> oligonucleotide for introducing mutation within the ISU of lentiviral ENV protein
<400> 323
   ctaaaggatc aacggctcct ggggatttg 29
<210> 324
   <211> 29
   <212> DNA
   <213> Artificial sequence
<220>
   <223> oligonucleotide for introducing mutation within the ISU of lentiviral ENV protein
<400> 324
   caaatcccca ggcgctgttg atcctttag 29
<210> 325
   <211> 29
   <212> DNA
   <213> Artificial sequence
<220>
   <223> oligonucleotide for introducing mutation within the ISU of lentiviral ENV protein
<400> 325
   ctaaaggatc aacagcgcct ggggatttg 29
<210> 326
   <211> 22
   <212> DNA
   <213> Artificial sequence
<220>
   <223> oligonucleotide for introducing mutation within the ISU of lentiviral ENV protein
<400> 326
   cccaaatccc ccggagctgt tg 22
<210> 327
   <211> 22
   <212> DNA
   <213> Artificial sequence
<220>
   <223> oligonucleotide for introducing mutation within the ISU of lentiviral ENV protein
<400> 327
   caacagctcc gggggatttg gg 22
<210> 328
   <211> 23
   <212> DNA
   <213> Artificial sequence
<220>
   <223> oligonucleotide for introducing mutation within the ISU of lentiviral ENV protein
<400> 328
   ccccaaatcc gcaggagctg ttg 23
<210> 329
   <211> 23
   <212> DNA
   <213> Artificial sequence
<220>
   <223> oligonucleotide for introducing mutation within the ISU of lentiviral ENV protein
<400> 329
   caacagctcc tgcggatttg ggg 23
<210> 330
   <211> 24
   <212> DNA
   <213> Artificial sequence
<220>
   <223> oligonucleotide for introducing mutation within the ISU of lentiviral ENV protein
<400> 330
   gcaaccccat ctccccagga gctg 24
<210> 331
   <211> 24
   <212> DNA
   <213> Artificial sequence
<220>
   <223> oligonucleotide for introducing mutation within the ISU of lentiviral ENV protein
<400> 331
   cagctcctgg ggagatgggg ttgc 24
<210> 332
   <211> 24
   <212> DNA
   <213> Artificial sequence
<220>
   <223> oligonucleotide for introducing mutation within the ISU of lentiviral ENV protein
<400> 332
   gcaaccccat ctccccagga gctg 24
<210> 333
   <211> 24
   <212> DNA
   <213> Artificial sequence
<220>
   <223> oligonucleotide for introducing mutation within the ISU of lentiviral ENV protein
<400> 333
   cagctcctgg ggagatgggg ttgc 24
<210> 334
   <211> 31
   <212> DNA
   <213> Artificial sequence
<220>
   <223> oligonucleotide for introducing mutation within the ISU of lentiviral ENV protein
<400> 334
   gttgatcctt tagttttctt tccacagcca g 31
<210> 335
   <211> 31
   <212> DNA
   <213> Artificial sequence
<220>
   <223> oligonucleotide for introducing mutation within the ISU of lentiviral ENV protein
<400> 335
   ctggctgtgg aaagaaaact aaaggatcaa c 31
<210> 336
   <211> 31
   <212> DNA
   <213> Artificial sequence
<220>
   <223> oligonucleotide for introducing mutation within the ISU of lentiviral ENV protein
<400> 336
   gttgatcctt taggtgtctt tccacagcca g 31
<210> 337
   <211> 31
   <212> DNA
   <213> Artificial sequence
<220>
   <223> oligonucleotide for introducing mutation within the ISU of lentiviral ENV protein
<400> 337
   ctggctgtgg aaagacacct aaaggatcaa c 31
<210> 338
   <211> 31
   <212> DNA
   <213> Artificial sequence
<220>
   <223> oligonucleotide for introducing mutation within the ISU of lentiviral ENV protein
<400> 338
   gttgatcctt tagggctctt tccacagcca g 31
<210> 339
   <211> 31
   <212> DNA
   <213> Artificial sequence
<220>
   <223> oligonucleotide for introducing mutation within the ISU of lentiviral ENV protein
<400> 339
   ctggctgtgg aaagagccct aaaggatcaa c 31
<210> 340
   <211> 31
   <212> DNA
   <213> Artificial sequence
<220>
   <223> oligonucleotide for introducing mutation within the ISU of lentiviral ENV protein
<400> 340
   gttgatcctt taggcctctt tccacagcca g 31
<210> 341
   <211> 31
   <212> DNA
   <213> Artificial sequence
<220>
   <223> oligonucleotide for introducing mutation within the ISU of lentiviral ENV protein
<400> 341
   ctggctgtgg aaagaggcct aaaggatcaa c 31
<210> 342
   <211> 31
   <212> DNA
   <213> Artificial sequence
<220>
   <223> oligonucleotide for introducing mutation within the ISU of lentiviral ENV protein
<400> 342
   gttgatcctt taggaatctt tccacagcca g 31
<210> 343
   <211> 31
   <212> DNA
   <213> Artificial sequence
<220>
   <223> oligonucleotide for introducing mutation within the ISU of lentiviral ENV protein
<400> 343
   ctggctgtgg aaagattcct aaaggatcaa c 31
<210> 344
   <211> 31
   <212> DNA
   <213> Artificial sequence
<220>
   <223> oligonucleotide for introducing mutation within the ISU of lentiviral ENV protein
<400> 344
   gttgatcctt taggagtctt tccacagcca g 31
<210> 345
   <211> 31
   <212> DNA
   <213> Artificial sequence
<220>
   <223> oligonucleotide for introducing mutation within the ISU of lentiviral ENV protein
<400> 345
   ctggctgtgg aaagactcct aaaggatcaa c 31
<210> 346
   <211> 31
   <212> DNA
   <213> Artificial sequence
<220>
   <223> oligonucleotide for introducing mutation within the ISU of lentiviral ENV protein
<400> 346
   acaaattaga tgttcatcaa atattacagg g 31
<210> 347
   <211> 44
   <212> DNA
   <213> Artificial sequence
<220>
   <223> oligonucleotide for introducing mutation within the ISU of lentiviral ENV protein
<400> 347
   gcaacagatg ctgttgggcc tcaatggccc tcagcaaatt gttc 44
<210> 348
   <211> 44
   <212> DNA
   <213> Artificial sequence
<220>
   <223> oligonucleotide for introducing mutation within the ISU of lentiviral ENV protein
<400> 348
   gaacaatttg ctgagggcca ttgaggccca acagcatctg ttgc 44
<210> 349
   <211> 32
   <212> DNA
   <213> Artificial sequence
<220>
   <223> oligonucleotide for introducing mutation within the ISU of lentiviral ENV protein
<400> 349
   gtgtattaag cttgtgtaat tgttaatttc tc 32
<210> 350
   <211> 25
   <212> DNA
   <213> Artificial sequence
<220>
   <223> oligonucleotide for introducing mutation within the ISU of lentiviral ENV protein
<400> 350
   ccgctcagtc ccgaacttta ttggc 25
<210> 351
   <211> 22
   <212> DNA
   <213> Artificial sequence
<220>
   <223> oligonucleotide for introducing mutation within the ISU of lentiviral ENV protein
<400> 351
   ggtggggaag agaacactgg cc 22
<210> 352
   <211> 45
   <212> DNA
   <213> Artificial sequence
<220>
   <223> oligonucleotide for introducing mutation within the ISU of lentiviral ENV protein
<400> 352
   cagactaggg tcactgccat ccgcaagtac ttaaaggacc aggcg 45
<210> 353
   <211> 45
   <212> DNA
   <213> Artificial sequence
<220>
   <223> oligonucleotide for introducing mutation within the ISU of lentiviral ENV protein
<400> 353
   cgcctggtcc tttaagtact tgcggatggc agtgacccta gtctg 45
<210> 354
   <211> 45
   <212> DNA
   <213> Artificial sequence
<220>
   <223> oligonucleotide for introducing mutation within the ISU of lentiviral ENV protein
<400> 354
   actagggtca ctgccatcga gcgctactta aaggaccagg cgcag 45
<210> 355
   <211> 45
   <212> DNA
   <213> Artificial sequence
<220>
   <223> oligonucleotide for introducing mutation within the ISU of lentiviral ENV protein
<400> 355
   ctgcgcctgg tcctttaagt agcgctcgat ggcagtgacc ctagt 45
<210> 356
   <211> 30
   <212> DNA
   <213> Artificial sequence
<220>
   <223> oligonucleotide for introducing mutation within the ISU of lentiviral ENV protein
<400> 356
   gccatcgaga agcgcttaaa ggaccaggcg 30
<210> 357
   <211> 30
   <212> DNA
   <213> Artificial sequence
<220>
   <223> oligonucleotide for introducing mutation within the ISU of lentiviral ENV protein
<400> 357
   cgcctggtcc tttaagcgct tctcgatggc 30
<210> 358
   <211> 42
   <212> DNA
   <213> Artificial sequence
<220>
   <223> oligonucleotide for introducing mutation within the ISU of lentiviral ENV protein
<400> 358
   gtcactgcca tcgagaagta ccgcaaggac caggcgcagc tg 42
<210> 359
   <211> 42
   <212> DNA
   <213> Artificial sequence
<220>
   <223> oligonucleotide for introducing mutation within the ISU of lentiviral ENV protein
<400> 359
   cagctgcgcc tggtccttgc ggtacttctc gatggcagtg ac 42
<210> 360
   <211> 45
   <212> DNA
   <213> Artificial sequence
<220>
   <223> oligonucleotide for introducing mutation within the ISU of lentiviral ENV protein
<400> 360
   gccatcgaga agtacttacg cgaccaggcg cagctgaatg cttgg 45
<210> 361
   <211> 45
   <212> DNA
   <213> Artificial sequence
<220>
   <223> oligonucleotide for introducing mutation within the ISU of lentiviral ENV protein
<400> 361
   ccaagcattc agctgcgcct ggtcgcgtaa gtacttctcg atggc 45
<210> 362
   <211> 39
   <212> DNA
   <213> Artificial sequence
<220>
   <223> oligonucleotide for introducing mutation within the ISU of lentiviral ENV protein
<400> 362
   gagaagtact taaagcgcca ggcgcagctg aatgcttgg 39
<210> 363
   <211> 39
   <212> DNA
   <213> Artificial sequence
<220>
   <223> oligonucleotide for introducing mutation within the ISU of lentiviral ENV protein
<400> 363
   attcagctgc gcctggcgct ttaagtactt ctcgatggc 39
<210> 364
   <211> 36
   <212> DNA
   <213> Artificial sequence
<220>
   <223> oligonucleotide for introducing mutation within the ISU of lentiviral ENV protein
<400> 364
   acatggccca gccggccgct gggatagtgc agcaac 36
<210> 365
   <211> 35
   <212> DNA
   <213> Artificial sequence
<220>
   <223> oligonucleotide for introducing mutation within the ISU of lentiviral ENV protein
<400> 365
   gtatacgcgt ttaaaacgca catccccaag cattc 35
<210> 366
   <211> 49
   <212> PRT
   <213> Artificial sequence
<220>
   <223> derived from the ISU domain of HIV1
<400> 366
   Ser Gly Ile Val Gln Gln Gln Asn Asn Leu Leu Arg Ala Ile Glu Ala
   1 5 10 15
   Gln Gln His Leu Leu Gln Leu Thr Val Trp Gly Ile Lys Gln Leu Gln
   20 25 30
   Ala Arg Ile Leu Ala Val Glu Arg Tyr Leu Lys Asp Gln Gln Leu Leu
   35 40 45
   Gly
<210> 367
   <211> 49
   <212> PRT
   <213> Artificial sequence
<220>
   <223> derived from the ISU domain of HIV1
<400> 367
   Ser Gly Ile Val Gln Gln Gln Asn Asn Leu Leu Arg Ala Ile Glu Ala
   1 5 10 15
   Gln Gln His Leu Leu Lys Leu Thr Val Trp Gly Ile Lys Gln Leu Gln
   20 25 30
   Ala Arg Ile Leu Ala Val Glu Arg Tyr Leu Lys Asp Gln Gln Leu Leu
   35 40 45
   Gly
<210> 368
   <211> 49
   <212> PRT
   <213> Artificial sequence
<220>
   <223> derived from the ISU domain of HIV1
<400> 368
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Val Leu Ala Val Glu Arg Tyr Leu Lys Asp Gln Gln Leu Leu
           35                  40                  45
   Gly
<210> 369
   <211> 49
   <212> PRT
   <213> Artificial sequence
<220>
   <223> derived from the ISU domain of HIV1
<400> 369
   Ser Gly Ile Val Gln Gln Gln Asn Asn Leu Leu Arg Ala Ile Glu Ala
   1               5                   10                  15
   Gln Gln His Leu Leu Gln Leu Thr Val Trp Gly Ile Lys Gln Leu Gln
               20                  25                  30
   Ala Arg Ile Leu Ala Val Glu Arg Tyr Leu Lys Asp Gln Gln Leu Leu
           35                  40                  45
   Gly
<210> 370
   <211> 49
   <212> PRT
   <213> Artificial sequence
<220>
   <223> derived from the ISU domain of HIV1
<400> 370
   Ser Gly Ile Val Gln Gln Gln Asn Asn Leu Leu Arg Ala Ile Glu Ala
   1 5 10 15
   Gln Gln Gln Met Leu Gln Leu Thr Val Trp Gly Ile Lys Gln Leu Arg
   20 25 30
   Ala Arg Val Leu Ala Val Glu Arg Tyr Leu Arg Asp Gln Gln Leu Leu
   35 40 45
   Gly
<210> 371
   <211> 49
   <212> PRT
   <213> Artificial Sequence
<220>
   <223> derived from the ISU domain of HIV1
<400> 371
   Ser Gly Ile Val Gln Gln Gln Ser Asn Leu Leu Arg Ala Ile Gln Ala
   1 5 10 15
   Arg Gln His Met Leu Gln Leu Thr Val Trp Gly Ile Lys Gln Leu Gln
   20 25 30
   Ala Arg Val Leu Ala Val Glu Arg Tyr Leu Arg Asp Gln Gln Leu Leu
   35 40 45
   Gly
<210> 372
   <211> 49
   <212> PRT
   <213> Artificial sequence
<220>
   <223> derived from the ISU domain of SIV
<400> 372
   Ser Gly Ile Val Gln Gln Gln Asn Asn Leu Leu Lys Ala Ile Glu Ala
   1 5 10 15
   Gln Gln His Leu Leu Gln Leu Ser Ile Trp Gly Val Lys Gln Leu Gln
   20 25 30
   Ala Arg Leu Leu Ala Val Glu Arg Tyr Leu Gln Asp Gln Gln Ile Leu
   35 40 45
   Gly
<210> 373
   <211> 49
   <212> PRT
   <213> Artificial sequence
<220>
   <223> derived from the ISU domain of SIV
<400> 373
   Ser Gly Ile Val Gln Gln Gln Asn Asn Leu Leu Arg Ala Ile Glu Ala
   1 5 10 15
   Gln Gln His Leu Leu Gln Leu Ser Val Trp Gly Ile Lys Gln Leu Gln
   20 25 30
   Ala Arg Val Leu Ala Ile Glu Arg Tyr Leu Arg Asp Gln Gln Ile Leu
   35 40 45
   Gly
<210> 374
   <211> 49
   <212> PRT
   <213> Artificial sequence
<220>
   <223> derived from the ISU domain of SIV
<400> 374
   Ala Gly Ile Val Gln Gln Gln Gln Gln Leu Leu Asp Val Val Lys Arg
   1 5 10 15
   Gln Gln Glu Leu Leu Arg Leu Thr Val Trp Gly Thr Lys Asn Leu Gln
   20 25 30
   Thr Arg Val Ser Ala Ile Glu Lys Tyr Leu Lys Asp Gln Ala Gln Leu
   35 40 45
   Asn
<210> 375
   <211> 49
   <212> PRT
   <213> Artificial Sequence
<220>
   <223> derived from the ISU domain of SIV
<400> 375
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Tyr Leu Lys Asp Gln Ala Gln Leu
           35                  40                  45
   Asn
<210> 376
   <211> 49
   <212> PRT
   <213> Artificial sequence
<220>
   <223> derived from the ISU domain of HIV2
<400> 376
   Ala Gly Ile Val Gln Gln Gln Gln Gln Leu Leu Asp Val Val Lys Arg
   1               5                   10                  15
   Gln Gln Glu Met Leu Arg Leu Thr Val Trp Gly Thr Lys Asn Leu Gln
               20                  25                  30
   Ala Arg Val Thr Ala Ile Glu Lys Tyr Leu Lys Asp Gln Ala Gln Leu
           35                  40                  45
   Asn
<210> 377
   <211> 49
   <212> PRT
   <213> Artificial Sequence
<220>
   <223> derived from the ISU domain of HIV2
<400> 377
   Ala Gly Ile Val Gln Gln Gln Gln Gln Leu Leu Asp Val Val Lys Arg
   1               5                   10                  15
   Gln Gln Glu Leu Leu Arg Leu Thr Val Trp Gly Thr Lys Asn Leu Gln
               20                  25                  30
   Thr Arg Val Thr Ala Ile Glu Lys Tyr Leu Lys Asp Gln Ala Leu Leu
           35                  40                  45
   Asn
<210> 378
   <211> 31
   <212> DNA
   <213> Artificial sequence
<220>
   <223> Oligonucleotide used for introducing a second mutation
<400> 378
   ctggctgtgg aagcacgcct aaaggatcaa c 31
<210> 379
   <211> 31
   <212> DNA
   <213> Artificial sequence
<220>
   <223> Oligonucleotide used for introducing a second mutation
<400> 379
   gttgatcctt taggcgtgct tccacagcca g 31
<210> 380
   <211> 31
   <212> DNA
   <213> Artificial sequence
<220>
   <223> Oligonucleotide used for introducing a second mutation
<400> 380
   ctggctgtgg aagaacgcct aaaggatcaa c 31
<210> 381
   <211> 31
   <212> DNA
   <213> Artificial sequence
<220>
   <223> Oligonucleotide used for introducing a second mutation
<400> 381
   gttgatcctt taggcgttct tccacagcca g 31
<210> 382
   <211> 31
   <212> DNA
   <213> Artificial sequence
<220>
   <223> Oligonucleotide used for introducing a second mutation
<400> 382
   ctggctgtgg aagctgccct aaaggatcaa c 31
<210> 383
   <211> 31
   <212> DNA
   <213> Artificial sequence
<220>
   <223> Oligonucleotide used for introducing a second mutation
<400> 383
   gttgatcctt tagggcagct tccacagcca g 31
<210> 384
   <211> 31
   <212> DNA
   <213> Artificial sequence
<220>
   <223> Oligonucleotide used for introducing a second mutation
<400> 384
   ctggctgtgg aagaagccct aaaggatcaa c 31
<210> 385
   <211> 31
   <212> DNA
   <213> Artificial sequence
<220>
   <223> Oligonucleotide used for introducing a second mutation
<400> 385
   gttgatcctt tagggcttct tccacagcca g 31
<210> 386
   <211> 31
   <212> DNA
   <213> Artificial sequence
<220>
   <223> Oligonucleotide used for introducing a second mutation
<400> 386
   ctggctgtgg aaagcgccct aaaggatcaa c 31
<210> 387
   <211> 31
   <212> DNA
   <213> Artificial sequence
<220>
   <223> Oligonucleotide used for introducing a second mutation
<400> 387
   gttgatcctt tagggcgctt tccacagcca g 31
<210> 388
   <211> 31
   <212> DNA
   <213> Artificial sequence
<220>
   <223> Oligonucleotide used for introducing a second mutation
<400> 388
   ctggctgtgg aaacagccct aaaggatcaa c 31
<210> 389
   <211> 31
   <212> DNA
   <213> Artificial sequence
<220>
   <223> Oligonucleotide used for introducing a second mutation
<400> 389
   gttgatcctt tagggctgtt tccacagcca g 31
<210> 390
   <211> 31
   <212> DNA
   <213> Artificial sequence
<220>
   <223> Oligonucleotide used for introducing a second mutation
<400> 390
   ctggctgtgg aagcttacct aaaggatcaa c 31
<210> 391
   <211> 31
   <212> DNA
   <213> Artificial sequence
<220>
   <223> Oligonucleotide used for introducing a second mutation
<400> 391
   gttgatcctt taggtaagct tccacagcca g 31
<210> 392
   <211> 31
   <212> DNA
   <213> Artificial sequence
<220>
   <223> Oligonucleotide used for introducing a second mutation
<400> 392
   ctggctgtgg aagaatacct aaaggatcaa c 31
<210> 393
   <211> 31
   <212> DNA
   <213> Artificial sequence
<220>
   <223> Oligonucleotide used for introducing a second mutation
<400> 393
   gttgatcctt taggtattct tccacagcca g 31
<210> 394
   <211> 31
   <212> DNA
   <213> Artificial sequence
<220>
   <223> Oligonucleotide used for introducing a second mutation
<400> 394
   ctggctgtgg aaagctacct aaaggatcaa c 31
<210> 395
   <211> 31
   <212> DNA
   <213> Artificial sequence
<220>
   <223> Oligonucleotide used for introducing a second mutation
<400> 395
   gttgatcctt taggtagctt tccacagcca g 31
<210> 396
   <211> 31
   <212> DNA
   <213> Artificial sequence
<220>
   <223> Oligonucleotide used for introducing a second mutation
<400> 396
   ctggctgtgg aaacatacct aaaggatcaa c 31
<210> 397
   <211> 31
   <212> DNA
   <213> Artificial sequence
<220>
   <223> Oligonucleotide used for introducing a second mutation
<400> 397
   gttgatcctt taggtatgtt tccacagcca g 31
<210> 398
   <211> 36
   <212> DNA
   <213> Artificial sequence
<220>
   <223> Oligonucleotide used for introducing a second mutation
<400> 398
   gctagttgga gtaatgcatc tctggaacag atttgg 36
<210> 399
   <211> 36
   <212> DNA
   <213> Artificial sequence
<220>
   <223> Oligonucleotide used for introducing a second mutation
<400> 399
   ccaaatctgt tccagagatg cattactcca actagc 36
<210> 400
   <211> 36
   <212> DNA
   <213> Artificial sequence
<220>
   <223> Oligonucleotide used for introducing a second mutation
<400> 400
   gctagttgga gtaatggatc tctggaacag atttgg 36
<210> 401
   <211> 36
   <212> DNA
   <213> Artificial sequence
<220>
   <223> Oligonucleotide used for introducing a second mutation
<400> 401
   ccaaatctgt tccagagatc cattactcca actagc 36
<210> 402
   <211> 36
   <212> DNA
   <213> Artificial sequence
<220>
   <223> Oligonucleotide used for introducing a second mutation
<400> 402
   gctagttgga gtaattcatc tctggaacag atttgg 36
<210> 403
   <211> 36
   <212> DNA
   <213> Artificial Sequence
<220>
   <223> Oligonucleotide used for introducing a second mutation
<400> 403
   ccaaatctgt tccagagatg aattactcca actagc 36
<210> 404
   <211> 54
   <212> DNA
   <213> Artificial Sequence
<220>
   <223> oligonucleotide for introducing mutation within the ISU of lentiviral ENV protein
<220>
   <221> misc_feature
   <222> (1)..(5)
   <223> n is a, c, g, or t
<400> 404
   nnnnnctcga gaccggtcca actagaacca tgagagtgaa ggagaaatat cagc 54
<210> 405
   <211> 31
   <212> DNA
   <213> Artificial Sequence
<220>
   <223> oligonucleotide for introducing mutation within the ISU of lentiviral ENV protein
<220>
   <221> misc_feature
   <222> (1)..(5)
   <223> n is a, c, g, or t
<400> 405
   nnnnnacgcg ttcaatatcc ctgcctaact c 31
<210> 406
   <211> 34
   <212> DNA
   <213> Artificial Sequence
<220>
   <223> oligonucleotide for introducing mutation within the ISU of lentiviral ENV protein
<220>
   <221> misc_feature
   <222> (1)..(5)
   <223> n is a, c, g, or t
<400> 406
   nnnnnacgcg tttatagcaa aatcctttcc aagc 34
<210> 407
   <211> 8
   <212> PRT
   <213> Artificial sequence
<220>
   <223> fragment of lentiviral ENV ISU
<400> 407
   Ala Val Glu Ala Ala Leu Lys Asp
   1 5
<210> 408
   <211> 8
   <212> PRT
   <213> Artificial sequence
<220>
   <223> fragment of lentiviral ENV ISU
<400> 408
   Ala Val Glu Glu Ala Leu Lys Asp
   1 5
<210> 409
   <211> 8
   <212> PRT
   <213> Artificial sequence
<220>
   <223> fragment of lentiviral ENV ISU
<400> 409
   Ala Val Glu Ser Ala Leu Lys Asp
   1 5
<210> 410
   <211> 8
   <212> PRT
   <213> Artificial sequence
<220>
   <223> fragment of lentiviral ENV ISU
<400> 410
   Ala Val Glu Thr Ala Leu Lys Asp
   1 5
<210> 411
   <211> 8
   <212> PRT
   <213> Artificial sequence
<220>
   <223> fragment of lentiviral ENV ISU
<400> 411
   Ala Ile Glu Ala Ala Leu Lys Asp
   1               5
<210> 412
   <211> 8
   <212> PRT
   <213> Artificial sequence
<220>
   <223> fragment of lentiviral ENV ISU
<400> 412
   Ala Ile Glu Glu Ala Leu Lys Asp
   1               5
<210> 413
   <211> 8
   <212> PRT
   <213> Artificial sequence
<220>
   <223> fragment of lentiviral ENV ISU
<400> 413
   Ala Ile Glu Ser Ala Leu Lys Asp
   1               5
<210> 414
   <211> 8
   <212> PRT
   <213> Artificial sequence
<220>
   <223> fragment of lentiviral ENV ISU
<400> 414
   Ala Ile Glu Thr Ala Leu Lys Asp
   1               5
<210> 415
   <211> 38
   <212> PRT
   <213> Artificial Sequence
<220>
   <223> derived from lentiviral ENV protein
<220>
   <221> MISC_FEATURE
   <222> (2) .. (2)
   <223> Xaa = I or V
<220>
   <221> MISC_FEATURE
   <222> (4) .. (4)
   <223> Xaa = K or R
<220>
   <221> misc_feature
   <222> (7)..(7)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (13)..(14)
   <223> Xaa can be any naturally occurring amino acid
<220>
   <221> MISC_FEATURE
   <222> (18)..(18)
   <223> Xaa = A or S
<220>
   <221> MISC_FEATURE
   <222> (19) .. (19)
   <223> Xaa = F or G
<220>
   <221> misc_feature
   <222> (20) .. (21)
   <223> Xaa can be any naturally occurring amino acid
<220>
   <221> misc_feature
   <222> (24) .. (24)
   <223> Xaa can be any naturally occurring amino acid
<220>
   <221> misc_feature
   <222> (26) .. (26)
   <223> Xaa can be any naturally occurring amino acid
<220>
   <221> misc_feature
   <222> (30)..(30)
   <223> Xaa can be any naturally occurring amino acid
<220>
   <221> MISC_FEATURE
   <222> (31)..(35)
   <223> Xaa = no or any amino acid
<220>
   <221> MISC_FEATURE
   <222> (36) .. (36)
   <223> Xaa = N or S
<220>
   <221> MISC_FEATURE
   <222> (37)..(37)
   <223> Xaa = E, D, N or A
<220>
   <221> MISC_FEATURE
   <222> (38)..(38)
   <223> Xaa = S or T
<400> 415
   Ala Xaa Glu Xaa Tyr Leu Xaa Asp Gln Xaa Xaa Leu Xaa Xaa Trp Gly
   1               5                   10                  15
   Cys Xaa Xaa Xaa Xaa Cys Val Xaa Thr Xaa Val Pro Trp Xaa Xaa Xaa
               20                  25                  30
   Xaa Xaa Xaa Xaa Xaa Xaa
           35
<210> 416
   <211> 9
   <212> PRT
   <213> Artificial sequence
<220>
   <223> fragment of lentiviral ENV ISU
<220>
   <221> MISC_FEATURE
   <222> (2) .. (2)
   <223> Xaa = I or V
<220>
   <221> MISC_FEATURE
   <222> (4) .. (4)
   <223> Xaa = K or R
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7) .. (7)
   <223> Xaa can be any naturally occuring amino acid
<400> 416
   Ala Xaa Glu Xaa Xaa Xaa Xaa Asp Gln
   1 5
<210> 417
   <211> 861
   <212> PRT
   <213> Human immunodeficiency virus type 1
<400> 417
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Gly Asn Ala Thr Asn Thr Asn Ser Ser Asn
       130                 135                 140
   Thr Asn Ser Ser Ser Gly Glu Met Met Met Glu Lys Gly Glu Ile Lys
   145                 150                 155                 160
   Asn Cys Ser Phe Asn Ile Ser Thr Ser Ile Arg Gly Lys Val Gln Lys
                   165                 170                 175
   Glu Tyr Ala Phe Phe Tyr Lys Leu Asp Ile Ile Pro Ile Asp Asn Asp
               180                 185                 190
   Thr Thr Ser Tyr Thr Leu Thr Ser Cys Asn Thr Ser Val Ile Thr Gln
           195                 200                 205
   Ala Cys Pro Lys Val Ser Phe Glu Pro Ile Pro Ile His Tyr Cys Ala
       210                 215                 220
   Pro Ala Gly Phe Ala Ile Leu Lys Cys Asn Asn Lys Thr Phe Asn Gly
   225                 230                 235                 240
   Thr Gly Pro Cys Thr Asn Val Ser Thr Val Gln Cys Thr His Gly Ile
                   245                 250                 255
   Arg Pro Val Val Ser Thr Gln Leu Leu Leu Asn Gly Ser Leu Ala Glu
               260                 265                 270
   Glu Glu Val Val Ile Arg Ser Ala Asn Phe Thr Asp Asn Ala Lys Thr
           275                 280                 285
   Ile Ile Val Gln Leu Asn Gln Ser Val Glu Ile Asn Cys Thr Arg Pro
       290                 295                 300
   Asn Asn Asn Thr Arg Lys Ser Ile Arg Ile Gln Arg Gly Pro Gly Arg
   305                 310                 315                 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465 470 475 480
   Arg Asp Asn Trp Arg Ser Glu Leu Tyr Lys Tyr Lys Val Val Lys Ile
   485 490 495
   Glu Pro Leu Gly Val Ala Pro Thr Lys Ala Lys Arg Arg Val Val Gln
   500 505 510
   Arg Glu Lys Arg Ala Val Gly Ile Gly Ala Leu Phe Leu Gly Phe Leu
   515 520 525
   Gly Ala Ala Gly Ser Thr Met Gly Ala Arg Ser Met Thr Leu Thr Val
   530 535 540
   Gln Ala Arg Gln Leu Leu Ser Gly Ile Val Gln Gln Gln Asn Asn Leu
   545 550 555 560
   Leu Arg Ala Ile Glu Ala Gln Gln His Leu Leu Gln Leu Thr Val Trp
   565 570 575
   Gly Ile Lys Gln Leu Gln Ala Arg Ile Leu Ala Val Glu Arg Tyr Leu
   580 585 590
   Lys Asp Gln Gln Leu Leu Gly Ile Trp Gly Cys Ser Gly Lys Leu Ile
   595 600 605
   Cys Thr Thr Ala Val Pro Trp Asn Ala Ser Trp Ser Asn Lys Ser Leu
   610 615 620
   Glu Gln Ile Trp Asn Asn Met Thr Trp Met Glu Trp Asp Arg Glu Ile
   625 630 635 640
   Asn Asn Tyr Thr Ser Leu Ile His Ser Leu Ile Glu Glu Ser Gln Asn
   645 650 655
   Gln Gln Glu Lys Asn Glu Gln Glu Leu Leu Glu Leu Asp Lys Trp Ala
   660 665 670
   Ser Leu Trp Asn Trp Phe Asn Ile Thr Asn Trp Leu Trp Tyr Ile Lys
   675 680 685
   Ile Phe Ile Met Ile Val Gly Gly Leu Val Gly Leu Arg Ile Val Phe
   690 695 700
   Ala Val Leu Ser Ile Val Asn Arg Val Arg Gln Gly Tyr Ser Pro Leu
   705 710 715 720
   Ser Phe Gln Thr His Leu Pro Thr Pro Arg Gly Pro Asp Arg Pro Glu
   725 730 735
   Gly Ile Glu Glu Glu Gly Gly Glu Arg Asp Arg Asp Arg Ser Ile Arg
   740 745 750
   Leu Val Asn Gly Ser Leu Ala Leu Ile Trp Asp Asp Leu Arg Ser Leu
   755 760 765
   Cys Leu Phe Ser Tyr His Arg Leu Arg Asp Leu Leu Leu Ile Val Thr
   770 775 780
   Arg Ile Val Glu Leu Leu Gly Arg Arg Gly Trp Glu Ala Leu Lys Tyr
   785 790 795 800
   Trp Trp Asn Leu Leu Gln Tyr Trp Ser Gln Glu Leu Lys Asn Ser Ala
   805 810 815
   Val Ser Leu Leu Asn Ala Thr Ala Ile Ala Val Ala Glu Gly Thr Asp
   820 825 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 418
   <211> 859
   <212> PRT
   <213> Human immunodeficiency virus type 2
<400> 418
   Met Cys Gly Arg Asn Gln Leu Phe Val Ala Ser Leu Leu Ala Ser Ala
   1 5 10 15
   Cys Leu Ile Tyr Cys Val Gln Tyr Val Thr Val Phe Tyr Gly Val Pro
   20 25 30
   Val Trp Arg Asn Ala Ser Ile Pro Leu Phe Cys Ala Thr Lys Asn Arg
   35 40 45
   Asp Thr Trp Gly Thr Ile Gln Cys Leu Pro Asp Asn Asp Asp Tyr Gln
   50 55 60
   Glu Ile Ala Leu Asn Val Thr Glu Ala Phe Asp Ala Trp Asn Asn Thr
   65 70 75 80
   Val Thr Glu Gln Ala Val Glu Asp Val Trp Ser Leu Phe Glu Thr Ser
   85 90 95
   Ile Lys Pro Cys Val Lys Leu Thr Pro Leu Cys Val Ala Met Arg Cys
   100 105 110
   Asn Ser Thr Thr Ala Lys Asn Thr Thr Ser Thr Pro Thr Thr Thr Thr
   115 120 125
   Thr Ala Asn Thr Thr Ile Gly Glu Asn Ser Ser Cys Ile Arg Thr Asp
   130 135 140
   Asn Cys Thr Gly Leu Gly Glu Glu Glu Met Val Asp Cys Gln Phe Asn
   145 150 155 160
   Met Thr Gly Leu Glu Arg Asp Lys Lys Lys Leu Tyr Asn Glu Thr Trp
   165 170 175
   Tyr Ser Lys Asp Val Val Cys Glu Ser Asn Asp Thr Lys Lys Glu Lys
   180 185 190
   Thr Cys Tyr Met Asn His Cys Asn Thr Ser Val Ile Thr Glu Ser Cys
   195 200 205
   Asp Lys His Tyr Trp Asp Thr Met Arg Phe Arg Tyr Cys Ala Pro Pro
   210 215 220
   Gly Phe Ala Leu Leu Arg Cys Asn Asp Thr Asn Tyr Ser Gly Phe Glu
   225 230 235 240
   Pro Asn Cys Ser Lys Val Val Ala Ala Thr Cys Thr Arg Met Met Glu
   245 250 255
   Thr Gln Thr Ser Thr Trp Phe Gly Phe Asn Gly Thr Arg Ala Glu Asn
   260 265 270
   Arg Thr Tyr Ile Tyr Trp His Gly Arg Asp Asn Arg Thr Ile Ile Ser
   275 280 285
   Leu Asn Lys Phe Tyr Asn Leu Thr Val His Cys Lys Arg Pro Gly Asn
   290 295 300
   Lys Thr Val Val Pro Ile Thr Leu Met Ser Gly Leu Val Phe His Ser
   305 310 315 320
   Gln Pro Ile Asn Arg Arg Pro Arg Gln Ala Trp Cys Trp Phe Lys Gly
                   325                 330                 335
   Glu Trp Lys Glu Ala Met Lys Glu Val Lys Leu Thr Leu Ala Lys His
               340                 345                 350
   Pro Arg Tyr Lys Gly Thr Asn Asp Thr Glu Lys Ile Arg Phe Ile Ala
           355                 360                 365
   Pro Gly Glu Arg Ser Asp Pro Glu Val Ala Tyr Met Trp Thr Asn Cys
       370                 375                 380
   Arg Gly Glu Phe Leu Tyr Cys Asn Met Thr Trp Phe Leu Asn Trp Val
   385                 390                 395                 400
   Glu Asn Arg Thr Asn Gln Thr Gln His Asn Tyr Val Pro Cys His Ile
                   405                 410                 415
   Lys Gln Ile Ile Asn Thr Trp His Lys Val Gly Lys Asn Val Tyr Leu
               420                 425                 430
   Pro Pro Arg Glu Gly Gln Leu Thr Cys Asn Ser Thr Val Thr Ser Ile
           435                 440                 445
   Ile Ala Asn Ile Asp Gly Gly Glu Asn Gln Thr Asn Ile Thr Phe Ser
       450                 455                 460
   Ala Glu Val Ala Glu Leu Tyr Arg Leu Glu Leu Gly Asp Tyr Lys Leu
   465                 470                 475                 480
   Ile Glu Val Thr Pro Ile Gly Phe Ala Pro Thr Pro Val Lys Arg Tyr
                   485                 490                 495
   Ser Ser Ala Pro Val Arg Asn Lys Arg Gly Val Phe Val Leu Gly Phe
               500                 505                 510
   Leu Gly Phe Leu Thr Thr Ala Gly Ala Ala Met Gly Ala Ala Ser Leu
           515                 520                 525
   Thr Leu Ser Ala Gln Ser Arg Thr Leu Leu Ala Gly Ile Val Gln Gln
       530                 535                 540
   Gln Gln Gln Leu Leu Asp Val Val Lys Arg Gln Gln Glu Met Leu Arg
   545                 550                 555                 560
   Leu Thr Val Trp Gly Thr Lys Asn Leu Gln Ala Arg Val Thr Ala Ile
                   565                 570                 575
   Glu Lys Tyr Leu Lys Asp Gln Ala Gln Leu Asn Ser Trp Gly Cys Ala
   580 585 590
   Phe Arg Gln Val Cys His Thr Thr Val Pro Trp Val Asn Asp Thr Leu
   595 600 605
   Thr Pro Asp Trp Asn Asn Met Thr Trp Gln Glu Trp Glu Gln Arg Ile
   610 615 620
   Arg Asn Leu Glu Ala Asn Ile Ser Glu Ser Leu Glu Gln Ala Gln Ile
   625 630 635 640
   Gln Gln Glu Lys Asn Met Tyr Glu Leu Gln Lys Leu Asn Ser Trp Asp
   645 650 655
   Val Phe Gly Asn Trp Phe Asp Leu Thr Ser Trp Ile Lys Tyr Ile Gln
   660 665 670
   Tyr Gly Val Tyr Ile Val Val Gly Ile Ile Val Leu Arg Ile Val Ile
   675 680 685
   Tyr Val Val Gln Met Leu Ser Arg Leu Arg Lys Gly Tyr Arg Pro Val
   690 695 700
   Phe Ser Ser Pro Pro Ala Tyr Phe Gln Gln Ile His Ile His Lys Asp
   705 710 715 720
   Arg Glu Gln Pro Ala Arg Glu Glu Thr Glu Glu Asp Val Gly Asn Ser
   725 730 735
   Val Gly Asp Asn Trp Trp Pro Trp Pro Ile Arg Tyr Ile His Phe Leu
   740 745 750
   Ile Arg Gln Leu Ile Arg Leu Leu Asn Arg Leu Tyr Asn Ile Cys Arg
   755 760 765
   Asp Leu Leu Ser Arg Ser Phe Gln Thr Leu Gln Leu Ile Ser Gln Ser
   770 775 780
   Leu Arg Arg Ala Leu Thr Ala Val Arg Asp Trp Leu Arg Phe Asn Thr
   785 790 795 800
   Ala Tyr Leu Gln Tyr Gly Gly Glu Trp Ile Gln Glu Ala Phe Arg Ala
   805 810 815
   Phe Ala Arg Ala Thr Gly Glu Thr Leu Thr Asn Ala Trp Arg Gly Phe
   820 825 830
   Trp Gly Thr Leu Gly Gln Ile Gly Arg Gly Ile Leu Ala Val Pro Arg
   835 840 845
   Arg Ile Arg Gln Gly Ala Glu Ile Ala Leu Leu
   850 855
<210> 419
   <211> 879
   <212> PRT
   <213> Simian immunodeficiency virus
<400> 419
   Met Gly Cys Leu Gly Asn Gln Leu Leu Ile Ala Ile Leu Leu Leu Ser
   1 5 10 15
   Val Tyr Gly Ile Tyr Cys Thr Leu Tyr Val Thr Val Phe Tyr Gly Val
   20 25 30
   Pro Ala Trp Arg Asn Ala Thr Ile Pro Leu Phe Cys Ala Thr Lys Asn
   35 40 45
   Arg Asp Thr Trp Gly Thr Thr Gln Cys Leu Pro Asp Asn Gly Asp Tyr
   50 55 60
   Ser Glu Val Ala Leu Asn Val Thr Glu Ser Phe Asp Ala Trp Asn Asn
   65 70 75 80
   Thr Val Thr Glu Gln Ala Ile Glu Asp Val Trp Gln Leu Phe Glu Thr
   85 90 95
   Ser Ile Lys Pro Cys Val Lys Leu Ser Pro Leu Cys Ile Thr Met Arg
   100 105 110
   Cys Asn Lys Ser Glu Thr Asp Arg Trp Gly Leu Thr Lys Ser Ile Thr
   115 120 125
   Thr Thr Ala Ser Thr Thr Ser Thr Thr Ala Ser Ala Lys Val Asp Met
   130 135 140
   Val Asn Glu Thr Ser Ser Cys Ile Ala Gln Asp Asn Cys Thr Gly Leu
   145 150 155 160
   Glu Gln Glu Gln Met Ile Ser Cys Lys Phe Asn Met Thr Gly Leu Lys
   165 170 175
   Arg Asp Lys Lys Lys Glu Tyr Asn Glu Thr Trp Tyr Ser Ala Asp Leu
   180 185 190
   Val Cys Glu Gln Gly Asn Asn Thr Gly Asn Glu Ser Arg Cys Tyr Met
   195 200 205
   Asn His Cys Asn Thr Ser Val Ile Gln Glu Ser Cys Asp Lys His Tyr
   210 215 220
   Trp Asp Ala Ile Arg Phe Arg Tyr Cys Ala Pro Pro Gly Tyr Ala Leu
   225 230 235 240
   Leu Arg Cys Asn Asp Thr Asn Tyr Ser Gly Phe Met Pro Lys Cys Ser
   245 250 255
   Lys Val Val Val Ser Ser Cys Thr Arg Met Met Glu Thr Gln Thr Ser
   260 265 270
   Thr Trp Phe Gly Phe Asn Gly Thr Arg Ala Glu Asn Arg Thr Tyr Ile
   275 280 285
   Tyr Trp His Gly Arg Asp Asn Arg Thr Ile Ile Ser Leu Asn Lys Tyr
   290 295 300
   Tyr Asn Leu Thr Met Lys Cys Arg Arg Pro Gly Asn Lys Thr Val Leu
   305 310 315 320
   Pro Val Thr Ile Met Ser Gly Leu Val Phe His Ser Gln Pro Ile Asn
   325 330 335
   Asp Arg Pro Lys Gln Ala Trp Cys Trp Phe Gly Gly Lys Trp Lys Asp
   340 345 350
   Ala Ile Lys Glu Val Lys Gln Thr Ile Val Lys His Pro Arg Tyr Thr
   355 360 365
   Gly Thr Asn Asn Thr Asp Lys Ile Asn Leu Thr Ala Pro Gly Gly Gly
   370 375 380
   Asp Pro Glu Val Thr Phe Met Trp Thr Asn Cys Arg Gly Glu Phe Leu
   385 390 395 400
   Tyr Cys Lys Met Asn Trp Phe Leu Asn Trp Val Glu Asp Arg Asn Thr
   405 410 415
   Ala Asn Gln Lys Pro Lys Glu Gln His Lys Arg Asn Tyr Val Pro Cys
   420 425 430
   His Ile Arg Gln Ile Ile Asn Thr Trp His Lys Val Gly Lys Asn Val
   435 440 445
   Tyr Leu Pro Pro Arg Glu Gly Asp Leu Thr Cys Asn Ser Thr Val Thr
       450                 455                 460
   Ser Leu Ile Ala Asn Ile Asp Trp Ile Asp Gly Asn Gln Thr Asn Ile
   465                 470                 475                 480
   Thr Met Ser Ala Glu Val Ala Glu Leu Tyr Arg Leu Glu Leu Gly Asp
                   485                 490                 495
   Tyr Lys Leu Val Glu Ile Thr Pro Ile Gly Leu Ala Pro Thr Asp Val
               500                 505                 510
   Lys Arg Tyr Thr Thr Gly Gly Thr Ser Arg Asn Lys Arg Gly Val Phe
           515                 520                 525
   Val Leu Gly Phe Leu Gly Phe Leu Ala Thr Ala Gly Ser Ala Met Gly
       530                 535                 540
   Ala Ala Ser Leu Thr Leu Thr Ala Gln Ser Arg Thr Leu Leu Ala Gly
   545                 550                 555                 560
   Ile Val Gln Gln Gln Gln Gln Leu Leu Asp Val Val Lys Arg Gln Gln
                   565                 570                 575
   Glu Leu Leu Arg Leu Thr Val Trp Gly Thr Lys Asn Leu Gln Thr Arg
               580                 585                 590
   Val Thr Ala Ile Glu Lys Tyr Leu Lys Asp Gln Ala Gln Leu Asn Ala
           595                 600                 605
   Trp Gly Cys Ala Phe Arg Gln Val Cys His Thr Thr Val Pro Trp Pro
       610                 615                 620
   Asn Ala Ser Leu Thr Pro Lys Trp Asn Asn Glu Thr Trp Gln Glu Trp
   625                 630                 635                 640
   Glu Arg Lys Val Asp Phe Leu Glu Glu Asn Ile Thr Ala Leu Leu Glu
                   645                 650                 655
   Glu Ala Gln Ile Gln Gln Glu Lys Asn Met Tyr Glu Leu Gln Lys Leu
               660                 665                 670
   Asn Ser Trp Asp Val Phe Gly Asn Trp Phe Asp Leu Ala Ser Trp Ile
           675                 680                 685
   Lys Tyr Ile Gln Tyr Gly Val Tyr Ile Val Val Gly Val Ile Leu Leu
   690                 695                 700
   Arg Ile Val Ile Tyr Ile Val Gln Met Leu Ala Lys Leu Arg Gln Gly
   705                 710                 715                 720
   Tyr Arg Pro Val Phe Ser Ser Pro Pro Ser Tyr Phe Gln Gln Thr His
                   725                 730                 735
   Ile Gln Gln Asp Pro Ala Leu Pro Thr Arg Glu Gly Lys Glu Arg Asp
               740                 745                 750
   Gly Gly Glu Gly Gly Gly Asn Ser Ser Trp Pro Trp Gln Ile Glu Tyr
           755                 760                 765
   Ile His Phe Leu Ile Arg Gln Leu Ile Arg Leu Leu Thr Trp Leu Phe
       770                 775                 780
   Ser Asn Cys Arg Thr Leu Leu Ser Arg Val Tyr Gln Ile Leu Gln Pro
   785                 790                 795                 800
   Ile Leu Gln Arg Leu Ser Ala Thr Leu Gln Arg Ile Arg Glu Val Leu
                   805                 810                 815
   Arg Thr Glu Leu Thr Tyr Leu Gln Tyr Gly Trp Ser Tyr Phe His Glu
               820                 825                 830
   Ala Val Gln Ala Val Trp Arg Ser Ala Thr Glu Thr Leu Ala Gly Ala
           835                 840                 845
   Trp Gly Asp Leu Trp Glu Thr Leu Arg Arg Gly Gly Arg Trp Ile Leu
       850                 855                 860
   Ala Ile Pro Arg Arg Ile Arg Gln Gly Leu Glu Leu Thr Leu Leu
   865                 870                 875
<210> 420
   <211> 859
   <212> PRT
   <213> Human immunodeficiency virus type 2
<400> 420
   Met Cys Gly Arg Asn Gln Leu Phe Val Ala Ser Leu Leu Ala Ser Ala
   1               5                   10                  15
   Cys Leu Ile Tyr Cys Val Gln Tyr Val Thr Val Phe Tyr Gly Val Pro
               20                  25                  30
   Val Trp Arg Asn Ala Ser Ile Pro Leu Phe Cys Ala Thr Lys Asn Arg
   35 40 45
   Asp Thr Trp Gly Thr Ile Gln Cys Leu Pro Asp Asn Asp Asp Tyr Gln
   50 55 60
   Glu Ile Ala Leu Asn Val Thr Glu Ala Phe Asp Ala Trp Asn Asn Thr
   65 70 75 80
   Val Thr Glu Gln Ala Val Glu Asp Val Trp Ser Leu Phe Glu Thr Ser
   85 90 95
   Ile Lys Pro Cys Val Lys Leu Thr Pro Leu Cys Val Ala Met Arg Cys
   100 105 110
   Asn Ser Thr Thr Ala Lys Asn Thr Thr Ser Thr Pro Thr Thr Thr Thr
   115 120 125
   Thr Ala Asn Thr Thr Ile Gly Glu Asn Ser Ser Cys Ile Arg Thr Asp
   130 135 140
   Asn Cys Thr Gly Leu Gly Glu Glu Glu Met Val Asp Cys Gln Phe Asn
   145 150 155 160
   Met Thr Gly Leu Glu Arg Asp Lys Lys Lys Leu Tyr Asn Glu Thr Trp
   165 170 175
   Tyr Ser Lys Asp Val Val Cys Glu Ser Asn Asp Thr Lys Lys Glu Lys
   180 185 190
   Thr Cys Tyr Met Asn His Cys Asn Thr Ser Val Ile Thr Glu Ser Cys
   195 200 205
   Asp Lys His Tyr Trp Asp Thr Met Arg Phe Arg Tyr Cys Ala Pro Pro
   210 215 220
   Gly Phe Ala Leu Leu Arg Cys Asn Asp Thr Asn Tyr Ser Gly Phe Glu
   225 230 235 240
   Pro Asn Cys Ser Lys Val Val Ala Ala Thr Cys Thr Arg Met Met Glu
   245 250 255
   Thr Gln Thr Ser Thr Trp Phe Gly Phe Asn Gly Thr Arg Ala Glu Asn
   260 265 270
   Arg Thr Tyr Ile Tyr Trp His Gly Arg Asp Asn Arg Thr Ile Ile Ser
   275 280 285
   Leu Asn Lys Phe Tyr Asn Leu Thr Val His Cys Lys Arg Pro Gly Asn
   290 295 300
   Lys Thr Val Val Pro Ile Thr Leu Met Ser Gly Leu Val Phe His Ser
   305 310 315 320
   Gln Pro Ile Asn Arg Arg Pro Arg Gln Ala Trp Cys Trp Phe Lys Gly
   325 330 335
   Glu Trp Lys Glu Ala Met Lys Glu Val Lys Leu Thr Leu Ala Lys His
   340 345 350
   Pro Arg Tyr Lys Gly Thr Asn Asp Thr Glu Lys Ile Arg Phe Ile Ala
   355 360 365
   Pro Gly Glu Arg Ser Asp Pro Glu Val Ala Tyr Met Trp Thr Asn Cys
   370 375 380
   Arg Gly Glu Phe Leu Tyr Cys Asn Met Thr Trp Phe Leu Asn Trp Val
   385 390 395 400
   Glu Asn Arg Thr Asn Gln Thr Gln His Asn Tyr Val Pro Cys His Ile
   405 410 415
   Lys Gln Ile Ile Asn Thr Trp His Lys Val Gly Lys Asn Val Tyr Leu
   420 425 430
   Pro Pro Arg Glu Gly Gln Leu Thr Cys Asn Ser Thr Val Thr Ser Ile
   435 440 445
   Ile Ala Asn Ile Asp Gly Gly Glu Asn Gln Thr Asn Ile Thr Phe Ser
   450 455 460
   Ala Glu Val Ala Glu Leu Tyr Arg Leu Glu Leu Gly Asp Tyr Lys Leu
   465 470 475 480
   Ile Glu Val Thr Pro Ile Gly Phe Ala Pro Thr Pro Val Lys Arg Tyr
   485 490 495
   Ser Ser Ala Pro Val Arg Asn Lys Arg Gly Val Phe Val Leu Gly Phe
   500 505 510
   Leu Gly Phe Leu Thr Thr Ala Gly Ala Ala Met Gly Ala Ala Ser Leu
   515 520 525
   Thr Leu Ser Ala Gln Ser Arg Thr Leu Leu Ala Gly Ile Val Gln Gln
   530 535 540
   Gln Gln Gln Leu Leu Asp Val Val Lys Arg Gln Gln Glu Met Leu Arg
   545 550 555 560
   Leu Thr Val Trp Gly Thr Lys Asn Leu Gln Ala Arg Val Thr Ala Ile
   565 570 575
   Glu Lys Arg Leu Lys Asp Gln Ala Gln Leu Asn Ser Trp Gly Cys Ala
   580 585 590
   Phe Arg Gln Val Cys His Thr Thr Val Pro Trp Val Asn Asp Thr Leu
   595 600 605
   Thr Pro Asp Trp Asn Asn Met Thr Trp Gln Glu Trp Glu Gln Arg Ile
   610 615 620
   Arg Asn Leu Glu Ala Asn Ile Ser Glu Ser Leu Glu Gln Ala Gln Ile
   625 630 635 640
   Gln Gln Glu Lys Asn Met Tyr Glu Leu Gln Lys Leu Asn Ser Trp Asp
   645 650 655
   Val Phe Gly Asn Trp Phe Asp Leu Thr Ser Trp Ile Lys Tyr Ile Gln
   660 665 670
   Tyr Gly Val Tyr Ile Val Val Gly Ile Ile Val Leu Arg Ile Val Ile
   675 680 685
   Tyr Val Val Gln Met Leu Ser Arg Leu Arg Lys Gly Tyr Arg Pro Val
   690 695 700
   Phe Ser Ser Pro Pro Ala Tyr Phe Gln Gln Ile His Ile His Lys Asp
   705 710 715 720
   Arg Glu Gln Pro Ala Arg Glu Glu Thr Glu Glu Asp Val Gly Asn Ser
   725 730 735
   Val Gly Asp Asn Trp Trp Pro Trp Pro Ile Arg Tyr Ile His Phe Leu
   740 745 750
   Ile Arg Gln Leu Ile Arg Leu Leu Asn Arg Leu Tyr Asn Ile Cys Arg
   755 760 765
   Asp Leu Leu Ser Arg Ser Phe Gln Thr Leu Gln Leu Ile Ser Gln Ser
   770 775 780
   Leu Arg Arg Ala Leu Thr Ala Val Arg Asp Trp Leu Arg Phe Asn Thr
   785 790 795 800
   Ala Tyr Leu Gln Tyr Gly Gly Glu Trp Ile Gln Glu Ala Phe Arg Ala
                   805                 810                 815
   Phe Ala Arg Ala Thr Gly Glu Thr Leu Thr Asn Ala Trp Arg Gly Phe
               820                 825                 830
   Trp Gly Thr Leu Gly Gln Ile Gly Arg Gly Ile Leu Ala Val Pro Arg
           835                 840                 845
   Arg Ile Arg Gln Gly Ala Glu Ile Ala Leu Leu
       850                 855
<210> 421
   <211> 879
   <212> PRT
   <213> Simian immunodeficiency virus
<400> 421
   Met Gly Cys Leu Gly Asn Gln Leu Leu Ile Ala Ile Leu Leu Leu Ser
   1               5                   10                  15
   Val Tyr Gly Ile Tyr Cys Thr Leu Tyr Val Thr Val Phe Tyr Gly Val
               20                  25                  30
   Pro Ala Trp Arg Asn Ala Thr Ile Pro Leu Phe Cys Ala Thr Lys Asn
           35                  40                  45
   Arg Asp Thr Trp Gly Thr Thr Gln Cys Leu Pro Asp Asn Gly Asp Tyr
       50                  55                  60
   Ser Glu Val Ala Leu Asn Val Thr Glu Ser Phe Asp Ala Trp Asn Asn
   65                  70                  75                  80
   Thr Val Thr Glu Gln Ala Ile Glu Asp Val Trp Gln Leu Phe Glu Thr
                   85                  90                  95
   Ser Ile Lys Pro Cys Val Lys Leu Ser Pro Leu Cys Ile Thr Met Arg
               100                 105                 110
   Cys Asn Lys Ser Glu Thr Asp Arg Trp Gly Leu Thr Lys Ser Ile Thr
           115                 120                 125
   Thr Thr Ala Ser Thr Thr Ser Thr Thr Ala Ser Ala Lys Val Asp Met
       130                 135                 140
   Val Asn Glu Thr Ser Ser Cys Ile Ala Gln Asp Asn Cys Thr Gly Leu
   145                 150                 155                 160
   Glu Gln Glu Gln Met Ile Ser Cys Lys Phe Asn Met Thr Gly Leu Lys
   165 170 175
   Arg Asp Lys Lys Lys Glu Tyr Asn Glu Thr Trp Tyr Ser Ala Asp Leu
   180 185 190
   Val Cys Glu Gln Gly Asn Asn Thr Gly Asn Glu Ser Arg Cys Tyr Met
   195 200 205
   Asn His Cys Asn Thr Ser Val Ile Gln Glu Ser Cys Asp Lys His Tyr
   210 215 220
   Trp Asp Ala Ile Arg Phe Arg Tyr Cys Ala Pro Pro Gly Tyr Ala Leu
   225 230 235 240
   Leu Arg Cys Asn Asp Thr Asn Tyr Ser Gly Phe Met Pro Lys Cys Ser
   245 250 255
   Lys Val Val Val Ser Ser Cys Thr Arg Met Met Glu Thr Gln Thr Ser
   260 265 270
   Thr Trp Phe Gly Phe Asn Gly Thr Arg Ala Glu Asn Arg Thr Tyr Ile
   275 280 285
   Tyr Trp His Gly Arg Asp Asn Arg Thr Ile Ile Ser Leu Asn Lys Tyr
   290 295 300
   Tyr Asn Leu Thr Met Lys Cys Arg Arg Pro Gly Asn Lys Thr Val Leu
   305 310 315 320
   Pro Val Thr Ile Met Ser Gly Leu Val Phe His Ser Gln Pro Ile Asn
   325 330 335
   Asp Arg Pro Lys Gln Ala Trp Cys Trp Phe Gly Gly Lys Trp Lys Asp
   340 345 350
   Ala Ile Lys Glu Val Lys Gln Thr Ile Val Lys His Pro Arg Tyr Thr
   355 360 365
   Gly Thr Asn Asn Thr Asp Lys Ile Asn Leu Thr Ala Pro Gly Gly Gly
   370 375 380
   Asp Pro Glu Val Thr Phe Met Trp Thr Asn Cys Arg Gly Glu Phe Leu
   385 390 395 400
   Tyr Cys Lys Met Asn Trp Phe Leu Asn Trp Val Glu Asp Arg Asn Thr
   405 410 415
   Ala Asn Gln Lys Pro Lys Glu Gln His Lys Arg Asn Tyr Val Pro Cys
   420 425 430
   His Ile Arg Gln Ile Ile Asn Thr Trp His Lys Val Gly Lys Asn Val
   435 440 445
   Tyr Leu Pro Pro Arg Glu Gly Asp Leu Thr Cys Asn Ser Thr Val Thr
   450 455 460
   Ser Leu Ile Ala Asn Ile Asp Trp Ile Asp Gly Asn Gln Thr Asn Ile
   465 470 475 480
   Thr Met Ser Ala Glu Val Ala Glu Leu Tyr Arg Leu Glu Leu Gly Asp
   485 490 495
   Tyr Lys Leu Val Glu Ile Thr Pro Ile Gly Leu Ala Pro Thr Asp Val
   500 505 510
   Lys Arg Tyr Thr Thr Gly Gly Thr Ser Arg Asn Lys Arg Gly Val Phe
   515 520 525
   Val Leu Gly Phe Leu Gly Phe Leu Ala Thr Ala Gly Ser Ala Met Gly
   530 535 540
   Ala Ala Ser Leu Thr Leu Thr Ala Gln Ser Arg Thr Leu Leu Ala Gly
   545 550 555 560
   Ile Val Gln Gln Gln Gln Gln Leu Leu Asp Val Val Lys Arg Gln Gln
   565 570 575
   Glu Leu Leu Arg Leu Thr Val Trp Gly Thr Lys Asn Leu Gln Thr Arg
   580 585 590
   Val Thr Ala Ile Glu Lys Arg Leu Lys Asp Gln Ala Gln Leu Asn Ala
   595 600 605
   Trp Gly Cys Ala Phe Arg Gln Val Cys His Thr Thr Val Pro Trp Pro
   610 615 620
   Asn Ala Ser Leu Thr Pro Lys Trp Asn Asn Glu Thr Trp Gln Glu Trp
   625 630 635 640
   Glu Arg Lys Val Asp Phe Leu Glu Glu Asn Ile Thr Ala Leu Leu Glu
   645 650 655
   Glu Ala Gln Ile Gln Gln Glu Lys Asn Met Tyr Glu Leu Gln Lys Leu
               660                 665                 670
   Asn Ser Trp Asp Val Phe Gly Asn Trp Phe Asp Leu Ala Ser Trp Ile
           675                 680                 685
   Lys Tyr Ile Gln Tyr Gly Val Tyr Ile Val Val Gly Val Ile Leu Leu
       690                 695                 700
   Arg Ile Val Ile Tyr Ile Val Gln Met Leu Ala Lys Leu Arg Gln Gly
   705                 710                 715                 720
   Tyr Arg Pro Val Phe Ser Ser Pro Pro Ser Tyr Phe Gln Gln Thr His
                   725                 730                 735
   Ile Gln Gln Asp Pro Ala Leu Pro Thr Arg Glu Gly Lys Glu Arg Asp
               740                 745                 750
   Gly Gly Glu Gly Gly Gly Asn Ser Ser Trp Pro Trp Gln Ile Glu Tyr
           755                 760                 765
   Ile His Phe Leu Ile Arg Gln Leu Ile Arg Leu Leu Thr Trp Leu Phe
       770                 775                 780
   Ser Asn Cys Arg Thr Leu Leu Ser Arg Val Tyr Gln Ile Leu Gln Pro
   785                 790                 795                 800
   Ile Leu Gln Arg Leu Ser Ala Thr Leu Gln Arg Ile Arg Glu Val Leu
                   805                 810                 815
   Arg Thr Glu Leu Thr Tyr Leu Gln Tyr Gly Trp Ser Tyr Phe His Glu
               820                 825                 830
   Ala Val Gln Ala Val Trp Arg Ser Ala Thr Glu Thr Leu Ala Gly Ala
           835                 840                 845
   Trp Gly Asp Leu Trp Glu Thr Leu Arg Arg Gly Gly Arg Trp Ile Leu
       850                 855                 860
   Ala Ile Pro Arg Arg Ile Arg Gln Gly Leu Glu Leu Thr Leu Leu
   865                 870                 875
<210> 422
   <211> 9
   <212> PRT
   <213> Artificial sequence
<220>
   <223> fragment of lentiviral ENV ISU
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7)..(7)
   <223> Xaa can be any naturally occuring amino acid
<400> 422
   Ala Ile Glu Lys Xaa Xaa Xaa Asp Gln
   1 5
<210> 423
   <211> 9
   <212> PRT
   <213> Artificial sequence
<220>
   <223> fragment of lentiviral ENV ISU
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7) .. (7)
   <223> Xaa can be any naturally occuring amino acid
<400> 423
   Ala Ile Glu Arg Xaa Xaa Xaa Asp Gln
   1 5
<210> 424
   <211> 9
   <212> PRT
   <213> Artificial sequence
<220>
   <223> fragment of lentiviral ENV ISU
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7) .. (7)
   <223> Xaa can be any naturally occuring amino acid
<400> 424
   Ala Val Glu Lys Xaa Xaa Xaa Asp Gln
   1               5
<210> 425
   <211> 9
   <212> PRT
   <213> Artificial sequence
<220>
   <223> fragment of lentiviral ENV ISU
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7)..(7)
   <223> Xaa can be any naturally occuring amino acid
<400> 425
   Ala Val Glu Arg Xaa Xaa Xaa Asp Gln
   1               5
<210> 426
   <211> 38
   <212> PRT
   <213> Artificial sequence
<220>
   <223> fragment of lentiviral ENV ISU
<220>
   <221> MISC_FEATURE
   <222> (2) .. (2)
   <223> Xaa = I or V
<220>
   <221> MISC_FEATURE
   <222> (4)..(4)
   <223> Xaa = K or R
<220>
   <221> MISC_FEATURE
   <222> (5)..(5)
   <223> Xaa is any naturally occuring amino acid or is mutated
<220>
   <221> MISC_FEATURE
   <222> (6) .. (6)
   <223> Xaa is any naturally occuring amino acid or is mutated
<220>
   <221> MISC_FEATURE
   <222> (7) .. (7)
   <223> Xaa can be any naturally occuring amino acid
<220>
   <221> MISC_FEATURE
   <222> (10)..(10)
   <223> Xaa can be any naturally occuring amino acid
<220>
   <221> MISC_FEATURE
   <222> (11)..(11)
   <223> Xaa can be any naturally occuring amino acid
<220>
   <221> MISC_FEATURE
   <222> (13)..(13)
   <223> Xaa can be any naturally occuring amino acid
<220>
   <221> MISC_FEATURE
   <222> (14)..(14)
   <223> Xaa can be any naturally occuring amino acid
<220>
   <221> MISC_FEATURE
   <222> (18)..(18)
   <223> Xaa = A or S
<220>
   <221> MISC_FEATURE
   <222> (19) .. (19)
   <223> Xaa = F or G
<220>
   <221> MISC_FEATURE
   <222> (20) .. (20)
   <223> Xaa can be any naturally occuring amino acid
<220>
   <221> MISC_FEATURE
   <222> (21) .. (21)
   <223> Xaa can be any naturally occuring amino acid
<220>
   <221> MISC_FEATURE
   <222> (24) .. (24)
   <223> Xaa can be any naturally occuring amino acid
<220>
   <221> MISC_FEATURE
   <222> (26).. (26)
   <223> Xaa can be any naturally occuring amino acid
<220>
   <221> MISC_FEATURE
   <222> (29) .. (29)
   <223> Xaa is any naturally occuring amino acid or is mutated
<220>
   <221> MISC_FEATURE
   <222> (30)..(30)
   <223> Xaa can be naturally occuring amino acid
<220>
   <221> MISC_FEATURE
   <222> (31)..(35)
   <223> Xaa can be any naturally occuring amino acid
<220>
   <221> MISC_FEATURE
   <222> (36) .. (36)
   <223> Xaa is any naturally occuring amino acid or is mutated
<220>
   <221> MISC_FEATURE
   <222> (37)..(37)
   <223> Xaa is any naturally occuring amino acid or is mutated
<220>
   <221> MISC_FEATURE
   <222> (38)..(38)
   <223> Xaa = S or T
<400> 426
   Ala Xaa Glu Xaa Xaa Xaa Xaa Asp Gln Xaa Xaa Leu Xaa Xaa Trp Gly
   1               5                   10                  15
   Cys Xaa Xaa Xaa Xaa Cys Val Xaa Thr Xaa Val Pro Xaa Xaa Xaa Xaa
               20                  25                  30
   Xaa Xaa Xaa Xaa Xaa Xaa
           35
<210> 427
   <211> 9
   <212> PRT
   <213> Artificial sequence
<220>
   <223> fragment of lentiviral ENV ISU
<220>
   <221> MISC_FEATURE
   <222> (2) .. (2)
   <223> Xaa = I or V
<220>
   <221> MISC_FEATURE
   <222> (4)..(4)
   <223> Xaa = K or R
<220>
   <221> MISC_FEATURE
   <222> (5)..(5)
   <223> Xaa is any naturally occuring amino acid
<220>
   <221> MISC_FEATURE
   <222> (6) .. (6)
   <223> Xaa is any naturally occuring amino acid
<220>
   <221> MISC_FEATURE
   <222> (7) .. (7)
   <223> Xaa can be any naturally occuring amino acid
<400> 427
   Ala Xaa Glu Xaa Xaa Xaa Xaa Asp Gln
   1               5
<210> 428
   <211> 15
   <212> PRT
   <213> Artificial sequence
<220>
   <223> fragment of lentiviral ENV ISU
<220>
   <221> MISC_FEATURE
   <222> (1)..(1)
   <223> Xaa is any naturally occuring amino acid or is mutated
<220>
   <221> MISC_FEATURE
   <222> (2) .. (2)
   <223> Xaa is any naturally occuring amino acid or is mutated
<220>
   <221> MISC_FEATURE
   <222> (3)..(3)
   <223> Xaa is any naturally occuring amino acid or is mutated
<220>
   <221> MISC_FEATURE
   <222> (4) .. (4)
   <223> Xaa is any naturally occuring amino acid or is mutated
<220>
   <221> MISC_FEATURE
   <222> (8) .. (8)
   <223> Xaa = I or V
<220>
   <221> MISC_FEATURE
   <222> (10)..(10)
   <223> Xaa = K or R
<220>
   <221> MISC_FEATURE
   <222> (11)..(11)
   <223> Xaa is any naturally occuring amino acid or is mutated
<220>
   <221> MISC_FEATURE
   <222> (12)..(12)
   <223> Xaa is any naturally occuring amino acid or is mutated
<220>
   <221> MISC_FEATURE
   <222> (13)..(13)
   <223> Xaa = any naturally occuring amino acid
<400> 428
   Xaa Xaa Xaa Xaa Ile Leu Ala Xaa Glu Xaa Xaa Xaa Xaa Asp Gln
   1               5                   10                  15